# EUROPEAN PATENT APPLICATION

(11) **EP 4 378 527 A2**
(43) Date of publication of application: **05.06.2024**
(21) Application number: 24170814.8
(22) Date of filing: 02.10.2020
(51) Int. Cl.: A61P 25/24

(54) **TRANSMUCOSAL THERAPEUTIC SYSTEM CONTAINING AGOMELATINE**

(30) Priority: 20.12.2019 EP 19218570
(62) Divisional of application: 20780760.3
(71) Applicant: LTS Lohmann Therapie-Systeme AG, 56626 Andernach (DE)
(72) Inventor: MOHR, Patrick, 53498 Bad Breisig (DE); RIETSCHER, René, 56626 Andernach (DE); EIFLER, René, 56072 Koblenz (DE); BOURQUAIN, Olga, 56307 Dürrholz (DE)
(74) Representative: Maiwald GmbH

(57) **Abstract**

The present invention relates to transmucosal therapeutic systems for the transmucosal administration of agomelatine comprising a mucoadhesive layer structure comprising a therapeutically effective amount of agomelatine, such agomelatine transmucosal therapeutic systems for use in a method of treatment, a method of treatment comprising applying such agomelatine transmucosal therapeutic systems, and processes of manufacture of such transmucosal therapeutic systems.

## Description

### TECHNICAL FIELD OF THE INVENTION

The present invention relates to a transmucosal therapeutic system for the transmucosal administration of agomelatine to the systemic circulation, and processes of manufacture, methods of treatment and uses thereof.

### BACKGROUND OF THE INVENTION

The active agent agomelatine (N-(2-(7-Methoxy-1-naphthyl)ethyl)acetamide) is a melatonergic antidepressant developed by Les Laboratoires Servier. The chemical structure is very similar to that of melatonin.

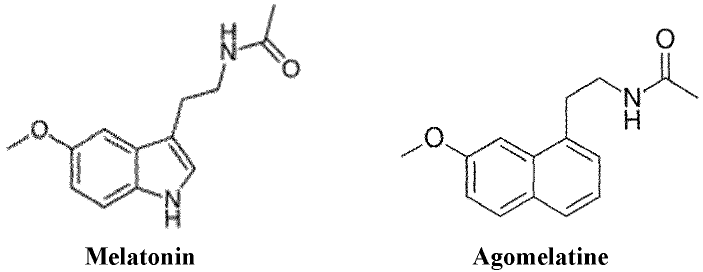

As a melatonergic agonist stimulating the MT1 and MT2 receptors, agomelatine is able to mediate synchronization of the circadian rhythm, much like melatonin. However, in addition and in contrast to melatonin, agomelatine is also a 5-HT2B / 5-HT2C antagonist, and blocking the serotonergic 5HT2C receptors causes enhanced release of dopamine and norepinephrine in the prefrontal cortex. Unexpected synergistic actions have been observed for the MT1/MT2 agonism and 5HT2C antagonism, and it is supposed that this synergy accounts for the antidepressant actions and unique clinical profile of agomelatine.

Agomelatine has been approved in Europe under the trade names Valdoxan^{®}, Melitor^{®} and Thymanax^{®} and is indicated for the treatment of major depressive disorder (MDD). The currently available form is a film tablet containing a dose of 25 mg, which is prescribed with an initial dose of one tablet to be taken at bedtime, with the option of doubling the dose if no improvement is seen. Agomelatine is the only antidepressant on the market with the mechanism of action described above.

Oral agomelatine undergoes extensive first pass and systemic metabolism, mainly *via* the cytochrome CYP1A2. Although agomelatine is well absorbed orally (> 80 %), overall bioavailability is very low (less than 5 %), with pronounced inter-individual variability. Both the time to reach maximal blood plasma concentration as well as elimination half-life t_{1/2} is about 1 to 2 hours, and in steady state, the volume of distribution is 35 liters, with a plasma protein binding of 95 %.

When compared to other antidepressants, agomelatine seems to have a more rapid onset of effects (usually within a week), and major side effects commonly known for other antidepressants such as weight gain, sexual dysfunction, anticholinergic symptoms and cardiotoxicity appear to be reduced. However, agomelatine bears the risk of hepatotoxicity of which the mechanism remains unclarified, manifesting as increased alanine aminotransferase (ALAT) and/or aspartate aminotransferase (ASAT) values, and in few exceptional cases, the outcome was fatal or necessitated liver transplantation. In addition, hepatic impairment was also reported to be related to a huge increase in agomelatine exposure, with up to 140-fold AUC and cₘₐₓ values observed for patients with moderate hepatic impairment compared to healthy subj ects.

Efforts were made by Servier and Novartis to establish a sublingual dosage form of agomelatine, supposedly in order to avoid the first-pass metabolism and the associated drawbacks as outlined above (low oral bioavailability and hepatotoxicity), resulting in placebo controlled randomized studies with 1 and 2 mg (Servier) or 0.5 and 1 mg agomelatine sublingual tablets (Novartis). No result is publicly available for the 2008/2009 Servier trial. The Novartis studies, commenced in 2011/2011, were essentially unsuccessful in that the efficacy of the agomelatine sublingual tablets was not better than placebo, and in that there was no clear dose response relationship, though at least liver toxicity was shown to be rare. One of the reasons for the unsuccessful trials appears to have been the pronounced irritant sensation caused by agomelatine when administered to the oral mucosa. As a consequence, the FDA decided not to grant approval of the drug in the USA, despite the advantages over other actives in the treatment of MDD.

Several patent applications from Servier indicate that the first approach of providing such novel dosage forms, referred to also as "orodispersible" formulations, e.g. tablets, was directed to achieving a rapid disintegration of a few minutes, such as less than three or even less than one minute(s), assumedly in order to obtain a very fast onset of release and to avoid as good as possible enteral delivery and the associated disadvantage of hepatic first-pass effect. A more recent approach included buccal formulations such as tablets to be sucked, that are intended to dissolve or disintegrate more slowly in the mouth, in order to keep the agomelatine concentration in the oral cavity low enough to limit the stinging sensation. This, however, bears the risk of the tablet being swallowed by the patient prematurely, i.e. before all of the active having been released. Bi- or multilayer tablets comprising e.g. a placebo core have then been proposed so that most part of the active will have been released after some time, leaving only the active-free placebo core, to ensure that active release is near complete even if the patient swallows the tablet prematurely.

Nonetheless, these orodispersible formulations appear not to have solved completely the issue of patient compliance possibly induced by agomelatine causing an irritant sensation, and premature tablet swallowing has not been prevented, but only the effect thereof mitigated. Incomplete active release still is a risk, depending on the timing of unintended tablet swallowing. In addition, sucking a tablet over a prolonged period of time means that the patient has to keep a disturbing object in the oral cavity, which is disadvantageous in terms of patient compliance. Finally, the drug delivery mechanism of such orodispersible formulations depend on the active being first dissolved in the saliva (open system), which is why the drug concentration and in consequence the drug delivery is very difficult to control. All these reasons may have led to currently no agomelatine formulations being on the market besides the conventional oral tablets outlined above.

Transmucosal therapeutic systems, or transmucosal delivery systems (also termed buccal patches by some), consist of one or more thin layers which are applied and adhere to the mucosa of the oral cavity to deliver the drug over a period of time. Dosage forms in the form of thin films for application in the oral cavity are also sometimes referred to as "Oral Thin Film" or OTF, however, OTFs are not necessarily intended to adhere to the mucosa. In a transmucosal therapeutic system, the active is contained in a dissolvable layer, and due to the film adhering to the mucosa, active delivery is achieved by a combination of direct active release from the transmucosal therapeutic system to the mucosa, and by an indirect active delivery *via* dissolution in the saliva, as in the orodispersible formulations outlined above. In order to prevent the indirect active delivery inherent to open systems, a backing layer may be employed, which serves to shield the active-containing layer from the remaining parts of the oral cavity, in particular from the saliva of the environment. In any case are OTFs advantageous when compared to orodispersible formulations intended to be sucked in that they do not provoke any negative sensation of having a disturbing object in the mouth, since the film, adhered to the mucosa, does not freely move around in the oral cavity, and since the film is usually thin enough not to be perceived by the patient once applied.

However, transmucosal therapeutic systems are a relatively new form of drug delivery, meaning that knowledge on formulation technology is limited. Formulating appropriate dosage forms for the transmucosal delivery by OTFs is challenging due to a multitude of aspects to be considered and issues to be solved. The main requirements for such transmucosal therapeutic systems are good adhesion and active permeation, combined with an appropriate behavior and time of disintegration. Also important are haptics, i.e. a good feeling in the mouth, as well as stability of the film (i.e. low friability) before application. The low solubility of agomelatine makes it a substance difficult to formulate, and as outlined above, a key issue is to address the irritating sensation the active substance causes in the oral cavity. In addition, since agomelatine is mainly used for re-synchronizing the circadian rhythm, the desired drug release profile is a rapid initial increase in drug delivery, followed by an only overnight and preferably decreasing release.

Up to date, no commercial agomelatine transmucosal therapeutic system is available, and no research or investigation on such agomelatine transmucosal therapeutic systems is known to the Applicant.

In summary, an alternative administration of agomelatine is much needed, overcoming the disadvantages of the oral as well as sublingual administration routes. As outlined above, a transmucosal therapeutic system would be able to address these disadvantages.

There is thus a need in the art for an agomelatine transmucosal therapeutic system.

### OBJECTS AND SUMMARY OF THE INVENTION

It is an object of the present invention to provide an agomelatine transmucosal therapeutic system as an alternative administration of agomelatine overcoming the above-mentioned disadvantages of current agomelatine administration.

Thus, it is an object of the present invention to provide an agomelatine transmucosal therapeutic system for the transmucosal administration of agomelatine providing a particularly high permeation rate which is thus sufficient for achieving a therapeutically effective dose.

It is a further object of the present invention to provide an agomelatine transmucosal therapeutic system for the transmucosal administration of agomelatine providing a drug release profile with a rapid initial increase and allowing an overnight application, e.g. appropriate for an administration shortly before going to bed.

It is also a further object of the present invention to provide an agomelatine transmucosal therapeutic system for the transmucosal administration of agomelatine which does not provoke an irritating sensation at the mucosa or otherwise in the oral cavity.

Another object of the present invention is to provide an agomelatine transmucosal therapeutic system for the transmucosal administration of agomelatine providing appropriate adhesion to the mucosa, e.g. initially but also over time.

One further object of the present invention is to provide an agomelatine transmucosal therapeutic system for the transmucosal administration of agomelatine providing appropriate disintegration behavior, e.g. in terms of the disintegration time but also in terms of integrity of the transmucosal therapeutic system (no premature falling apart of the layers in case multiple layers are present).

It is another object of the present invention to provide an agomelatine transmucosal therapeutic system for the transmucosal administration of agomelatine, wherein hepatotoxicity and the inter-individual variability is reduced and the bioavailability is increased when compared to oral administration.

It is also one object of the present invention to provide an agomelatine transmucosal therapeutic system for the transmucosal administration of agomelatine providing good haptics, i.e. a good feeling in the mouth.

It is further an object of the present invention to provide an agomelatine transmucosal therapeutic system for the transmucosal administration of agomelatine which complies with the needs of a convenient application and handling in view of size and thickness, provides for good patient compliance and/or which is easy and cost-efficient to manufacture.

These objects and others are accomplished by the present invention, which according to one aspect relates to a transmucosal therapeutic system for the transmucosal administration of agomelatine comprising a mucoadhesive layer structure, said mucoadhesive layer structure comprising:
A) an agomelatine-containing layer comprising:
   i) agomelatine; and
   ii) a dissolvable film-forming agent.

According to certain embodiments of the invention, the transmucosal therapeutic system according to the invention is for use in a method of treatment, preferably for use in a method of treating major depression.

According to other embodiments, the present invention relates to a method of treatment, and in particular a method of treating major depression, including applying a transmucosal therapeutic system according to the invention to the mucosa of a human patient.

According to yet other embodiments, the present invention relates to the use of the inventive transmucosal therapeutic system for the manufacture of a medicament for a treatment, preferably for treating major depression.

According to yet another aspect, the invention relates to a process of manufacture of an agomelatine-containing layer comprising the steps of:
i) combining at least agomelatine and a dissolvable film-forming agent in a solvent to obtain a coating composition;
ii) coating the coating composition onto a release liner; and
iii) drying the coated coating composition to form the agomelatine-containing layer.

According to certain embodiments, the invention also relates to a transmucosal therapeutic system for the transmucosal administration of agomelatine obtainable by such a process of manufacture.

According to certain embodiments, the invention also relates to a transmucosal therapeutic system for the transmucosal administration of agomelatine comprising a mucoadhesive layer structure, said mucoadhesive layer structure comprising at least:
A) an agomelatine-containing layer comprising:
   i) 3 to 10 wt-% agomelatine;
   ii) a dissolvable film-forming agent;
   iii) from 5 to 15 wt-% of a fatty acid;
   iv) from 0.1 to 2 wt-% of one or more sweeteners; and
   v) from 0.2 to 2.0 wt-% of a flavoring agent;
wherein
the dissolvable polymer is selected from the group consisting of polyvinylpyrrolidone and hydroxypropyl cellulose, and
the area weight of the agomelatine-containing layer ranges from 100 to 150 g/m².

Within the meaning of this invention, the term "transmucosal therapeutic system" or "transmucosal delivery system" refers to a system by which the active agent (agomelatine) is administered to the systemic circulation *via* transmucosal delivery by application to the mucosa of the oral cavity, and refers to the entire individual dosing unit that is applied to the mucosa of a patient, and which comprises a therapeutically effective amount of agomelatine in a mucoadhesive layer structure and optionally an additional overlay on top of the agomelatine-containing mucoadhesive layer structure. The mucoadhesive layer structure may be located on a release liner (a detachable protective layer), thus, the transmucosal therapeutic system may further comprise a release liner. Within the meaning of this invention, the term "transmucosal therapeutic system" in particular refers to a system providing passive transmucosal delivery excluding active transport as in methods including microporation. Also, in contrast to certain oral thin films which are not necessarily mucoadhesive and which are intended to disintegrate very fast in the saliva (sometimes referred to as "flash wafers"), enteral delivery is entirely unintended in transmucosal therapeutic systems.

Within the meaning of this invention, the term "agomelatine-containing mucoadhesive layer structure" or "mucoadhesive layer structure containing a therapeutically effective amount of agomelatine" refers to the active agent-containing structure providing the area of release for agomelatine during administration. Any additional overlay adds to the overall size of the transmucosal therapeutic system but does not add to the area of release. The agomelatine-containing mucoadhesive layer structure comprises at least one agomelatine-containing layer.

Within the meaning of this invention, the term "therapeutically effective amount" refers to a quantity of active agent in the transmucosal therapeutic system sufficient to provide, if administered by the transmucosal therapeutic system to a patient, agomelatine blood levels of a similar range (e.g. of about 10 % to about 1000 % as measured as an AUC) when compared to blood levels obtained in a one-time administration of 25 mg oral agomelatine.

Within the meaning of this invention, the terms "active", "active agent", and the like, as well as the term "agomelatine" refer to agomelatine in any pharmaceutically acceptable chemical and morphological form and physical state. These forms include without limitation agomelatine in its free, dissociated or any associated form such as hydrates, solvates and so on, as well as agomelatine in the form of particles which may be in micronized form, crystalline form, and in particular in one of its polymorph forms, and/or in amorphous form, and in any hybrid type form of any of the aforementioned forms or a mixture thereof. The agomelatine, where contained in a medium such as a solvent, may be dissolved or dispersed or in part dissolved and in part dispersed.

When agomelatine is mentioned to be used in a particular form in the manufacture of the transmucosal therapeutic system, this does not exclude interactions between this form of agomelatine and other ingredients of the agomelatine-containing self-adhesive layer structure so that the active is present in another form in the final transmucosal therapeutic system. This means that, even if agomelatine is included in a free, dissociated form, it may be present in the final transmucosal therapeutic system in the form of a hydrate or a solvate, or, if it is included in one of its polymorph forms, it may be present in amorphous form in the final transmucosal therapeutic system. Unless otherwise indicated, in particular the amount of agomelatine in the mucoadhesive layer structure relates to the amount of agomelatine included in the transmucosal therapeutic system during manufacture of the transmucosal therapeutic system and is calculated based on agomelatine in the free form, i.e. when agomelatine is included in an amount of 0.1 mmol, the amount of agomelatine in the self-adhesive layer structure is, within the meaning of the invention, considered to be 24.3 mg (the molecular weight of agomelatine is 243 g/mol), regardless of whether the agomelatine has been included in the transmucosal therapeutic system during manufacture in its free form or in any associated form.

The agomelatine starting material included in the transmucosal therapeutic system during manufacture of the transmucosal therapeutic system may be in the form of particles. Agomelatine may e.g. be present in the mucoadhesive layer structure in the form of particles, dispersed and/or dissolved.

Within the meaning of this invention, the term "particles" refers to a solid, particulate material comprising individual particles, the dimensions of which are negligible compared to the material. In particular, the particles are solid, including plastic/deformable solids, including amorphous and crystalline materials.

Within the meaning of this invention, the term "dispersing" refers to a step or a combination of steps wherein a starting material (e.g. agomelatine) is not totally dissolved. Dispersing in the sense of the invention comprises the dissolution of a part of the starting material (e.g. agomelatine particles), depending on the solubility of the starting material (e.g. the solubility of agomelatine in the coating composition).

There are two main types of transmucosal therapeutic systems, i.e. those using backing layers, and those without. As also outlined in the introductory background section, active delivery of an open system type transmucosal therapeutic system using no backing layer will always be a combination of direct delivery from the transmucosal therapeutic system through the mucosa at the adhesion site, and indirect delivery *via* dissolution of the active from the transmucosal therapeutic system into the saliva, and from the saliva through the mucosa. The proportion of the different delivery routes depends mainly on factors such as the solubility of the active and the disintegration time of the transmucosal therapeutic system. The higher the solubility and the faster disintegration of the transmucosal therapeutic system, dissolution into the saliva will be favored over direct delivery into the mucosa at the adhesion site. Such an indirect delivery has the huge advantage of providing a practical increase by several factors of the mucosal surface area through which the active is released systemically. Dissolution into the saliva on the other hand means that the active concentration, and thus the final delivered amount is difficult to control, and that there may be a risk of enteral delivery by unintended swallowing of the saliva. In particular with respect to agomelatine, this also poses the problem of an active, which has proven to potentially provide irritating sensation, is freely dissolved in the whole oral cavity.

Transmucosal therapeutic systems using a backing layer have a completely different approach, i.e. in such systems, the loss of being restricted in the drug release area (to the actual size of the patch) is accepted in exchange for limiting the delivery route to the direct transmucosal delivery, which can be much better controlled. Thus, in the sense of the present invention, a "backing layer" is any layer within a transmucosal therapeutic system which is able to prevent (at least a substantial amount of) the active contained within the transmucosal therapeutic system to be dissolved into the saliva. Such a backing layer can be non-dissolvable, or dissolvable over time. In the latter case, the time the backing layer takes for dissolution is at least as long as (a substantial amount of) the active takes to be delivered through the mucosa.

In this context, it also becomes clear that terms such as "dissolution", "dissolvable", "dissolve" and the like with respect to any of the layers of a transmucosal therapeutic system (e.g. backing layer, active-containing layer) and with respect to the film-forming agent when casted into a film, are to be understood very broadly, and not in the strict scientific sense of chemically dissolving a molecule in a solvent. Any transformation of the solid state of the layer concerned to a liquid state, such as dispersing, forming of a suspension, gelling of the film and disintegrating into smaller parts of gel, etc. has to be regarded as "dissolving" in the sense of the present invention, as long as the "dissolved" material is able to freely move around in the liquid (e.g. saliva) so that anything that was present below the layer concerned (i.e. the mucosa if a mucosa-contacting layer was dissolved, or e.g. the active-containing layer if a backing layer was dissolved before the active-containing layer) becomes accessible to liquid other than the "dissolved" material. In preferred embodiments, the meaning is limited to the usual chemical sense of dissolving a molecule in a solvent. It should be noted that the term "dissolve" with respect to substances *per se,* such as the active agent agomelatine, or any excipients, will continue to be used in the usual chemical sense of dissolving a molecule in a solvent. E.g., agomelatine in dissolved form obviously does not include agomelatine in dispersed form. The film-forming agent *per se* can be present in the coating composition during manufacture of the transmucosal therapeutic system in dissolved form in the common chemical sense (e.g. is not dispersed, in form of small parts of gel, etc.), but where the film-forming agent is casted into a film, "dissolving" such a film also includes gelling of the film and disintegrating into smaller parts of gel.

The active agent-containing layer is the final, solidified layer e.g. obtained after coating and drying the solvent-containing coating composition. The active agent-containing layer may also be manufactured by laminating two or more such solidified layers (e.g. dried layers) of the same composition to provide the desired area weight. The active agent-containing layer may be mucoadhesive (in the form of a mucoadhesive layer) or the transmucosal therapeutic system may comprise an additional mucosa-contacting layer of a mucoadhesive for providing sufficient adhesion. In particular, the active agent-containing layer is a mucoadhesive layer.

Within the meaning of this invention, the term "mucoadhesive" refers to a material that in particular adheres to and upon contact with a mucosa, but which preferably is non-tacky and can be touched e.g. with the fingers and manipulated, e.g. for application into the oral cavity, without unintentionally adhering to the skin of the fingers, when in dry state. A mucoadhesive layer, when in contact with the mucosa, is "self-adhesive", i.e. provides adhesion to the mucosa so that typically no further aid for fixation is needed. The adhesion strength is preferably strong enough that typical movements in the oral cavity are not sufficient to displace a mucoadhesive layer adhered to the mucosa. A "mucoadhesive" layer structure includes a mucoadhesive layer for mucosa contact which may be provided in the form of a mucoadhesive active agent-containing layer or in the form of an additional layer, i.e. a mucoadhesive mucosa-contacting layer. A mucoadhesive overlay may still be employed to advance adhesion.

Within the meaning of this invention, the term "mucosa-contacting layer" refers to a layer included in the transmucosal therapeutic system to be in direct contact with the mucosa of the patient during administration. When the transmucosal therapeutic systems comprises a mucosa-contacting layer, the other layers do not contact the mucosa and do not necessarily have mucoadhesive properties. The area of release is provided by the area of the active agent-containing layer. A mucosa-contacting layer may be used to enhance adherence. The sizes of an additional mucosa-contacting layer and the active agent-containing layer are usually coextensive and correspond to the area of release.

Within the meaning of this invention, the term "area weight" refers to the dry weight of a specific layer, e.g. of the active agent-containing layer, provided in g/m². The area weight values are subject to a tolerance of ± 10 %, preferably ± 7.5 %, due to manufacturing variability.

If not indicated otherwise "%" refers to weight-%.

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2,000, preferably above 5,000 and more preferably above 10,000 Dalton. Correspondingly, compounds with a molecular weight below 2,000, preferably below 5,000 or more preferably below 10,000 Dalton are usually referred to as oligomers.

Within the meaning of this invention, the term "cross-linking agent" refers to a substance which is able to cross-link functional groups contained within the polymer.

Within the meaning of this invention, the term "mucoadhesive overlay" refers to a mucoadhesive layer, which is located on top of the agomelatine-containing mucoadhesive layer structure, free of active agent, larger in area than the active agent-containing structure and which provides additional area adhering to the mucosa, but no area of release of the active agent. It enhances thereby the overall adhesive properties of the transmucosal therapeutic system.

The transmucosal therapeutic systems according to the present invention can be characterized by certain parameters as measured in an *in vitro* permeation test.

The *in vitro* permeation test is performed with human or animal mucosa and preferably with dermatomed split-thickness pig mucosa with a thickness of 400 µm and an intact barrier function, and with phosphate buffer pH 5.5 or 7.4 as receptor medium (37 °C) with or without addition of a maximum of 40 vol-% organic solvent e.g. ethanol, acetonitrile, isopropanol, dipropylene glycol, PEG 400 so that a receptor medium may e.g. contain 60 vol-% phosphate buffer pH 5.5, 30 vol-% dipropylene glycol and 10 vol-% acetonitrile.

Where not otherwise indicated, the *in vitro* permeation test is performed with dermatomed split-thickness pig mucosa (mucosa oesophagus) with a thickness of 400 µm and an intact barrier function, and with phosphate buffer pH 7.4 as receptor medium (37 °C). The amount of active permeated into the receptor medium is determined in regular intervals using an HPLC method with a UV photometric detector by taking a sample volume. The measured amount of active permeated relates to the amount permeated between the two last sampling points and not the total amount permeated so far.

Thus, within the meaning of this invention, the parameter "permeated amount" is provided in µg/cm² and relates to the amount of active permeated in a sample interval at certain elapsed time per area of release. E.g., in an *in vitro* permeation test as described above, wherein the amount of active permeated into the receptor medium has been e.g. measured at hours 0, 2, 4, 8, 12 and 24, the "permeated amount" of active can be given e.g. for the sample interval from hour 8 to hour 12 and corresponds to the measurement at hour 12.

The permeated amount can also be given as a "cumulative permeated amount", corresponding to the cumulated amount of active permeated at a certain point in time. E.g., in an *in vitro* permeation test as described above, wherein the amount of active permeated into the receptor medium has been e.g. measured at hours 0, 2, 4, 8, 12 and 24, the "cumulative permeated amount" of active at hour 12 corresponds to the sum of the permeated amounts from hour 0 to hour 2, hour 2 to hour 4, hour 4 to hour 8 and hour 8 to hour 12.

Within the meaning of this invention, the parameter "mucosa permeation rate" for a certain sample interval at certain elapsed time is provided in µg/(cm² h) and is calculated from the permeated amount in said sample interval as measured by *in vitro* permeation test as described above in µg/cm², divided by the hours of said sample interval. E.g. the mucosa permeation rate in an *in vitro* permeation test as described above, wherein the amount of active permeated into the receptor medium has been e.g. measured at hours 0, 2, 4, 8, 12 and 24, the "mucosa permeation rate" at hour 12 is calculated as the permeated amount in the sample interval from hour 8 to hour 12 divided by 4 hours.

A "cumulative mucosa permeation rate" can be calculated from the respective cumulative permeated amount by dividing the cumulative permeated amount by the elapsed time. E.g. in an *in vitro* permeation test as described above, wherein the amount of active permeated into the receptor medium has been e.g. measured at hours 0, 2, 4, 8, 12 and 24, the "cumulative mucosa permeation rate" at hour 12 is calculated as the cumulative permeated amount for hour 12 (see above) divided by 12 hours.

Within the meaning of this invention, the above parameters permeated amount and mucosa permeation rate (as well as cumulative permeated amount and cumulative mucosa permeation rate) refer to mean values calculated from 3 *in vitro* permeation test experiments.

The transmucosal therapeutic system according to the present invention can also be characterized by certain parameters as measured in an *in vivo* clinical study.

Within the meaning of this invention, the term "administration" refers to the application of the dosage form, i.e. the transmucosal therapeutic system, to the oral mucosa of the patient, which is then maintained on the mucosa until the agomelatine-containing layer structure is dissolved.

In a typical continuous treatment of MDD, the frequency of drug administration is kept sufficiently high so as to maintain a therapeutically effective blood plasma concentration. The interval between two dosage form administrations, also called dosing interval, needs to be adapted accordingly. Within the meaning of the present invention, the term "dosing interval" refers to the period of time between two consecutive administrations, i.e. the interval between two consecutive points in time a transmucosal therapeutic system is applied to the oral mucosa of the patient. In order to maintain the blood plasma concentration at therapeutic level constantly, the transmucosal therapeutic system would have to be replaced as soon as the active-agent containing layer of the previous transmucosal therapeutic system has dissolved away, or soon after, which time a new transmucosal therapeutic system is applied. In such a mode (constant blood plasma level around the clock), the dosing interval corresponds roughly to the disintegration time of the active agent-containing layer, and may be e.g. 6 hours, 8 hours, or 12 hours. After this period, the active agent-containing layer of the transmucosal therapeutic system has dissolved, any remains (e.g. a non-dissolvable backing layer) is removed from the oral cavity and a new transmucosal therapeutic system is applied. Thus, a dosing interval of 12 hours allows a b.i.d. transmucosal therapeutic system exchange mode in an around-the-clock treatment.

However, for a continuous treatment with agomelatine, the transmucosal therapeutic system will be usually administered once daily (dosing interval of 24 hours), and preferably at bedtime, and the disintegration time will preferably be shorter than the dosing interval. The transmucosal therapeutic system may in particular be applied to the mucosa of the patient shortly (e.g. 5 to 30 minutes) before going to bed in order to account for the delay in drug onset. Since it appears for agomelatine that an around-the-clock maintenance of blood plasma concentration at therapeutic level is not necessary, or may be even contraindicated for re-synchronization of the circadian rhythm, it is not necessary to apply another transmucosal therapeutic system as soon as the active agent-containing layer of the previous transmucosal therapeutic system has dissolved away, e.g. the next morning, so that the patient does not need have a transmucosal therapeutic system applied thereafter, e.g. during the whole daytime.

Within the meaning of this invention, the term "room temperature" refers to the unmodified temperature found indoors in the laboratory where the experiments are conducted and usually lies within 15 to 35 °C, preferably about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated.

Within the meaning of this invention the term "pharmacokinetic parameters" refers to parameters describing the blood plasma curve, e.g. Cₘₐₓ, Cₜ and AUCₜ₁₋ₜ₂ obtained in a clinical study, e.g. by single-dose or multi-dose administration of the agomelatine transmucosal therapeutic system to healthy human subjects. The pharmacokinetic parameters of the individual subjects are summarized using arithmetic and geometric means, e.g. a mean Cₘₐₓ, a mean AUCt and a mean AUC_{INF}, and additional statistics such as the respective standard deviations and standard errors, the minimum value, the maximum value, and the middle value when the list of values is ranked (Median). In the context of the present invention, pharmacokinetic parameters, e.g. the Cₘₐₓ, Ct and AUCₜ₁₋ₜ₂ refer to arithmetic or geometric mean values and preferably refer to geometric mean values. It cannot be precluded that the absolute mean values obtained for a certain transmucosal therapeutic system in a clinical study vary to a certain extent from study to study. To allow a comparison of absolute mean values between studies, a reference formulation, e.g. in the future any product based on the invention, may be used as internal standard. A comparison of the AUC per area of release of the respective reference product in the earlier and later study can be used to obtain a correction factor to take into account differences from study to study.

Clinical studies according to the present invention refer to studies performed in full compliance with the International Conference for Harmonization of Clinical Trials (ICH) and all applicable local Good Clinical Practices (GCP) and regulations.

Within the meaning of this invention, the term "healthy human subject" refers to a male or female subject with a body weight ranging from 55 kg to 100 kg and a body mass index (BMI) ranging from 18 to 29 and normal physiological parameters, such as blood pressure, etc. Healthy human subjects for the purposes of the present invention are selected according to inclusion and exclusion criteria which are based on and in accordance with recommendations of the ICH.

Within the meaning of this invention, the term "subject population" refers to at least ten individual healthy human subjects.

Within the meaning of this invention, the term "geometric mean" refers to the mean of the log transformed data back-transformed to the original scale.

Within the meaning of this invention, the term "arithmetic mean" refers to the sum of all values of observation divided by the total number of observations.

Within the meaning of this invention, the parameter "AUC" corresponds to the area under the plasma concentration-time curve. The AUC value is proportional to the amount of active agent absorbed into the blood circulation in total and is hence a measure for the bioavailability.

Within the meaning of this invention, the parameter "AUCₜ₁₋ₜ₂" is provided in (ng / ml) h and relates to the area under the plasma concentration-time curve from hour t1 to t2 and is calculated by the linear trapezoidal method.

Within the meaning of this invention, the parameter "Cₘₐₓ" is provided in (ng / ml) and relates to the maximum observed blood plasma concentration of the active agent.

Within the meaning of this invention, the parameter "Ct" is provided in (ng / ml) and relates to the blood plasma concentration of the active agent observed at hour t.

Within the meaning of this invention, the parameter "tₘₐₓ" is provided in h and relates to the time point at which the Cₘₐₓ value is reached. In other words, tₘₐₓ is the time point of the maximum observed plasma concentration.

Within the meaning of this invention, the parameter "t_{lag}" is provided in h and relates to the delay between the time of administration (in case of a transmucosal therapeutic system the time when the transmucosal therapeutic system is first applied to the oral mucosa, i.e. t = 0) and the time of appearance of measurable blood plasma concentration. The t_{lag} can be calculated approximatively as the mean arithmetic value of the first point in time when a measurable (i.e. non-zero) active agent blood plasma concentration is obtained or represented by a median value.

Within the meaning of this invention, the term "mean plasma concentration" is provided in (ng / ml) and is a mean of the individual plasma concentrations of active agent, e.g. agomelatine, at each point in time.

Within the meaning of this invention, the term "coating composition" refers to a composition comprising all components of the drug-containing layer in a solvent, which may be coated onto the backing layer or release liner to form the drug-containing layer upon drying.

Within the meaning of this invention, the term "dissolve" in the context of the preparation of the coating composition, e.g. dissolving components of the coating composition such as the active agent, refers to the process of obtaining a solution, which is clear and does not contain any particles, as visible to the naked eye.

Within the meaning of this invention, the term "solvent" refers to any liquid substance, which preferably is a volatile organic liquid such as methanol, ethanol, isopropanol, acetone, ethyl acetate, methylene chloride, hexane, n-heptane, heptanes, toluene and mixtures thereof.

Within the meaning of this invention, and unless otherwise specified, the term "about" refers to an amount that is ± 10 % of the disclosed amount. In some embodiments, the term "about" refers to an amount that is ± 5 % of the disclosed amount. In some embodiments, the term "about" refers to an amount that is ± 2 % of the disclosed amount.

### BRIEF DESCRIPTION OF THE DRAWINGS

Fig. 1a depicts the agomelatine mucosa permeation rate of transmucosal therapeutic systems prepared according to Examples 1a to 1f for hours 0 to 7.
Fig. 1b depicts the utilization of agomelatine of transmucosal therapeutic systems prepared according to Examples 1a to 1f after 7 hours.
Fig. 2a depicts the agomelatine mucosa permeation rate of transmucosal therapeutic systems prepared according to Examples 1a, 2 and 3a for hours 0 to 7.
Fig. 2b depicts the utilization of agomelatine of transmucosal therapeutic systems prepared according to Examples 1a, 2 and 3a after 7 hours.
Fig. 3a depicts the agomelatine mucosa permeation rate of transmucosal therapeutic systems prepared according to Examples 3a to 3d for hours 0 to 6.
Fig. 3b depicts the agomelatine mucosa permeation rate of transmucosal therapeutic systems prepared according to Examples 3b and 3e to 3h for hours 0 to 6.
Fig. 3c depicts the utilization of agomelatine of transmucosal therapeutic systems prepared according to Examples 3a to 3h after 6 hours.
Fig. 4a depicts the agomelatine mucosa permeation rate of transmucosal therapeutic systems prepared according to Examples 4a to 4f for hours 0 to 6.
Fig. 4b depicts the utilization of agomelatine of transmucosal therapeutic systems prepared according to Examples 4a to 4f after 6 hours.
Fig. 5a depicts the agomelatine mucosa permeation rate of transmucosal therapeutic systems prepared according to Examples 4b and 5a to 5d for hours 0 to 6.
Fig. 5b depicts the utilization of agomelatine of transmucosal therapeutic systems prepared according to Examples 4b and 5a to 5d after 6 hours.
Fig. 6a depicts the agomelatine mucosa permeation rate of transmucosal therapeutic systems prepared according to Examples 6a to 6d for hours 0 to 6.
Fig. 6b depicts the utilization of agomelatine of transmucosal therapeutic systems prepared according to Examples 6a to 6d after 6 hours.
Fig. 7a depicts the agomelatine mucosa permeation rate of transmucosal therapeutic systems prepared according to Examples 4b, 6b, 6c, 6d, 7e and 7g for hours 0 to 4.
Fig. 7b depicts the agomelatine mucosa permeation rate of transmucosal therapeutic systems prepared according to Examples 4b, 7a to 7d and 7f for hours 0 to 4.
Fig. 7c depicts the utilization of agomelatine of transmucosal therapeutic systems prepared according to Examples 4b, 6b, 6c, 6d and 7a to 7g after 7 hours.
Fig. 8a depicts the agomelatine blood plasma concentration obtained in an *in vivo* clinical study of the transmucosal therapeutic systems prepared according to Examples 8a to 8c for hours 0 to 8.
Fig. 9a depicts the agomelatine mucosa permeation rate of transmucosal therapeutic systems prepared according to Examples 4b and 9a to 9f for hours 0 to 4.
Fig. 9b depicts the utilization of agomelatine of transmucosal therapeutic systems prepared according to Examples 4b and 9a to 9f after 4 hours.
Fig. 9c depicts the sum of possible degradation substances and the agomelatine amount detected in a storage stability test at 25 °C and 60 % RH as well as 40 °C and 75 % RH at different time points for a TTS prepared according to Example 9a.
Fig. 9d depicts the sum of possible degradation substances and the agomelatine amount detected in a storage stability test at 25 °C and 60 % RH as well as 40 °C and 75 % RH at different time points for a TTS prepared according to Example 9c.

### DETAILED DESCRIPTION

### STRUCTURE OF THE TRANSMUCOSAL THERAPEUTIC SYSTEM

The present invention is related to a transmucosal therapeutic system for the transmucosal administration of agomelatine comprising a mucoadhesive layer structure containing agomelatine.

The mucoadhesive layer structure contains therapeutically effective amounts of agomelatine and comprises an agomelatine-containing layer comprising i) agomelatine and ii) a dissolvable film-forming agent.

Thus, the transmucosal therapeutic system for the transmucosal administration of agomelatine comprises a mucoadhesive layer structure containing a therapeutically effective amount of agomelatine, said mucoadhesive layer structure comprising:
A) an agomelatine-containing layer comprising:
   i) agomelatine; and
   ii) a dissolvable film-forming agent.

The transmucosal therapeutic system of the present invention attempts to achieve a particularly high active delivery in order to ensure that the drug amount delivered is sufficient for enabling a therapeutically effective dose. As outlined in detail in the introductory section, so-called open systems, wherein active delivery is achieved by a combination of direct active release from the transmucosal therapeutic system to the mucosa, and by an indirect active delivery *via* dissolution in the saliva, are particularly advantageous in terms of high active permeation rates.

Thus, in certain embodiments of the present invention, the transmucosal therapeutic system does not comprise a backing layer. As outlined above, a backing layer in the sense of the present invention is any layer within a transmucosal therapeutic system which is able to prevent (at least a substantial amount of) the active contained within the transmucosal therapeutic system to be dissolved into the saliva. Thus, in particular embodiments, the time the backing layer takes for dissolution is at least as long as (a substantial amount of) the active takes to be delivered through the mucosa, e.g. as long as the active-containing layer takes for dissolving. Thus, in certain embodiments, the backing layer does not dissolve in less than 15 minutes, in less than 10 minutes, or in less than 5 minutes upon administration of the transmucosal therapeutic system to a human patient. In some embodiments, the backing layer does not completely dissolve in water, in artificial or natural saliva, or in any other aqueous medium, at 37 °C and 150 rpm, in less than 30 minutes, in less than 15 minutes, or in less than 10 minutes.

In some specific embodiments, the mucoadhesive layer structure may further comprise one or more further layers selected from:
B) a mucosa-contacting layer, and
C) a cosmetic layer.

Thus, in specific embodiments, the mucoadhesive layer structure according to the invention comprises an additional mucosa-contacting layer. In other embodiments, the mucoadhesive layer structure according to the invention does not comprise an additional mucosa-contacting layer. In such embodiments, but also in specific other embodiments, the agomelatine-containing layer may be mucoadhesive. The additional mucosa-contacting layer, if present, is mucoadhesive and provides for (improved) adhesion between the mucoadhesive layer structure and the mucosa of the patient during administration. A mucosa-contacting layer is provided just below the active-agent containing layer, and thus, forms an adhesive layer between the mucosa and the agomelatine-containing layer during administration. In view of convenience of manufacture and restricting the overall patch size, the size of the agomelatine-containing layer and the size of the mucosa-contacting layer are preferably coextensive

As indicated above, the mucoadhesive layer structure may also comprise a cosmetic layer. In another embodiment, the mucoadhesive layer structure does not comprise a cosmetic layer.

In contrast to the mucosa-contacting layer, a cosmetic layer is located on top of the agomelatine-containing layer and is not (necessarily) intended to contact the mucosa.

As a result, in some specific embodiments, the mucoadhesive layer structure may further comprise one or more further layers selected from:
B) a mucosa-contacting layer, and
C) a cosmetic layer,
wherein
the further layers adjoin the agomelatine-containing layer, and
the mucosa-contacting layer and the cosmetic layer, if both are present, adjoin the agomelatine-containing layer on opposite sides.

The cosmetic layer may provide for a decorative means such as coloring or imprinting, or may simply prevent the patient from touching the agomelatine-containing layer during administration of the transmucosal therapeutic system. For such a protective function, it is preferable that the cosmetic layer covers the agomelatine-containing layer completely. Thus, in certain embodiments, the mucoadhesive layer structure further comprises a cosmetic layer, and the size of the agomelatine-containing layer and the size of the cosmetic layer are coextensive, or the cosmetic layer is larger in size than and extends the surface area of the agomelatine-containing layer.

On the other hand, a cosmetic layer is not to be confused with a backing layer. It is not a function of a cosmetic layer and would indeed be undesirable if the cosmetic layer posed an obstacle for the active to be released into the saliva. Thus, a cosmetic layer dissolves fast enough not to hinder the active to be dissolved into the saliva, and in certain embodiments, the cosmetic layer dissolves in water, in artificial or natural saliva, or in any other aqueous medium at 37 °C and 150 rpm, in less than 3 minutes, less than 1 minute, or in less than 30 seconds.

An elegant solution for a transmucosal therapeutic system, in terms of ease of manufacture and also in terms of simplicity, is, however, when the agomelatine-containing layer is the transmucosal therapeutic system itself. In other words, in certain preferred embodiments, a transmucosal therapeutic system of the present invention does neither comprise a mucosa-contacting layer, nor a cosmetic layer, and in particular, the mucoadhesive layer structure may simply consist of the agomelatine-containing layer.

Thus, according to certain embodiments of the invention, the transmucosal therapeutic system may further comprise a mucoadhesive overlay or does not comprise a mucoadhesive overlay, and preferably does not comprise a mucoadhesive overlay. This mucoadhesive overlay is in particular larger than the agomelatine-containing mucoadhesive layer structure and is attached thereto for enhancing the adhesive properties of the overall transmucosal therapeutic system. The area of said mucoadhesive overlay adds to the overall size of the transmucosal therapeutic system but does not add to the area of release. The mucoadhesive overlay comprises a mucoadhesive polymer or a mucoadhesive polymer mixture selected from the group of hydroxyethyl cellulose and hydroxypropyl cellulose, which may be identical to or different from any dissolvable film-forming agent included in the active agent-containing mucoadhesive layer structure.

As outlined also above, a transmucosal therapeutic system consists of one or more thin layers, thus, in certain embodiments, the transmucosal therapeutic system is in the form of a film. Such a film may have a circular, rectangular or square shape.

The film preferably has a certain degree of thickness, as otherwise it will be difficult to incorporate the required amount of active, and as very thin films are not easy to manufacture, in particular with respect to providing an even thickness. Thus, in certain embodiments, the transmucosal therapeutic system is in the form of a thin film having an area weight of at least 25 g/m², preferably at least 35 g/m², or more preferably at least 40 g/m². Or, put into thickness, the transmucosal therapeutic system is in the form of a thin film having a layer thickness of at least 15 µm, preferably at least 25 µm, and more preferably at least 35 µm. On the other hand, very thick films will be perceived by the patient as a disturbing object in the oral cavity, and thus are disadvantageous in terms of patient compliance. Thus, in certain embodiments, the transmucosal therapeutic system is in the form of a thin film having an area weight of less than or equal to 300 g/m², preferably less than or equal to 250 g/m², or more preferably less than or equal to 200 g/m². Or, in terms of thickness, the transmucosal therapeutic system is in the form of a thin film having a layer thickness of less than 550 µm, preferably less than 400 µm, and more preferably less than 300 µm. In preferred embodiments, the transmucosal therapeutic system is in the form of a thin film having an area weight of from 25 to 300 g/m², preferably from 35 to 250 g/m², or more preferably from 40 to 200 g/m², or having a layer thickness from 15 to 550 µm, preferably from 25 to 400 µm, and more preferably from 35 to 300 µm.

The transmucosal therapeutic system according to the invention is normally stored in a seam-sealed pouch without any further means of protection. However, the mucoadhesive layer structure may also be located on a detachable protective layer (release liner) from which it is removed immediately before application to the mucosa of the patient's oral cavity. Thus, the transmucosal therapeutic system may or may not further comprise a release liner. A transmucosal therapeutic system protected by a release liner is usually also stored in a seam-sealed pouch. The packaging may be child resistant and/or senior friendly.

### AGOMELATINE-CONTAINING LAYER

As outlined in more detail above, the transmucosal therapeutic system according to the present invention comprises a mucoadhesive layer structure comprising an agomelatine-containing layer.

Further, the agomelatine-containing layer comprises:
i) agomelatine; and
ii) a dissolvable film-forming agent.

The area weight of the agomelatine-containing layer is one of the factors decisive for the amount of active. A certain thickness is required in order to obtain a sufficient amount of active, and it is also difficult to coat very thin layers in particular with sufficient accuracy. On the other hand, thick layers may not only provoke an uncomfortable feeling in the oral cavity, but are also difficult to manufacture, and may result in the layer taking too long to dissolve for the desired release profile. On balance, it is preferred that the agomelatine-containing layer has an area weight of at least 25 g/m², more preferably at least 35 g/m², or most preferably at least 40 g/m², or has an area weight of less than or equal to 300 g/m², more preferably less than or equal to 250 g/m², or most preferably less than or equal to 200 g/m², or has an area weight of from 25 to 300 g/m², more preferably of from 35 to 250 g/m², or most preferably of from 40 to 200 g/m². In terms of the thickness of the agomelatine-containing layer, it is preferred that the agomelatine-containing layer has a layer thickness of at least 15 µm, preferably at least 25 µm, and more preferably at least 35 µm, or has a layer thickness of less than 550 µm, preferably less than 400 µm, and more preferably less than 300 µm.

In an open system transmucosal therapeutic system comprising no backing layer, which is particularly preferred in the present invention (see above), the active delivery is controlled by a combination of direct and indirect delivery as explained above, which is why in preferred embodiments, the area of release, i.e. the surface area of the agomelatine-containing layer, plays a minor role in the control of the effective dose. However, a certain minimum size is required in order to ensure that the patch does not detach prematurely from the mucosa, and also for being able to include a sufficient amount of active without having to use very thick films. On the other hand, if the area of release is too large, the transmucosal therapeutic system will be huge in size, uncomfortable to apply and to wear, leading to low patient compliance. Considering this, in certain embodiments of the invention, the transmucosal therapeutic system has an area of release of at least 0.1 cm², preferably at least 0.2 cm², or more preferably at least 0.5 cm², or has an area of release of less than or equal to 10 cm², preferably less than or equal to 7 cm², or more preferably less than or equal to 5 cm², or has an area of release of from 0.1 to 10 cm², preferably of from 0.2 to 7 cm², or more preferably of from 0.5 to 5 cm².

As outlined also above and without wishing to be bound by theory, it is believed that a sufficient amount of active agent contained in the transmucosal therapeutic system is necessary to achieve certain advantageous features of the transmucosal therapeutic system according to the present invention, such as good *in vitro* permeation. On the other hand, if the amount of active is too high, this might lead not only to undesirable storage stability issues such as re-crystallization of the active where the agomelatine is present in dissolved form, but also to potential irritating sensations in the oral cavity due to the drug concentration being too high. The amount of agomelatine contained in the transmucosal therapeutic system can be controlled two-way by adjusting concentration and/or the area weight of the agomelatine-containing layer. Details concerning the area weight are outlined above. With respect to the concentration, the agomelatine-containing layer comprises at least 1 wt-% agomelatine, preferably at least 2 wt-% agomelatine, and more preferably at least 3 wt-% agomelatine, or the agomelatine-containing layer comprises less than or equal to 25 wt-% agomelatine, preferably less than or equal to 20 wt-% agomelatine, and more preferably less than or equal to 10 wt-% agomelatine, or the agomelatine-containing layer comprises from 1 to less than or equal to 25 wt-% agomelatine, preferably from 2 to less than or equal to 20 wt-% agomelatine, and more preferably from 3 to less than or equal to 10 wt-% agomelatine.

Thus, in certain embodiments of the invention, the agomelatine-containing layer comprises at least 0.1 mg/cm², preferably at least 0.2 mg/cm², or more preferably at least 0.4 mg/cm² agomelatine, or wherein the agomelatine-containing layer comprises less than or equal to 2.0 mg/cm², preferably less than or equal to 1.5 mg/cm², or more preferably less than or equal to 1.2 mg/cm² agomelatine, or wherein the agomelatine-containing layer comprises from 0.1 to 2.0 mg/cm², preferably from 0.2 to 1.5 mg/cm², or more preferably from 0.4 to 1.2 mg/cm² agomelatine per area of release.

In terms of active amount, the mucoadhesive layer structure may comprise at least 0.1 mg, preferably at least 0.2 mg, or more preferably at least 0.4 mg agomelatine, or less than or equal to 20 mg, preferably less than or equal to 15 mg, or more preferably less than or equal to 10 mg agomelatine, or from 0.1 mg to 20 mg, preferably from 0.2 mg to 15 mg, or more preferably from 0.4 mg to 10 mg agomelatine.

As outlined in more detail above, a correct dissolving behavior of the agomelatine-containing layer is very important for controlling the delivery routes. The faster the disintegration of the transmucosal therapeutic system, the more dissolution into the saliva will be favored over direct delivery into the mucosa at the adhesion site. As it is one of the objects of the present invention to achieve a particularly high permeation rate, indirect delivery, which will be able to make use of the whole mucosa for drug delivery, is very important and to be favored over direct delivery. This means that the transmucosal therapeutic system should disintegrate relatively quickly. Thus, the mucoadhesive layer structure may e.g. dissolve in water, in artificial or natural saliva, or in any other aqueous medium at 37 °C and 150 rpm, in less than 5 hours, preferably in less than 3 hours, and more preferably in less than 2 hours. On the other hand, if dissolving too fast, the risk of unintentionally swallowing a substantial part of the active becomes high. So-called "flash wafers" are films designed to disintegrate very fast in order to achieve enteral delivery, wherein swallowing is intentional (and easier when compared to tablets). Thus, transmucosal therapeutic systems differ from flash wafers by way of its delivery mechanism, and usually take longer than flash wafers to dissolve. In the particular situation of agomelatine as active, high concentrations of active are also undesirable as there is the risk of irritating sensations, which is another reason why the dissolution preferably is not too fast. Thus, the mucoadhesive layer structure may e.g. dissolve in water, in artificial or natural saliva, or in any other aqueous medium at 37 °C and 150 rpm, in more than 30 seconds, preferably in more than 1 minute, and more preferably in more than 2 minutes. In preferred embodiments, the mucoadhesive layer structure dissolves in water, in artificial or natural saliva, or in any other aqueous medium at 37 °C and 150 rpm, in more than 30 seconds and less than 5 hours, preferably in more than 1 minute and less than 3 hours, and more preferably in more than 2 minutes and less than 2 hours.

As it is preferred that the agomelatine-containing layer is able to directly adhere to the mucosa, in certain preferred embodiments of the invention, the agomelatine-containing layer is mucoadhesive.

As will be appreciated further below in more detail, in certain embodiments, it is preferable that the coating composition for preparing the agomelatine-containing layer makes use of ethanol as solvent, but no water. Thus, the agomelatine-containing layer according to the present invention may be obtainable (and/or is obtained) by drying a coated coating composition comprising the agomelatine, the dissolvable film-forming agent, and ethanol. On the other hand, certain dissolvable film-forming agents have a high solubility in water, but limited solubility in other solvents. Thus, there is also an advantage to use water as a solvent, which is why the agomelatine-containing layer may be obtainable (and/or is obtained) by drying a coated coating composition comprising the agomelatine, the dissolvable film-forming agent, and water. A combination of ethanol and water is also possible, i.e. the agomelatine-containing layer may also be obtainable (and/or is obtained) by drying a coated coating composition comprising the agomelatine, the dissolvable film-forming agent, ethanol and water. In terms of the amount of water, the agomelatine-containing layer may be obtainable (and/or is obtained) by drying a coated coating composition comprising less than 50 wt-%, or less than 20 wt-%, or less than 10 wt-%, or less than 5 wt-% water.

Considering the stability of the agomelatine-containing layer with respect to its composition, it is preferable that the agomelatine-containing layer does not comprise any volatile constituents, which bear the risk of evaporating and changing the composition upon storage. Thus, in certain embodiments, the agomelatine-containing layer comprises substantially no volatile solvent. A volatile solvent in this sense may be selected from the group consisting of C1 to C3 linear and branched alcohols, ethyl acetate, hexane, n-heptane, and any mixtures thereof. In view of the transmucosal therapeutic system being applied in the oral cavity, the volatile solvents include particularly those which should better not be digested such as methanol, ethyl acetate, hexane, n-heptane, and mixtures thereof. In particular, the agomelatine-containing layer comprises less than or equal to 5 wt-%, preferably less than or equal to 3 wt-%, and more preferably less than or equal to 1 wt-% volatile solvent.

As agomelatine has a low solubility in water, substantial amounts of water in the agomelatine-containing layer bear the risk of re-crystallization where the active is present in dissolved state. Thus, in certain embodiments, the agomelatine-containing layer comprises substantially no water, e.g. comprises less than or equal to 12 wt-%, less than or equal to 8 wt-%, less than or equal to 5 wt-%, or less than or equal to 4 wt-% water.

### AGOMELATINE

In accordance with the invention, the mucoadhesive layer structure contains agomelatine in a therapeutically effective amount, and the mucoadhesive layer structure comprises an agomelatine-containing layer.

While in accordance with the present invention, the active agent agomelatine may be present in the transmucosal therapeutic system, and in particular in the agomelatine-containing layer in any form, i.e. in its free, dissociated or any associated form such as hydrates, solvates and so on, as well as in the form of particles which may be in micronized form, crystalline form, and in particular in one of its polymorph forms, and/or in amorphous form, and in any hybrid type form of any of the aforementioned forms or a mixture thereof, it is preferred that the agomelatine is present in the free, dissociated form.

Further, in certain embodiments, the agomelatine is included in the agomelatine-containing layer in dissolved form, in dispersed form, in crystalline form, in particular in one of its polymorph forms, in an amorphous form, as a hydrate, a solvate, a hybrid type form of any of the foregoing forms or a mixture thereof.

In certain embodiments, the agomelatine-containing layer is obtainable (and/or is obtained) by incorporating the agomelatine in dissolved form, in dispersed form, in crystalline form, in particular in one of its polymorph forms, in an amorphous form, as a hydrate, a solvate, a hybrid type form of any of the foregoing forms or a mixture thereof.

The agomelatine in the agomelatine-containing layer may be (completely) dissolved, or the agomelatine-containing layer may comprise agomelatine particles, preferably constituted of agomelatine in its free, dissociated form, so that the agomelatine is present in dispersed form. Needless to say, if the agomelatine is present in dispersed form, the agomelatine-containing layer nonetheless may comprise agomelatine also in dissolved form, depending on the solubility of the active in the agomelatine-containing layer (which is e.g. saturated or super-saturated).

In a preferred embodiment, the agomelatine is completely dissolved, e.g. at least 90 mol%, preferably at least 95 mol%, more preferably at least 98 mol% or most preferably at least 99 mol% of the agomelatine in the agomelatine-containing layer is present in dissolved form. It is also preferred that the agomelatine-containing layer is free of agomelatine crystals.

As outlined above, the amount of agomelatine in the transmucosal therapeutic system is believed to be important for a good release of the active, and can be e.g. adjusted by the agomelatine concentration. Thus, in certain embodiments, the concentration of agomelatine in the agomelatine-containing layer ranges from 1 to 25 wt-% agomelatine, preferably from 2 to 20 wt-% agomelatine, and more preferably from 3 to 10 wt-% agomelatine of the agomelatine-containing layer.

In certain embodiments, the agomelatine has a purity of at least 95 %, preferably of at least 98 % and more preferably of at least 99 % as determined by quantitative HPLC. Quantitative HPLC may be performed with Reversed-Phase-HPLC with UV detection. In particular, the following conditions can be used if HPLC is performed isocratically:

| | |
|---|---|
| Column: | RP Octadecyl phase |
| | XTerra RP18 100 mm × 3.9 mm; 3.5 µm or equivalent |
| Mobile phase: | 0.06 molar KH₂PO₄ Buffer/acetonitril (60:40; v:v); pH 2.5 |
| Gradient: | isocratic |
| Flux: | 1.0 ml |
| Injection volume: | 20 µl |
| Column temperature: | 23 °C |
| Wavelength: | 229 nm and 275 nm |
| Run time: | 5 min |

The transmucosal therapeutic systems according to the present invention advantageously show an improved stability in terms of the agomelatine content as well as agomelatine degradation.

Thus, in certain embodiments, the agomelatine-containing layer contains initially (i.e. shortly after manufacture e.g. within one week) an amount of agomelatine of at least 95 %, preferably of at least 97 %, more preferably of at least 98 % and even more preferably of at least 99 % of the theoretical amount of agomelatine included in the agomelatine-containing layer. The theoretical amount of agomelatine is calculated from the agomelatine amount used for the coating composition and the (actual) area weight of the coated and dried agomelatine-containing layer of the tested transmucosal therapeutic system.

The agomelatine-containing layer may also contain initially a total amount of agomelatine-related degradation substances of less than 0.5 %, preferably of less than 0.2 %, more preferably of less than 0.1 % and even more preferably of less than 0.05 %.

In certain other embodiments, the transmucosal therapeutic system according to the present invention are stable upon storage, i.e. they may maintain the initial agomelatine content values or present low amounts of degradation products, as follows:

In one of such embodiments, the agomelatine-containing layer contains, after having been stored at 25 °C and 60 % relative humidity for at least 3 months, preferably at least 6 months, more preferably at least 9 months and most preferably at least 12 months, an amount of agomelatine of at least 95 %, preferably of at least 97 %, more preferably of at least 98 % and even more preferably of at least 99 % of the theoretical amount of agomelatine included in the agomelatine-containing layer.

The agomelatine-containing layer may also contain, after having been stored at 25 °C and 60 % relative humidity for at least 3 months, preferably at least 6 months, more preferably at least 9 months and most preferably at least 12 months, a total amount of agomelatine-related degradation substances of less than 0.5 %, preferably of less than 0.2 %, more preferably of less than 0.1 % and even more preferably of less than 0.05 %.

In one of such embodiments, the agomelatine-containing layer contains, after having been stored at 40 °C / 75 % RH for at least 3 months and preferably at least 6 months, an amount of agomelatine of at least 95 %, preferably of at least 97 %, more preferably of at least 98 % and even more preferably of at least 99 % of the theoretical amount of agomelatine included in the agomelatine-containing layer.

The agomelatine-containing layer may also contain, after having been stored at 40 °C / 75 % RH for at least 3 months and preferably at least 6 months, a total amount of agomelatine - related degradation substances of less than 0.5 %, preferably of less than 0.2 %, more preferably of less than 0.1 % and even more preferably of less than 0.05 %.

The method for determining the agomelatine content and the total amount of agomelatine-related degradation substances, as well as the adhesion force and the peel force is preferably conducted as described for Examples 9a and 9c.

### DISSOLVABLE FILM-FORMING AGENT

As outlined above, the transmucosal therapeutic system according to the present invention comprises a mucoadhesive layer structure comprising an agomelatine-containing layer comprising a dissolvable film-forming agent.

This dissolvable film-forming agent provides for sufficient cohesion of the agomelatine-containing layer as long as it is kept in dry state. According to certain embodiments, the dissolvable film-forming agent may also provide for sufficient adhesion to the mucosa once wet, i.e. when having been brought in contact with the mucosa. In such embodiments, but also in general, dissolvable film-forming agent may be selected from mucoadhesive polymers.

The film-forming agent is the primary control over the dissolution behavior of the agomelatine-containing layer. This is why the film-forming agent is "dissolvable". This means in certain specific embodiments that the dissolvable film-forming agent, if casted into a film having an area weight of from 30 to 100 g/m², or of 50 g/m², dissolves in water, in artificial or natural saliva, or in any other aqueous medium, at 37 °C and 150 rpm, in less than 5 hours, preferably less than 3 hours, more preferably less than 2 hours, and most preferably less than 1 hour. The dissolvable film-forming agent, if casted into a film having an area weight of from 30 to 100 g/m², or of 50 g/m², may also dissolve in water, in artificial or natural saliva, or in any other aqueous medium, at 37 °C and 150 rpm, in more than 5 seconds, preferably more than 30 seconds, more preferably more than 1 minute, and most preferably more than 2 minutes. Notably, the dissolvable film-forming agent, if casted into a film having an area weight of from 30 to 100 g/m², or of 50 g/m², may dissolve in more than 5 seconds and less than 5 hours, preferably in more than 30 seconds and less than 3 hours, more preferably more than 1 minute and less than 2 hours, and most preferably in more than 2 minutes and less than 1 hour.

Film-forming agents which are suitable as the dissolvable film-forming agent in accordance with the invention are e.g. selected from the group consisting of polymers such as polyvinylpyrrolidone (commercially available as Kollidon^{®} 30F from BASF), methyl cellulose (commercially available as Methocel^{®} from Colorcon), ethyl cellulose (commercially available as Ethocel^{®} from Colorcon), hydroxyethyl cellulose (commercially available as Natrosol^{®} 250 L from Ashland Industries), hydroxypropyl cellulose (commercially available as Klucel^{®} from Ashland Industries), hydroxypropylmethyl cellulose (also known as hypromellose, commercially available as Pharmacoat^{®} from Shin-Etsu), carboxymethyl cellulose sodium (uncrosslinked sodium salt of carboxymethyl cellulose also referred to as CMC or carmellose, commercially available as Blanose^{®} from Ashland Industries), polyethylene glycol- polyvinyl acetate- and polyvinylcaprolactame-based graft copolymers (commercially available as Soluplus^{®} from BASF), polyvinyl alcohol (commercially available as Mowiol^{®} 4-88 from Kuraray), polyvinyl alcohol-polyethylene glycol copolymers (commercially available as Kollicoat^{®} IR from BASF), polyvinylpyrrolidone-polyvinylacetate copolymers (also referred to as copovidones and commercially available e.g. as Kollidon^{®} VA64 from BASF), polyethylene oxides, polyethylene glycols, methacrylic acid - methyl methacrylate copolymers (commercially available as Eudragit^{®} L100, Eudragit^{®} L12,5, Eudragit^{®} S100 and Eudragit^{®} S12,5 from Evonik), and methacrylic acid - ethyl methacrylate copolymers (commercially available as Eudragit^{®} L100-55 and Eudragit^{®} L30D55 from Evonik), and natural film-forming agents such as shellac, pectin, gelatine, alginate, pullulan and starch derivatives, and any mixtures thereof.

The dissolvable film-forming agent should be able not only to provide sufficient cohesion to the agomelatine-containing layer, but preferably provides a film that is not tacky in dry state so that the patient is able to touch and manipulate the agomelatine-containing layer, e.g. apply it to the oral mucosa, without the same adhering to the fingers. In addition, since the dissolvable film-forming agent is the primary control over the dissolution behavior of the agomelatine-containing layer which needs to be neither too fast nor too slow, the dissolvable film-forming agent is preferably soluble, dispersible or otherwise disintegrable in aqueous media, specifically in saliva, or, simplified, in water. On the other hand, in terms of ease of manufacture and for allowing a water-free process of manufacture, which is advantageous in terms of avoiding crystals, or re-crystallization of the active agent, film-forming agents that are soluble in other solvents such as C1-C3 alcohols, in particular ethanol, are also preferred. While it is therefore particularly preferable that the film-forming agent is soluble in both, water and ethanol, a high solubility may lead to the agomelatine-containing layer dissolving too quickly. Thus, selecting the film-forming agent is not a simple task.

The inventors have surprisingly found that, in view of the above, polymers such as polyvinylpyrrolidone, methyl cellulose, hydroxyethyl cellulose, hydroxypropyl cellulose, hydroxypropylmethyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof are preferred for the dissolvable film-forming agents. Particularly preferred for the dissolvable film-forming agent are polymers such as polyvinylpyrrolidone, hydroxypropyl cellulose, polyethylene glycol- polyvinyl acetate- and polyvinylcaprolactame-based graft copolymers, polyvinylpyrrolidone-polyvinylacetate copolymers, and any mixtures thereof, and most preferably, the dissolvable film-forming agent is a hydroxypropyl cellulose or a polyvinylpyrrolidone, or a mixture thereof.

Hydroxypropyl cellulose is commercially available from Ashland under the brand name Klucel^{™} and is provided in several grades.

The grades differ from each other by molecular weight MW (as measured by GPC-size exclusion chromatography) and Brookfield viscosity (25 °C, LVF, Moisture Free), and are as follows:
The HF grade has a MW of 1,150,000 and a Brookfield Viscosity of 1500-3000 (1 % in water),
the MF grade has a MW of 850,000 and a Brookfield Viscosity of 4000-6500 (2 % in water),
the GF grade has a MW of 370,000 and a Brookfield Viscosity of 150-400 (2 % in water),
the JF grade has a MW of 140,000 and a Brookfield Viscosity of 150-400 (5 % in water),
the LF grade has a MW of 95,000 and a Brookfield Viscosity of 75-150 (5 % in water),
the EF grade has a MW of 80,000 and a Brookfield Viscosity of 300-600 (10 % in water),
the ELF grade has a MW of 40,000 and a Brookfield Viscosity of 150-300 (10 % in water).

Thus, in certain embodiments, the hydroxypropyl cellulose has a molecular weight (as measured by GPC-size exclusion chromatography) of from 30,000 to 1,500,000, and more preferably, the hydroxypropyl cellulose has a molecular weight (as measured by GPC-size exclusion chromatography) selected from
between 35,000 and 45,000, in particular 40,000
between 75,000 and 85,000, in particular 80,000
between 90,000 and 100,000, in particular 95,000
between 130,000 and 150,000, in particular 140,000
between 350,000 and 400,000, in particular 370,000,
between 800,000 and 900,000, in particular 850,000
between 1,100,000 and 1,200,000, in particular 1,150,000. Particularly preferred is hydroxypropyl cellulose having a molecular weight (as measured by GPC-size exclusion chromatography) of between 75,000 and 85,000, in particular 80,000.

The polyvinylpyrrolidone is preferably a soluble polyvinylpyrrolidone.

The term "soluble polyvinylpyrrolidone" refers to polyvinylpyrrolidone, also known as povidone, which is soluble with more than 10 % in at least ethanol, preferably also in water, diethylene glycol, methanol, n-propanol, 2-propanol, n-butanol, chloroform, methylene chloride, 2-pyrrolidone, macrogol 400, 1,2 propylene glycol, 1,4 butanediol, glycerol, triethanolamine, propionic acid and acetic acid. Examples of polyvinylpyrrolidones which are commercially available include Kollidon^{®} 12 PF, Kollidon^{®} 17 PF, Kollidon^{®} 25, Kollidon^{®} 30 and Kollidon^{®} 90 F supplied by BASF, or povidone K90F. The different grades of Kollidon^{®} are defined in terms of the K-Value reflecting the average molecular weight of the polyvinylpyrrolidone grades. Kollidon^{®} 12 PF is characterized by a K-Value range of 10.2 to 13.8, corresponding to a nominal K-Value of 12. Kollidon^{®} 17 PF is characterized by a K-Value range of 15.3 to 18.4, corresponding to a nominal K-Value of 17. Kollidon^{®} 25 is characterized by a K-Value range of 22.5 to 27.0, corresponding to a nominal K-Value of 25, Kollidon^{®} 30 is characterized by a K-Value range of 27.0 to 32.4, corresponding to a nominal K-Value of 30. Kollidon^{®} 90 F is characterized by a K-Value range of 81.0 to 97.2, corresponding to a nominal K-Value of 90. Preferred Kollidon^{®} grades are Kollidon^{®} 12 PF, Kollidon^{®} 30 and Kollidon^{®} 90 F. For all grades and types of polyvinylpyrrolidone, it is preferred that the amount of peroxides is within certain limits, in particular, the peroxide amount is equal to or less than 500 ppm, more preferably equal to or less than 150 ppm, and most preferably equal to or less than 100 ppm.

Within the meaning of this invention, the term "K-Value" refers to a value calculated from the relative viscosity of polyvinylpyrrolidone in water according to the European Pharmacopoeia (Ph.Eur.) and USP monographs for "Povidone".

Thus, in certain embodiments, the polyvinylpyrrolidone is selected from polyvinylpyrrolidones having a K-Value within a range selected from the group of ranges consisting of
9 to 15, and preferably 10.2 to 13.8,
15 to 20, and preferably 15.3 to 18.4,
20 to 27, and preferably 22.5 to 27.0,
27 to 35, and preferably 27.0 to 32.4, and
75 to 110, and preferably 81.0 to 97.2,
or any mixtures thereof, and more preferably is a polyvinylpyrrolidone having a K-Value within a range of 27.0 to 32.4 or of 81.0 to 97.2, and any mixtures thereof, and most preferably is a polyvinylpyrrolidone having a K-Value within range of 81.0 to 97.2.

In order to be able to provide sufficient cohesion to the agomelatine-containing layer, a certain amount of the dissolvable film-forming agent should be included. Thus, in certain preferred embodiments, the amount of the dissolvable film-forming agent is at least 65 wt-%, more preferably at least 75 wt-%, and most preferably at least 85 wt-%. On the other hand, the amount of the dissolvable film-forming agent may also be less than or equal to 98 wt-%, less than or equal to 94 wt-%, or less than or equal to 90 wt-%. In certain embodiments, the amount of the dissolvable film-forming agent ranges from 65 to 98 wt-%, from 75 to 94 wt-%, or from 80 to 90 wt-% of the agomelatine-containing layer.

Such a film-forming agent may be present as the dissolvable film-forming agent in the agomelatine-containing layer, but may also be contained in an optional overlay.

### FURTHER ADDITIVES

The agomelatine-containing layer of the transmucosal therapeutic system according to the invention may comprise further excipients or additives selected from the group consisting of fatty acids, sweeteners, flavoring agents, colorants, permeation enhancers, solubilizers, plasticizers, humectants, disintegrants, emulsifiers, antioxidants, stabilizers, buffer reagents and further film-forming agents.

Such additives may be present in the agomelatine-containing layer in an amount of from 0.001 to 15 wt-% of the agomelatine-containing layer per additive. In a certain embodiment, the total amount of all additives is from 0.001 to 25 wt-% of the agomelatine-containing layer. Hereinafter, where a range for an amount of a specific additive is given, such a range refers to the amount per individual additive.

It should be noted that in pharmaceutical formulations, the formulation components are categorized according to their physicochemical and physiological properties, and in accordance with their function. This means in particular that a substance or a compound falling into one category is not excluded from falling into another category of formulation component. E.g. a certain polymer can be a crystallization inhibitor but also a tackifier. Some substances may e.g. be a typical softener but at the same time act as a permeation enhancer. The skilled person is able to determine based on his general knowledge in which category or categories of formulation component a certain substance or compound belongs to. In the following, details on the excipients and additives are provided which are, however, not to be understood as being exclusive. Other substances not explicitly listed in the present description may be as well used in accordance with the present invention, and substances and/or compounds explicitly listed for one category of formulation component are not excluded from being used as another formulation component in the sense of the present invention.

In view of the potentially stinging or irritating effect of agomelatine, substances that are able to mask or modify the taste, or which might alleviate the effect of agomelatine, are particularly preferred. For example, researchers have shown that certain fatty acids may reduce capsaicin-induced effects such as pain / itch.

Thus, in certain preferred embodiments, the agomelatine-containing layer further comprises one or more excipients selected from the group consisting of fatty acids, sweeteners, and flavoring agents.

The fatty acid may in particular be a saturated or unsaturated, linear or branched carboxylic acid comprising 4 to 24 carbon atoms, and in particular may be selected from the group consisting of caprylic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid. Particularly preferred are oleic acid or linoleic acid.

In terms of amount, the agomelatine-containing layer preferably comprises one or more fatty acids in an amount of at least 1 wt-%, more preferably at least 3 wt-%, and more preferably at least 4 wt-%. The agomelatine-containing layer may also comprise one or more fatty acids in an amount of less than or equal to 15 wt-%, preferably less than or equal to 12 wt-%, and more preferably less than or equal to 10 wt-%. Finally, the agomelatine-containing layer may also comprise one or more fatty acids in an amount of from 1 to 15 wt-%, preferably of from 3 to 12 wt-%, and more preferably of from 4 to 10 wt-%.

In certain preferred embodiments, the agomelatine-containing layer comprises one or more natural or artificial sweeteners selected from the group consisting of saccharose, glucose, fructose, sorbitol, mannitol, isomalt, maltitol, lactitol, xylitol, erythritol, sucralose, acesulfame potassium, aspartame, cyclamate, neohesperidine, neotame, steviol glycosides, thaumatin and saccharin sodium. Particularly preferably, the agomelatine-containing layer comprises one or more natural or artificial sweeteners selected from the group consisting of saccharose, sucralose and saccharin sodium. In such a particularly preferred embodiment, i.e. wherein the agomelatine-containing layer comprises one or more natural or artificial sweeteners selected from the group consisting of sucralose, saccharose and saccharin sodium, the amount of the sweetener is at least 0.05 wt-%, preferably at least 0.1 wt-% and more preferably at least 0.3 wt-%, and/or is less than or equal to 2.0 wt-%, preferably less than or equal to 1.5 wt-% and more preferably less than or equal to 1.0 wt-%, and/or is from 0.05 to 2.0 wt-%, preferably from 0.1 to 1.5 wt-%, and more preferably from 0.3 to 1.0 wt-% each.

In also preferred embodiments, the agomelatine-containing layer comprises one or more natural or artificial flavoring agents selected from the group consisting of vanillin, methyl salicylate, menthol, manzanate, diacetyl, acetylpropionyl, acetoin, isoamyl acetate, benzaldehyde, cinnamaldehyde, ethyl propionate, methyl anthranilate, limonene, ethyl decadienoate, allyl hexanoate, ethyl maltol, 2,4-dithiapentane, ethylvanillin and eucalyptol as well as flavoring compositions such as peppermint flavor. Vanillin, methyl salicylate, menthol, peppermint flavor and eucalyptol are particularly preferred. In such a particularly preferred embodiment, i.e. wherein the agomelatine-containing layer comprises one or more flavoring agents selected from the group consisting of vanillin, methyl salicylate, menthol, peppermint flavor and eucalyptol, the amount of the flavoring agent is at least 0.1 wt-%, preferably at least 0.3 wt-% and more preferably at least 0.4 wt-%, and/or is less than or equal to 10 wt-%, preferably less than or equal to 6 wt-%, and more preferably less than or equal to 4 wt-%, and/or is from 0.1 to 10 wt-%, preferably from 0.3 to 6 wt-%, and more preferably from 0.4 to 4 wt-% each, or the amount is at least 0.1 wt-%, preferably at least 0.5 wt-% and more preferably at least 0.7 wt-%, and/or is less than or equal to 15 wt-%, preferably less than or equal to 10 wt-%, and more preferably less than or equal to 8 wt-%, and/or is from 0.1 to 15 wt-%, preferably from 0.5 to 10 wt-%, and more preferably from 0.7 to 8 wt-% in total. Suitable flavoring agents are also commercially available from the company Mane, and any of those, which are identified by tonalities such as apple, caramel, chocolate, lemon, mint, etc. can be used as a flavoring agent in the present invention.

In certain embodiments, the agomelatine-containing layer comprises one or more colorants. Any colorant suitable for use in pharmaceutical / food applications can be included, in particular those admitted for use by the US FDA or by the European Agencies EFSA / EMA. Such colorants may be e.g. selected from the group consisting of titanium dioxide, brilliant blue FCF, indigo carmine, fast green FCF, erythrosine, allura red AC, tartrazine and sunset yellow FCF, curcumin, riboflavin, rivoflavin-5'-phosphate, quinoline yellow, orange yellow S, cochineal, carminic acid, azorubine, carmoisine, amaranth, ponceau 4R, cochineal red A, patent blue V, indigotine, chlorophylls, chlorophyllins, copper complexes of chlorophyll and chlorophyllins, green S, plain caramel, caustic sulphite caramel, ammonia caramel, sulphite ammonia caramel, brilliant black BN, black PN, vegetable carbon, brown HT, carotenes, annatto, bixin, norbixin, paprika extract, capsanthian, capsorubin, lycopene, beta-apo-8'-carotenal, lutein, canthaxanthin, beetroot red, betanin, anthocyanins, calcium carbonate, iron oxides and hydroxides, aluminium, silver, gold and litholrubine BK.

The agomelatine-containing layer may comprise one or more further film-forming agents in addition to those disclosed for the dissolvable film-forming agent above. Such a further film-forming agent is different from those disclosed previously for the dissolvable film-forming agent. The one or more further film-forming agents may be comprised in the agomelatine-containing layer in an amount of at least 2 wt-%, preferably at least 5 wt-% and more preferably at least 10 wt-%, and/or in an amount of less than or equal to 40 wt-%, preferably less than or equal to 30 wt-%, and more preferably less than or equal to 25 wt-%, and/or in an amount of from 2 to 40 wt-%, preferably from 5 to 30 wt-%, and more preferably from 10 to 25 wt-% each, and/or in an amount of at least 5 wt-%, preferably at least 15 wt-% and more preferably at least 20 wt-%, or in an amount of less than or equal to 40 wt-%, preferably less than or equal to 30 wt-%, and more preferably less than or equal to 25 wt-%, and/or in an amount of from 5 to 40 wt-%, preferably from 15 to 30 wt-%, and more preferably from 20 to 25 wt-% in total.

In certain embodiments, the agomelatine-containing layer comprises one or more solubilizers. Suitable solubilizers may be e.g. selected from the group consisting of ethoxylated sorbitan esterified with fatty acids such as polyoxyethylene sorbitan monolaurate, polyoxyethylene sorbitan monopalmitate, polyoxyethylene sorbitan monostearate and polyoxyethylene sorbitan monooleate (commercially available as Tween 80 or Polysorbate 80), safflower oleosomes, propanediol and polyethoxylated castor oil. Such solubilizers may be comprised in the agomelatine-containing layer in an amount of at least 0.3 wt-%, preferably at least 0.5 wt-% and more preferably at least 1.0 wt-%, and/or in an amount of less than or equal to 5 wt-%, preferably less than or equal to 4 wt-%, and more preferably less than or equal to 3 wt-%, and/or in an amount of from 0.3 to 5 wt-%, preferably of from 0.5 to 4 wt-%, and more preferably of from 1.0 to 3 wt-% each.

The agomelatine-containing layer may also comprise one or more emulsifiers. Such emulsifiers may for example be selected from the group consisting of soy lecithin, sodium phosphates, mono- and diglycerides of fatty acids, sodium stearoyl lactylate, diacetyl tartaric acid esters of mono- and diglycerides, and polyethoxylated hydrogenated castor oil (commercially available as Chremophor RH 40 from BASF). Emulsifiers may be e.g. comprised in the agomelatine-containing layer in an amount of at least 1 wt-%, preferably at least 3 wt-% and more preferably at least 5 wt-%, and/or in an amount of less than or equal to 25 wt-%, preferably less than or equal to 20 wt-%, and more preferably less than or equal to 15 wt-%, and/or in an amount of from 1 to 25 wt-%, preferably of from 3 to 20 wt-%, and more preferably of from 5 to 15 wt-% each.

In specific embodiments, the agomelatine-containing layer comprises one or more plasticizers. The one or more plasticizer may be selected from the group consisting of mono-, di-, oligo- and polysaccharides and derivatives such as sorbitol (commercially available as Sorbidex^{™} from Cargill), polyethylene glycol, triacetin, triethyl citrate, propylene glycol, glycerol and medium chain triglycerides. The agomelatine-containing layer may comprise one or more plasticizers in an amount of at least 0.5 wt-%, preferably at least 1 wt-% and more preferably at least 5 wt-%, and/or in an amount of less than or equal to 25 wt-%, preferably less than or equal to 20 wt-%, or more preferably less than or equal to 15 wt-%, and/or in an amount of from 0.5 to 25 wt-%, preferably of from 1 to 20 wt-%, and more preferably of from 5 to 15 wt-% each.

The agomelatine-containing layer may also comprise a permeation enhancer. In one embodiment, the agomelatine-containing layer comprises a permeation enhancer selected from the group consisting of diethylene glycol monoethyl ether (transcutol), dipropylene glycol, levulinic acid, 2,5-dimethyl isosorbide (dottisol), lauryl lactate, lactic acid, dimethylethylene urea, N,N-Diethyl-meta-toluamide (DEET), propylene glycol monocaprylate, 2-methoxy-4-(prop-2-en-1-yl)phenol and laurocapram. Such permeation enhancers can be comprised in the agomelatine-containing layer in an amount of at least 1 wt-%, preferably at least 2 wt-% and more preferably at least 5 wt-%, and/or in an amount of less than or equal to 20 wt-%, preferably less than or equal to 15 wt-%, and more preferably less than or equal to 10 wt-%, and/or in an amount of from 1 to 20 wt-%, preferably of from 2 to 15 wt-%, and more preferably of from 5 to 10 wt-% each.

On the other hand, certain compounds which may be regarded as permeation enhancers are not preferable. Thus, in some embodiments, the agomelatine-containing layer does not comprise a permeation enhancer selected from the group consisting of bile acid, bile acid salts, bile acid derivatives, acyl carnitines, sodium dodecylsulfate, dimethylsulfoxide, sodium laurylsulfate, terpenes, cyclodextrins, cyclodextrin derivatives, saponins, saponin derivatives, chitosan, EDTA, citric acid, and salicylates in an amount of more than 5 wt-%, preferably more than 1 wt-%, more preferably more than 0.2 wt-%, and most preferably more than 0.1 wt-%.

The agomelatine-containing layer according to the invention may comprise a pH regulator. Preferably, the pH regulator is selected from mono- and polytropic acids, mono-, di- and triacidic bases, buffer solutions with mixtures of a weak acid and its conjugate base, amine derivatives, inorganic alkali derivatives, polymers with basic and acidic functionality, respectively.

### RELEASE CHARACTERISTICS

The transmucosal therapeutic system in accordance with the invention are designed for transmucosally administering a certain amount of agomelatine to the systemic circulation in particular during night time.

An administration of the inventive transmucosal therapeutic system in general and preferably consists of applying the mucoadhesive layer structure (after removal of an eventually present release liner) to the mucosa of the oral cavity of a human patient and maintaining the same on the mucosa until dissolved. The application site may be buccal, sublingual, gingival or palatal, i.e. in a preferred embodiment, the administration of the transmucosal therapeutic system consists of applying the mucoadhesive layer structure to the buccal, sublingual, gingival or palatal mucosa, and preferably the buccal mucosa of the oral cavity of a human patient and maintaining the same on the mucosa until dissolved.

In specific embodiments of the invention, the transmucosal therapeutic system according to the invention as described above provides a mucosal permeation rate of agomelatine as measured with pig esophagus mucosa of from 10 µg/cm²-hr to 150 µg/cm²-hr after 1 hour.

In certain embodiments, the transmucosal therapeutic system according to the invention provides a cumulative release of agomelatine as measured with pig esophagus mucosa of at least 0.02 mg/cm², preferably at least 0.05 mg/cm² and more preferably at least 0.1 mg/cm², and/or less than or equal to 0.5 mg/cm², preferably less than or equal to 0.4 mg/cm², and more preferably less than or equal to 0.3 mg/cm², and/or of from 0.02 mg/cm² to 0.5 mg/cm², preferably from 0.05 mg/cm² to 0.4 mg/cm², and more preferably from 0.1 mg/cm² to 0.3 mg/cm² over a time period of 8 hours.

### METHOD OF TREATMENT / MEDICAL USE

In accordance with a specific aspect of the present invention, the transmucosal therapeutic system according to the invention is for use in a method of treatment, and in particular in a method of treating a human patient. In accordance with another aspect, the present invention is related to a method of treatment, wherein the transmucosal therapeutic system according to the invention is administered to a human patient. In yet another aspect, the present invention relates to the use of the inventive transmucosal therapeutic system for the manufacture of a medicament for a treatment, preferably for the treatment of a human patient.

The majority of patients, more than 80% of those who suffer from classical mood disorders (major depression, depressive phases of bipolar disorder, or generalized anxiety disorder) show disruptions of the sleep-wake cycle and of the sleep architecture. Characteristically, difficulty falling asleep (increased sleep latency) followed by fitful sleep results in a pronounced daytime sleepiness that further compromises their ability to function properly in daily life, establishing a vicious cycle. As concerns depression, although there are no specific treatment guidelines, available options generally base themselves on the premise that depression and sleep disturbances share a bidirectional relationship and so, the successful treatment of one of the conditions will reciprocally benefit the other.

In recent years it has become increasingly clear that circadian rhythm disruption - maladjustment and dysfunction of the "body clock" that locks core physiological functions such as body temperature and blood pressure, but also very complex neurotransmitter responses, to the time of the day - is a major factor in major depressive disorder that would warrant therapeutic attention. Malfunctioning of the link between the suprachiasmatic nucleus, the pineal gland, and the neurohormone it produces - melatonin - has been suggested as the main cause for these phenomena. Melatonin has been heavily advocated (and marketed) as a "non-photic circadian rhythm resynchronizing agent" to treat jet lag and insomnia associated with shift working, and a lot has been published on the antidepressant and anxiolytic effects of melatonin. Because the oral bioavailability is low for melatonin, synthetic melatonin receptor agonists have been investigated; the most prominent of which is agomelatine.

While agomelatine is approved for the treatment of depression, treatment of other indications such as bipolar disorders, generalized anxiety disorder, Smith-Magenis syndrome, periventricular leukomalacia and OCD has been suggested.

Thus, in certain embodiments, the transmucosal therapeutic system according to the invention is preferably for use in a method of treating major depression. Likewise, in certain other embodiments, the invention is related to a method of treating major depression, wherein the transmucosal therapeutic system according to the invention is administered to a human patient. In yet other embodiments, the present invention relates to the use of the inventive transmucosal therapeutic system for the manufacture of a medicament for treating major depression.

The treatment of depression, or major depression, also called major depressive disorder, may include treatment of conditions such as major depressive episodes, anxiety symptoms, sleep-wake cycle disturbances, daytime sleepiness and insomnia (the majority of MDD patients, i.e. over 80 % suffer from depression combined with insomnia) in depressive / MDD patients. In other embodiments, the treatment in general may also refer to treating bipolar disorder, generalized anxiety disorder, Smith-Magenis syndrome, periventricular leukomalacia, or OCD.

As outlined above, transmucosal delivery avoids the first-pass effect and thus, the transmucosal therapeutic system according to the invention has a lower risk of hepatotoxicity, unlike oral agomelatine dosage forms. Thus, there is no limitation with respect to the patient group to be treated. The treatment includes the treatment of human patients with or without hepatic impairment, including those patients with at least mild or at least moderate hepatic impairment.

Also, in a certain embodiment, treatment with the inventive transmucosal therapeutic system provides a reduction in at least one agomelatine-related side effect relative to an equivalent oral dose of agomelatine. As outlined above, in certain specific embodiments, such an agomelatine-related side effect is hepatotoxicity. Relative to an equivalent oral dose of agomelatine should be understood as a comparison in the incidence and intensity of side effects in a clinical study when using a dose of transmucosal and oral agomelatine that leads substantially to the same blood plasma exposure of agomelatine. The incidence of the at least one agomelatine-related side effect relative to an equivalent oral dose of agomelatine may be reduced by at least about 30 %, preferably at least about 40 %, more preferably at least about 70 % and most preferably at least about 80 %, and/or the intensity of the at least one agomelatine-related side effect relative to an equivalent oral dose of agomelatine may be reduced. The intensity of a side effect can be determined e.g. by classifying the side effects on a scale indicating "mild", "moderate" or "severe" intensity, and a reduction of the intensity can be quantified by comparing the median intensity.

In any of the treatments outlined for the above aspects and embodiments, the transmucosal therapeutic system is preferably administered by applying the mucoadhesive layer structure to the mucosa of the oral cavity of a human patient and maintained on the mucosa until dissolved. In preferred embodiments, the transmucosal therapeutic system is administered by applying the mucoadhesive layer structure to the buccal, sublingual, gingival or palatal mucosa of the oral cavity of a human patient and maintained on the mucosa until dissolved. In yet preferred embodiments, the transmucosal therapeutic system is administered in the evening or at night time before going to bed.

In another embodiment, the transmucosal therapeutic system according to the invention may also be for use in a method of reducing, in a patient, at least one agomelatine-related side effect relative to an equivalent oral dose of agomelatine.

The invention is also related to a method of reducing at least one agomelatine-related side effect in a patient being treated with oral agomelatine therapy, the method comprising
a) discontinuing oral agomelatine therapy; and
b) administering a transmucosal therapeutic system according to the invention to the mucosa of the oral cavity of a human patient, wherein the transmucosal therapeutic system provides a reduction in at least one agomelatine-related side effect relative to an equivalent oral dose of agomelatine.

In such a method, the transmucosal therapeutic system may deliver an amount of agomelatine equivalent to the amount of agomelatine originally provided by the oral agomelatine therapy.

### PROCESS OF MANUFACTURE

The invention further relates to a process of manufacture of an agomelatine-containing layer for use in a transmucosal therapeutic system and a corresponding mucoadhesive layer structure comprising the agomelatine-containing layer and a corresponding transmucosal therapeutic system.

In accordance with the invention, the process of manufacture of an agomelatine-containing layer comprises the steps of:
i) combining at least agomelatine and a dissolvable film-forming agent in a solvent to obtain a coating composition;
ii) coating the coating composition onto a release liner; and
iii) drying the coated coating composition to form the agomelatine-containing layer.

In such a process, suitable dissolvable film-forming agents are the same as those mentioned previously. Thus, in certain embodiments, the dissolvable film-forming agent, if casted into a film having an area weight of 50 g/m², dissolves in water, in artificial or natural saliva, or in any other aqueous medium, at 37 °C and 150 rpm, in less than 5 hours, preferably in less than 3 hours, more preferably in less than 2 hours, and most preferably in less than 1 hour, and/or in more than 5 seconds, preferably in more than 30 seconds, more preferably in more than 1 minute, and most preferably in more than 2 minutes, and/or in more than 5 seconds and less than 5 hours, preferably in more than 30 seconds and less than 3 hours, more preferably in more than 1 minute and less than 2 hours, and most preferably in more than 2 minutes and less than 1 hour.

The dissolvable film-forming agent may also be selected from the group consisting of polymers such as polyvinylpyrrolidone, methyl cellulose, ethyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, methacrylic acid - methyl methacrylate copolymers, and methacrylic acid - ethyl methacrylate copolymers, natural film-forming agents such as shellac, pectin, gelatine, alginate, pullulan and starch derivatives, and any mixtures thereof. The dissolvable film-forming agent preferably is selected from the group consisting of polymers such as polyvinylpyrrolidone, methyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof, and more preferably is selected from the group consisting of polymers such as polyvinylpyrrolidone, hydroxypropyl cellulose, polyethylene glycol- polyvinyl acetate- and polyvinylcaprolactame-based graft copolymers, polyvinylpyrrolidone-polyvinylacetate copolymers, and any mixtures thereof.

In particular embodiments, the dissolvable film-forming agent may be a hydroxypropyl cellulose, preferably a hydroxypropyl cellulose having a molecular weight of from 50,000 to 1,500,000, and more preferably the dissolvable film-forming agent is a hydroxypropyl cellulose having a molecular weight of 80,000, 95,000, 370,000 or 1,150,000.

In other particular embodiments, the dissolvable film-forming agent is a polyvinylpyrrolidone, preferably is selected from soluble polyvinylpyrrolidones, and in particular from polyvinylpyrrolidones having a K-Value within a range selected from the group of ranges consisting of
9 to 15, and preferably 10.2 to 13.8,
15 to 20, and preferably 15.3 to 18.4,
20 to 27, and preferably 22.5 to 27.0,
27 to 35, and preferably 27.0 to 32.4, and
75 to 110, and preferably 81.0 to 97.2.

In this process of manufacture, in step i) the agomelatine may be dissolved or may be dispersed to obtain a coating composition.

Since agomelatine is poorly soluble in water and thus would be at risk of recrystallizing, it is preferred that no water is used. Thus, in a preferred embodiment, the solvent does not comprise water in an amount of more than 5 wt-%, preferably of more than 2 wt-%, more preferably of more than 1 wt-% and most preferably of more than 0.5 wt-%. On the other hand, depending on the solubility of the dissolvable film-forming agent to be used in different solvents, it may be of advantage to use water. Thus, in some embodiments, the solvent comprises water.

Thus, in the above described process, the solvent preferably comprises an alcoholic solvent selected from methanol, ethanol, isopropanol and mixtures thereof, and more preferably, the solvent comprises ethanol, or consists of ethanol.

The preferences for the dissolvable film-forming agent and other constituents of the agomelatine-containing layer are as outlined above. Thus, in one embodiment, step i) consists of combining at least agomelatine, a dissolvable film-forming agent, and one or more excipients selected from the group consisting of fatty acids, sweeteners, and flavoring agents, in a solvent to obtain a coating composition. Also, in step i), the agomelatine may be combined in dissolved form, in dispersed form, in crystalline form, in particular in one of its polymorph forms, in an amorphous form, as a hydrate, a solvate, a hybrid type form of any of the foregoing forms or a mixture thereof.

In step iii), drying is performed preferably in one or more cycles at room temperature and/or at a temperature of from 40 to 90 °C, more preferably of from 60 to 80 °C.

A mucoadhesive layer structure comprising the agomelatine-containing layer and a corresponding transmucosal therapeutic system can be manufactured using the above-outlined process, using further manufacturing steps such as punching out individual transmucosal therapeutic system and packaging, e.g. by sealing in a pouch of a primary packaging material, as known to the skilled person. Such further steps preferably lead to a mucoadhesive layer structure or a transmucosal therapeutic system as described in the previous chapters.

The present invention in particular also relates to agomelatine-containing layers as well as mucoadhesive layer structures and transmucosal therapeutic system obtainable (and/or obtained) by the above-described processes.

### EXAMPLES

The present invention will now be more fully described with reference to the accompanying examples. It should be understood, however, that the following description is illustrative only and should not be taken in any way as a restriction of the invention. Numerical values provided in the examples regarding the amount of ingredients in the composition or the area weight may vary slightly due to manufacturing variability.

### EXAMPLES 1A-1F

### Coating composition

The formulations of the agomelatine-containing coating compositions of Examples 1a to 1f are summarized in Table 1.1 below. The formulations are based on weight percent, as also indicated in Table 1.1.

**Table 1.1**

| **Agomelatine-containing layer** | | | | | | | | |
|---|---|---|---|---|---|---|---|---|
| **Ingredient (Trade Name)** | **Examples 1a - 1d** | | | | **Ex. 1e** | | **Ex. 1f** | |
| | **Amt [g]** | | **Solids [%]** | | **Amt [g]** | **Solids [%]** | **Amt [g]** | **Solids [%]** |
| Agomelatine | 3.73 | | 14.88 | | 0.50 | 9.91 | 1.00 | 19.87 |
| Eucalyptol | 0.14 | | 0.56 | | 0.03 | 0.53 | 0.03 | 0.64 |
| Menthol | 0.25 | | 1.00 | | 0.05 | 1.01 | 0.05 | 1.00 |
| Methyl salicylate | 0.14 | | 0.55 | | 0.03 | 0.58 | 0.03 | 0.58 |
| Novamint Fresh Peppermint | 0.91 | | 3.62 | | 0.18 | 3.61 | 0.18 | 3.57 |
| Kolliphor RH 40 (Cremophor) | 0.50 | | 1.98 | | 0.12 | 2.47 | 0.11 | 2.12 |
| FD&C Red #40 | 0.03 | | 0.10 | | 0.01 | 0.10 | 0.01 | 0.11 |
| Sucralose | 0.13 | | 0.50 | | 0.03 | 0.50 | 0.02 | 0.49 |
| Polyvinylpyrrolidone (Povidone K90) | 18.75 | | 74.81 | | 4.00 | 79.37 | 3.50 | 69.50 |
| Polysorbate 80 (Tween 80) | 0.50 | | 2.01 | | 0.10 | 1.92 | 0.11 | 2.12 |
| Ethanol | 7.65 | | - | | 1.72 | - | 2.09 | - |
| Aqua dest. | 95.12 | | - | | 19.05 | - | 19.07 | - |
| Total | 127.85 | | 100.01 | | 25.82 | 100.00 | 26.20 | 100.00 |

| | **Ex. 1a** | **Ex. 1b** | **Ex. 1c** | **Ex. 1d** | **Ex. 1e** | | **Ex. 1f** | |
|---|---|---|---|---|---|---|---|---|
| Area weight [g/m²] | 57.9 | 53.9 | 34.9 | 72.1 | 50.5 | | 53.4 | |
| Agomelatine content [µg/cm²] | 861.7 | 802.2 | 519.4 | 1073.1 | 500.6 | | 1059.9 | |
| Diecut size [cm²] | 0.798 | | | | | | | |

| **Backing layer** | | | | | | | | |
|---|---|---|---|---|---|---|---|---|
| | | | **Ex. 1a** | **Examples 1b - 1f** | | | | |
| **Ingredient (Trade Name)** | | | | **Amt [g]** | | | **Solids [%]** | |
| Ethyl cellulose N100 | | | | 16.35 | | | 65.17 | |
| Castor Oil | | | | 8.74 | | | 34.83 | |
| Ethanol | | | | 141.70 | | | - | |
| Total | | | | 166.79 | | | 100.0 | |

| | | | **Ex. 1a** | **Ex. 1b** | **Ex. 1c** | **Ex. 1d** | **Ex. 1e** | **Ex. 1f** |
|---|---|---|---|---|---|---|---|---|
| Area weight [g/m²] | | | | 20.9 | 20.8 | 20.8 | 20.9 | 20.9 |
| Diecut size [cm²] | | | 0.798 | | | | | |

### Preparation of a first coating composition (agomelatine-containing layer)

For Examples 1a to 1f, a first beaker was loaded with agomelatine. Approx. a third of the ethanol, Eucalyptol, Menthol, methyl salicylate, Novamint Fresh Peppermint, Kolliphor RH 40, FD&C Red #40, and Sucralose were added and then stirred. The polyvinylpyrrolidone was added under stirring. A second beaker was loaded with aqua dest.. Polysorbate 80 was added and the mixture was then stirred. The remaining ethanol was added under stirring. The content of the two beakers were mixed under stirring to obtain a slightly red-colored solution with visible crystalline precipitation.

### Preparation of a second coating composition (backing layer)

For Examples 1b to 1f, a beaker was loaded with ethanol and then stirred. Ethyl cellulose and castor oil were added under stirring to obtain a slightly opaque mixture.

The resulting backing composition was coated on a polyester film (polyethylene terephthalate film, one side siliconized, 75 µm thickness, which may function as release liner) and dried for approx. 10 min at room temperature and 20 min at 70 °C. The coating thickness gave an area weight of 20.9 g/m² (Ex. 1b, 1e and 1f) and 20.8 g/m² (Ex. 1c and 1d).

The release liner was removed before applying of the backing layer to the agomelatine-containing coating.

### Coating of the first coating composition

The resulting agomelatine-containing first coating composition of Example 1a was coated on a polyester film (polyethylene terephthalate film, one side siliconized, 75 µm thickness, which may function as release liner) and dried for approx. 5 min at room temperature and 10 min at 50 °C (Ex. 1a). For Example 1a, the dried film is the final agomelatine-containing mucoadhesive layer structure.

The coating thickness gave an area weight of 57.9 g/m² (Ex. 1a).

The resulting agomelatine-containing first coating compositions of Examples 1b to 1f were coated on top of the dried backing layer and dried for approx. 5 min at room temperature, 10 min at 50 °C, and 4 min at 90 °C (Ex. 1b and Ex. 1f), for approx. 5 min at room temperature and 10 min at 50 °C (Ex. 1c), for approx. 6 min at room temperature, 12 min at 50 °C, and 4 min at 90 °C (Ex. 1d), and for approx. 5 min at room temperature, 10 min at 50 °C and 2 min at 90 °C (Ex. 1e), respectively.

The coating thickness gave an area weight of 57.9 g/m² (Ex. 1a), 53.9 g/m² (Ex. 1b), 34.9 g/m² (Ex. 1c), 72.1 g/m² (Ex. 1d), 50.5 g/m² (Ex. 1e), and 53.4 g/m² (Ex. 1f), respectively, for the agomelatine-containing layer.

### Preparation of the transmucosal therapeutic system (concerning all examples)

Individual transmucosal therapeutic systems were then punched out from the agomelatine-containing mucoadhesive layer structure. This is of advantage when the OTF, on the basis of its physical properties alone, does not adhere sufficiently to the mucosa and/or when the agomelatine-containing layer, for the purpose of avoiding waste, has pronounced corners (square or rectangular shapes). The transmucosal therapeutic systems are then punched, and the transmucosal therapeutic systems is sealed into pouches of the primary packaging material as conventional in the art, e.g. under protective atmosphere, by flushing with nitrogen gas.

### Measurement of mucosa permeation rate

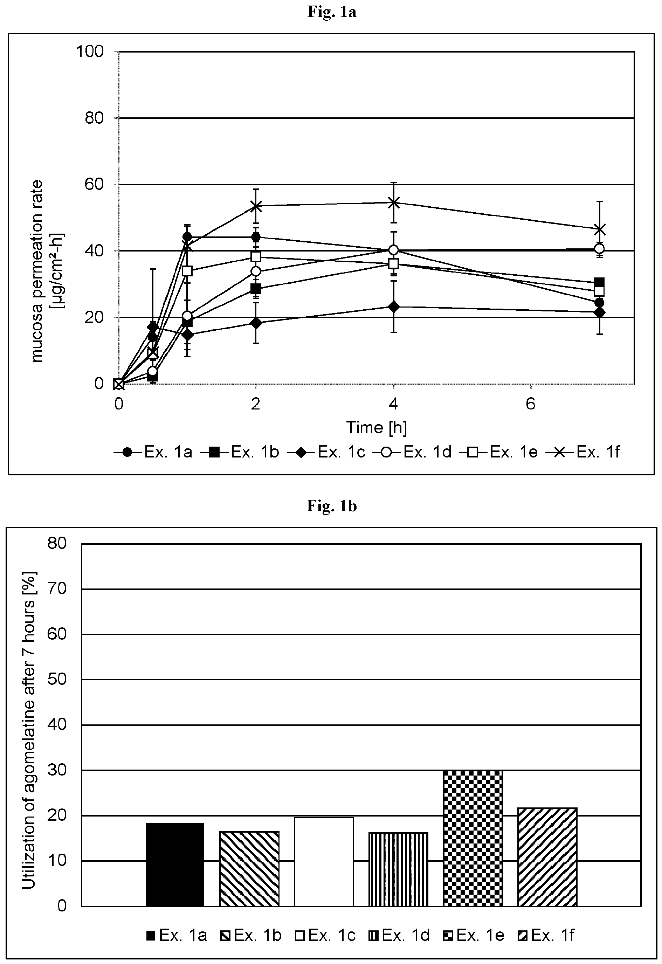

The permeated amount and the corresponding mucosa permeation rates of the transmucosal therapeutic systems prepared according to Examples 1a to 1f were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. Diecuts with an area of 0.798 cm² were punched from the transmucosal therapeutic systems, applied to the mucosa and the mucosa with the transmucosal therapeutic system was immersed on the top side in artificial saliva (the bottom side being in contact with receptor medium, and the top side being compartmentalized to a mucosa area of 1.145 cm²). The agomelatine permeated amount in the receptor medium (phosphate buffer solution pH 7.4) at a temperature of 37 ± 1°C was measured and the corresponding mucosa permeation rate calculated. The results are shown in Table 1.2 and Figure 1a.

**Table 1.2**

| **Mucosa permeation rate with SD [µg/(cm² h)]** | | | | | | | | | | | | |
|---|---|---|---|---|---|---|---|---|---|---|---|---|
| **Elapsed time [h]** | **Ex. 1a (n = 3)** | | **Ex. 1b (n = 3)** | | **Ex. 1c (n = 3)** | | **Ex. 1d (n = 3)** | | **Ex. 1e (n = 3)** | | **Ex. 1f (n = 3)** | |
| | **Rate** | **SD** | **Rate** | **SD** | **Rate** | **SD** | **Rate** | **SD** | **Rate** | **SD** | **Rate** | **SD** |
| **0.5** | 14.02 | 4.73 | 2.38 | 1.21 | 17.16 | 17.48 | 3.82 | 3.40 | 9.01 | 5.28 | 9.70 | 5.31 |
| **1** | 44.31 | 3.17 | 18.75 | 6.54 | 14.81 | 6.54 | 20.48 | 10.03 | 33.98 | 13.92 | 41.55 | 6.54 |
| **2** | 44.19 | 1.36 | 28.61 | 2.84 | 18.38 | 6.11 | 33.80 | 7.49 | 38.20 | 8.85 | 53.56 | 5.11 |
| **4** | 40.28 | 0.59 | 36.20 | 3.07 | 23.34 | 7.76 | 40.29 | 5.51 | 36.19 | 3.54 | 54.64 | 6.04 |
| **7** | 24.45 | 3.26 | 30.39 | 1.39 | 21.61 | 6.57 | 40.66 | 1.95 | 27.84 | 2.08 | 46.53 | 8.42 |

| | | | | | | | | | | | | |
|---|---|---|---|---|---|---|---|---|---|---|---|---|
| *: Standard deviation in this Example was, as in all other Examples, calculated based on the n-method. | | | | | | | | | | | | |

### Utilization of agomelatine

The utilization of agomelatine at 7 hours was calculated based on the cumulative permeated amount at 7 hours and the initial agomelatine content. The results are shown in Table 1.3 and in Figure 1b.

**Table 1.3**

| **Utilization of agomelatine after 7 hours [%]** | | | | | |
|---|---|---|---|---|---|
| **Ex. 1a (n = 3)** | **Ex. 1b (n = 3)** | **Ex. 1c (n = 3)** | **Ex. 1d (n = 3)** | **Ex. 1e (n = 3)** | **Ex. 1f (n = 3)** |
| **18.37** | **16.40** | **19.58** | **16.12** | **30.03** | **21.61** |

The *in vitro* experiments show a good mucosa permeation rate and a good utilization. In particular, it shows that Example 1a without a backing layer has an advantageous fast release of active, when compared to those examples with backing layer. Examples 1b to 1d show that, with increasing area weight (and thus increasing active amount), the permeation rate also increases. Similarly, increasing the active amount by changing the active concentration also leads to an increase in the permeation rate (Examples 1e and 1f).

### EXAMPLE 2

### Preparation of permeation sample

In Example 2, the mucosa permeation rate of pure agomelatine in natural saliva was determined against the transmucosal therapeutic systems of Examples 1a and 3a. Thus, for Example 2, instead of transmucosal therapeutic systems, an agomelatine-containing solution was prepared from 0.877 mg agomelatine in 300 µl natural saliva.

### Measurement of mucosa permeation rate

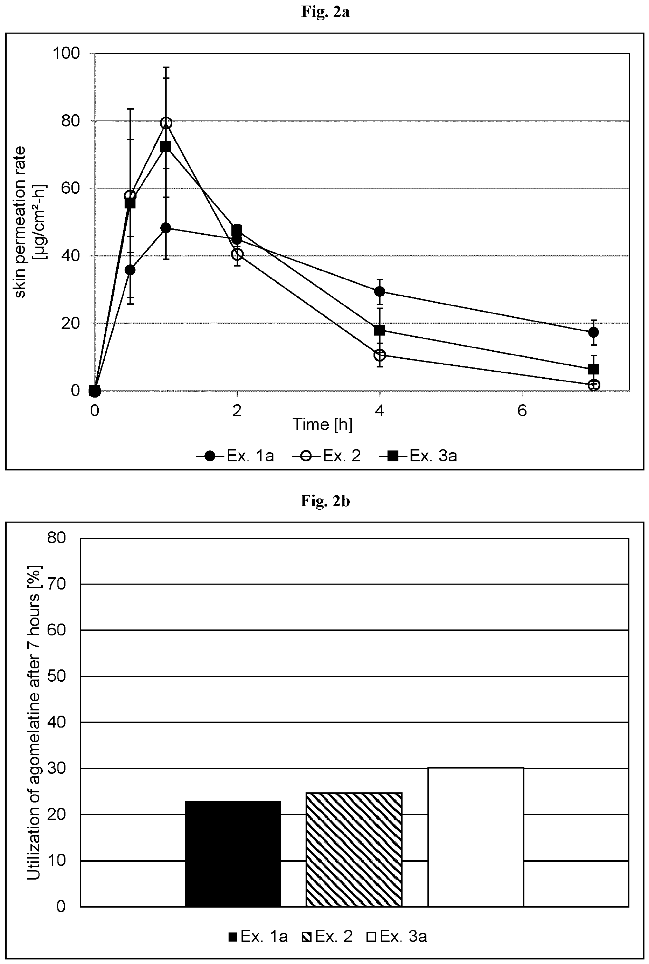

The permeated amount and the corresponding mucosa permeation rates of the transmucosal therapeutic systems prepared according to Examples 1a (containing some crystalline material), 3a (crystal-free) as well as the agomelatine-solution described above (Ex. 2) were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. For Examples 1a and 3a, diecuts with an area of 0.798 cm² were punched from the transmucosal therapeutic systems, and applied to the mucosa. The mucosa with the transmucosal therapeutic system was immersed on the top side in 300 µl natural saliva (the bottom side being in contact with receptor medium, and the top side being compartmentalized to a mucosa area of 1.595 cm²). For Example 2, instead of applying the transmucosal therapeutic system and adding natural saliva, the permeation sample described above was directly applied to the mucosa (also with an area of 1.595 cm²), so that the API content per mucosa area was about 0.55 mg/cm². The agomelatine permeated amount in the receptor medium (phosphate buffer solution pH 7.4) at a temperature of 37 ± 1°C was measured and the corresponding mucosa permeation rate calculated. The results are shown in Table 2.1 and Figure 2a.

**Table 2.1**

| **mucosa permeation rate with SD [µg/(cm² h)]** | | | | | | |
|---|---|---|---|---|---|---|
| **Elapsed time [h]** | **Ex. 1a (n = 3)** | | **Ex. 2 (n = 3)** | | **Ex. 3a (n = 3)** | |
| | **Rate** | **SD** | **Rate** | **SD** | **Rate** | **SD** |
| **0.5** | 35.82 | 9.95 | 57.82 | 16.79 | 55.67 | 27.95 |
| **1** | 48.21 | 9.18 | 79.36 | 13.33 | 72.46 | 23.51 |
| **2** | 44.88 | 2.14 | 40.57 | 3.51 | 47.51 | 1.72 |
| **4** | 29.44 | 3.67 | 10.66 | 3.50 | 18.00 | 6.52 |
| **7** | 17.35 | 3.69 | 1.75 | 1.09 | 6.36 | 4.21 |

| | | | | | | |
|---|---|---|---|---|---|---|
| *: Standard deviation in this Example was, as in all other Examples, calculated based on the n-method. | | | | | | |

### Utilization of agomelatine

The utilization of agomelatine at 7 hours was calculated based on the cumulative permeated amount at 7 hours and the initial agomelatine content. The results are shown in Table 2.2 and in Figure 2b.

**Table 2.2**

| **Utilization of agomelatine after 7 hours [%]** | | |
|---|---|---|
| **Example 1a (n = 3)** | **Example 2 (n = 3)** | **Example 3a (n = 3)** |
| **22.96** | **24.68** | **30.21** |

The *in vitro* experiments show a good mucosa permeation rate and a satisfying utilization.

### EXAMPLES 3A-3H

### Coating composition

The formulations of the agomelatine-containing coating compositions of Examples 3a to 3h are summarized in Tables 3.1 and 3.2 below. The formulations are based on weight percent, as also indicated in Tables 3.1 and 3.2.

**Table 3.1**

| **Agomelatine-containing layer** | | | | | | |
|---|---|---|---|---|---|---|
| **Ingredient (Trade Name)** | **Ex. 3a** | | **Examples 3b, 3c and 3d** | | | |
| | **Amt [g]** | **Solids [%]** | **Amt [g]** | | **Solids [%]** | |
| Agomelatine | 0.50 | 9.96 | 3.50 | | 9.98 | |
| Eucalyptol | 0.04 | 0.83 | 0.17 | | 0.49 | |
| Menthol | 0.05 | 1.00 | 0.35 | | 1.00 | |
| Methyl salicylate | 0.03 | 0.52 | 0.18 | | 0.51 | |
| Novamint Fresh Peppermint | 0.18 | 3.52 | 1.24 | | 3.52 | |
| Kolliphor RH 40 (Cremophor) | 0.12 | 2.43 | 0.72 | | 2.06 | |
| FD&C Red #40 | 0.004 | 0.09 | 0.04 | | 0.10 | |
| Sucralose | 0.03 | 0.50 | 0.18 | | 0.50 | |
| Polyvinylpyrrolidone (Povidone K90) | 4.00 | 79.14 | 27.98 | | 79.80 | |
| Polysorbate 80 (Tween 80) | 0.10 | 2.02 | 0.71 | | 2.03 | |
| Ethanol | 21.89 | - | 155.60 | | - | |
| Total | 26.94 | 100.01 | 190.67 | | 99.99 | |
| Area weight [g/m²] | 55.4 | | 50.0 | | | |
| Agomelatine content [µg/cm²] | 551.6 | | 499.2 | | | |
| Diecut size [cm²] | 0.522 | | | | | |
| | | | | | | |

| **Backing layer** | | | | | | |
|---|---|---|---|---|---|---|
| | **Ex. 3a** | | **Ex. 3b** | **Ex. 3c** | | **Ex.** 3d |
| **Ingredient (Trade Name)** | | | | **Amt [g]** | **Solids [%]** | FO-PET 15µm |
| Ethyl cellulose N50F | | | | 5.22 | 65.09 | |
| Castor Oil | | | | 2.80 | 34.91 | |
| Ethanol | | | | 45.33 | - | |
| Total | | | | 53.35 | 100.00 | |
| Area weight [g/m²] | | | | 12.3 | | |
| Diecut size [cm²] | 0.522 | | | | | |

**Table 3.2**

| **Agomelatine-containing layer** | | | | | | | | |
|---|---|---|---|---|---|---|---|---|
| **Ingredient (Trade Name)** | **Examples 3e** - **3h** | | | | | | | |
| | **Amt [g]** | | | | **Solids [%]** | | | |
| Agomelatine | 3.50 | | | | 9.98 | | | |
| Eucalyptol | 0.17 | | | | 0.49 | | | |
| Menthol | 0.35 | | | | 1.00 | | | |
| Methyl salicylate | 0.18 | | | | 0.51 | | | |
| Novamint Fresh Peppermint | 1.24 | | | | 3.52 | | | |
| Kolliphor RH 40 (Cremophor) | 0.72 | | | | 2.06 | | | |
| FD&C Red #40 | 0.04 | | | | 0.10 | | | |
| Sucralose | 0.18 | | | | 0.50 | | | |
| Polyvinylpyrrolidone (Povidone K90) | 27.98 | | | | 79.80 | | | |
| Polysorbate 80 (Tween 80) | 0.71 | | | | 2.03 | | | |
| Ethanol | 155.60 | | | | - | | | |
| Total | 190.67 | | | | 99.99 | | | |
| Area weight [g/m²] | 50.0 | | | | | | | |
| Agomelatine content [µg/cm²] | 499.2 | | | | | | | |
| Diecut size [cm²] | 0.522 | | | | | | | |
| | | | | | | | | |

| **Backing layer** | | | | | | | | |
|---|---|---|---|---|---|---|---|---|
| | **Ex. 3e** | | **Ex. 3f** | | **Ex. 3g** | | **Ex. 3h** | |
| **Ingredient (Trade Name)** | **Amt [g]** | **Solids [%]** | **Amt [g]** | **Solids [%]** | **Amt [g]** | **Solids [%]** | **Amt [g]** | **Solids [%]** |
| Kollidon VA 64 | 9.29 | 46.36 | 9.31 | 39.63 | 8.40 | 42.11 | 8.50 | 42.50 |
| Eudragit L100-55 | 9.22 | 46.01 | 9.21 | 39.21 | 8.40 | 42.11 | 8.50 | 42.50 |
| Glycerol (99.5% solid content) | 1.54 | 7.63 | 1.51 | 6.39 | 1.55 | 7.72 | 3.02 | 15.00 |
| NaOH 0.1N | - | - | 3.47 | 14.77 | 1.61 | 8.07 | - | - |
| Ethanol | 22.49 | - | 22.53 | - | 22.5 | - | 22.49 | - |
| Aqua purificata | 7.50 | - | 7.55 | - | 7.52 | - | 7.56 | - |
| Total | 50.04 | 100.00 | 53.58 | 100.00 | 49.98 | 100.01 | 50.07 | 100.00 |
| Area weight [g/m²] | 26.8 | | 26.0 | | 20.5 | | 22.9 | |
| pH | 3.75 | | 5.01 | | 4.46 | | 3.62 | |
| Diecut size [cm²] | 0.522 | | | | | | | |

### Preparation of a first coating composition (agomelatine-containing layer)

For Examples 3a, a beaker was loaded with agomelatine. Ethanol, Eucalyptol, Menthol, methyl salicylate, Novamint Fresh Peppermint, Kolliphor RH 40, FD&C Red #40, Sucralose, and Polysorbate 80 were added and the mixture was then stirred. The polyvinylpyrrolidone was added under stirring to obtain a clear, red-colored solution after about 2.5 hour stirring.

For Examples 3b to 3h, the same coating composition was used for the agomelatine-containing layer, which was prepared as follows: a beaker was loaded with agomelatine. Ethanol, Menthol, Eucalyptol, methyl salicylate, Kolliphor RH 40, FD&C Red #40, Sucralose, and Polysorbate 80 were added and the mixture was then stirred. A clear solution was obtained. The polyvinylpyrrolidone was added, and after overnight stirring, the Novamint Fresh Peppermint was added dropwise under stirring to obtain a clear, red-colored solution.

### Preparation of a second coating composition (backing layer)

For Example 3c, a beaker was loaded with ethyl cellulose. Ethanol was added and the mixture was then stirred. Castor oil was added under stirring to obtain a slightly opaque mixture.

For Examples 3e to 3h, a beaker was loaded with ethanol. Aqua purificata, and Kollidon were added and the mixture was then stirred to obtain a solution. Eudragit and Glycerol were added under stirring to obtain a clear mixture. For Examples 3f and 3g, sodium hydroxide solution was added to result in a pH as indicated in table 3.2 above.

The resulting second coating composition of Examples 3c and 3e to 3h was coated on a polyester film (polyethylene terephthalate film, one side siliconized, 75 µm thickness, which may function as release liner) and dried for approx. 10 min at room temperature and 20 min at 70 °C (Ex. 3c), and for approx. 5 min at room temperature, 10 min at 35 °C, and 2 min at 80 °C (Ex. 3e to 3h), respectively. The coating thickness gave an area weight of 12.3 g/m² (Ex. 3c), 26.8 g/m² (Ex. 3e), 26.0 g/m² (Ex. 3f), 20.5 g/m² (Ex. 3g) and 22.9 g/m² (Ex. 3h), respectively. For Example 3d, a commercially available polyethylene terephthalate film with 15 µm thickness was used as a backing layer.

### Coating of the first coating composition

The resulting agomelatine-containing first coating composition of Examples 3a and 3b were coated on a polyester film (polyethylene terephthalate film, one side siliconized, 75 µm thickness, which may function as release liner) and dried for approx. 15 min at room temperature and 5 min at 70 °C (Ex. 3a) or for approx. 5 min at room temperature, 10 min at 50 °C, and 2 min at 90 °C (Ex. 3b), respectively. For Examples 3a and 3b, the dried film is the final agomelatine-containing mucoadhesive layer structure.

The resulting agomelatine-containing first coating compositions of Examples 3c and 3e to 3h were coated on top of the dried backing layer and dried for approx. 5 min at room temperature, 10 min at 50 °C, and 2 min at 90 °C (Ex. 3c and 3e to 3h).

The coating thickness gave an area weight of 55.4 g/m² (Ex. 3a) and 50.0 g/m² (3b, 3c and 3e to 3h), respectively. The coating process of 3d was identical to that of examples 3b, 3c and 3e to 3h, except that the coating composition was coated on a polyethylene terephthalate film of 15 µm thickness, thus giving a transmucosal therapeutic system with a backing layer (of a polyethylene terephthalate film).

### Preparation of the transmucosal therapeutic system

The transmucosal therapeutic systems of Examples 3b, 3c and 3e to 3h were prepared by laminating the backing layers to the agomelatine-containing layers. The release liner was removed before laminating.

The further steps (e.g. punching out individual devices) were conducted as in Example 1.

### Measurement of mucosa permeation rate

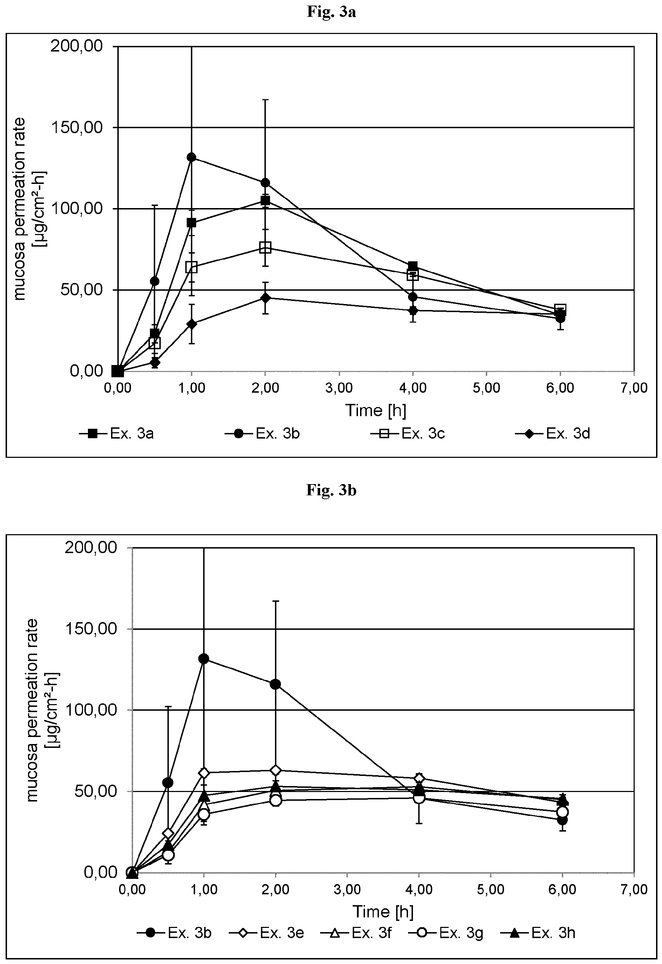

The permeated amount and the corresponding mucosa permeation rates of the transmucosal therapeutic systems prepared according to Examples 3a to 3h were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. Diecuts with an area of 0.522 cm² were punched from the transmucosal therapeutic systems, applied to the mucosa and the mucosa with the transmucosal therapeutic system was immersed on the top side in artificial saliva (the bottom side being in contact with receptor medium, and the top side being compartmentalized to a mucosa area of 1.145 cm²). The agomelatine permeated amount in the receptor medium (phosphate buffer solution pH 7.4) at a temperature of 37 ± 1°C was measured and the corresponding mucosa permeation rate calculated. The results are shown in Tables 3.3 and 3.4 and Figures 3a and 3b.

**Table 3.3**

| **mucosa permeation rate with SD [µg/(cm² h)]** | | | | | | | | |
|---|---|---|---|---|---|---|---|---|
| **Elapsed time [h]** | **Ex. 3a (n = 3)** | | **Ex. 3b (n = 3)** | | **Ex. 3c (n = 3)** | | **Ex. 3d (n = 3)** | |
| | **Rate** | **SD** | **Rate** | **SD** | **Rate** | **SD** | **Rate** | **SD** |
| **0.5** | 23.19 | 5.57 | 55.30 | 46.92 | 17.07 | 6.03 | 5.44 | 3.18 |
| **1** | 91.36 | 7.68 | 131.57 | 84.76 | 63.94 | 8.91 | 29.20 | 12.09 |
| **2** | 105.02 | 3.93 | 115.97 | 51.31 | 75.98 | 11.34 | 45.16 | 9.80 |
| **4** | 64.60 | 0.46 | 45.71 | 15.35 | 59.38 | 0.99 | 37.46 | 2.22 |
| **6** | 34.53 | 1.21 | 32.42 | 6.60 | 37.75 | 1.01 | 35.10 | 3.86 |

| | | | | | | | | |
|---|---|---|---|---|---|---|---|---|
| *: Standard deviation in this Example was, as in all other Examples, calculated based on the n-method | | | | | | | | |

**Table 3.4**

| **mucosa permeation rate with SD [µg/(cm² h)]** | | | | | | | | |
|---|---|---|---|---|---|---|---|---|
| **Elapsed time [h]** | **Ex. 3e (n = 3)** | | **Ex. 3f (n = 3)** | | **Ex. 3g (n = 3)** | | **Ex. 3h (n = 3)** | |
| | **Rate** | **SD** | **Rate** | **SD** | **Rate** | **SD** | **Rate** | **SD** |
| **0.5** | 24.26 | 1.05 | 12.66 | 7.08 | 11.06 | 3.42 | 17.28 | 7.68 |
| **1** | 61.51 | 2.48 | 41.72 | 12.24 | 35.91 | 4.34 | 47.51 | 11.58 |
| **2** | 63.17 | 1.45 | 50.58 | 6.03 | 44.57 | 3.58 | 53.13 | 7.70 |
| **4** | 58.15 | 2.65 | 52.93 | 3.42 | 46.19 | 2.17 | 50.98 | 4.52 |
| **6** | 43.26 | 3.14 | 45.01 | 0.75 | 37.46 | 2.54 | 45.56 | 2.35 |

| | | | | | | | | |
|---|---|---|---|---|---|---|---|---|
| *: Standard deviation in this Example was, as in all other Examples, calculated based on the n-method | | | | | | | | |

### Utilization of agomelatine

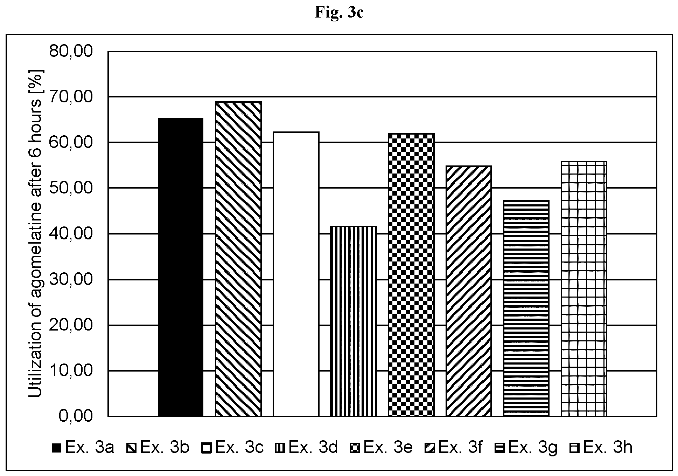

The utilization of agomelatine at 6 hours was calculated based on the cumulative permeated amount at 6 hours and the initial agomelatine content. The results are shown in Table 3.5 and in Figure 3c.

**Table 3.5**

| **Utilization of agomelatine after 6 hours [%]** | | | | | | | |
|---|---|---|---|---|---|---|---|
| **Ex. 3a (n = 3)** | **Ex. 3b (n = 3)** | **Ex. 3c (n = 3)** | **Ex. 3d (n = 3)** | **Ex. 3e (n = 3)** | **Ex. 3f (n = 3)** | **Ex. 3g (n = 3)** | **Ex. 3h (n = 3)** |
| **65.4** | **68.9** | **62.3** | **41.6** | **61.9** | **54.8** | **47.2** | **55.8** |

The *in vitro* experiments show a good mucosa permeation rate and a good utilization.

### EXAMPLES 4A-4F

### Coating composition

The formulations of the agomelatine-containing coating compositions of Examples 4a to 4f are summarized in Tables 4.1 and 4.2 below. The formulations are based on weight percent, as also indicated in Tables 4.1 and 4.2.

**Table 4.1**

| **Agomelatine-containing layer** | | | | | | |
|---|---|---|---|---|---|---|
| **Ingredient (Trade Name)** | **Ex. 4a** | | **Ex. 4b** | | **Ex. 4c** | |
| | **Amt [g]** | **Solids [%]** | **Amt [g]** | **Solids [%]** | **Amt [g]** | **Solids [%]** |
| Agomelatine | 3.50 | 9.96 | 0.50 | 9.94 | 1.00 | 9.99 |
| Eucalyptol | 0.18 | 0.50 | 0.03 | 0.60 | 0.06 | 0.56 |
| Menthol | 0.35 | 1.00 | 0.05 | 1.01 | 0.10 | 1.01 |
| Methyl salicylate | 0.19 | 0.53 | 0.03 | 0.58 | 0.06 | 0.58 |
| Novamint Fresh Peppermint | 1.28 | 3.64 | 0.18 | 3.58 | 0.35 | 3.46 |
| Kolliphor RH 40 (Cremophor) | 0.72 | 2.06 | 0.09 | 1.89 | 0.20 | 1.98 |
| FD&C Red #40 | 0.04 | 0.10 | 0.004 | 0.08 | 0.01 | 0.10 |
| Sucralose | 0.18 | 0.50 | 0.03 | 0.51 | 0.05 | 0.50 |
| Polyvinyl-pyrrolidone (Povidone K90) | 28.00 | 79.69 | - | - | - | - |
| Polysorbate 80 (Tween 80) | 0.71 | 2.02 | 0.12 | 2.43 | 0.20 | 2.02 |
| Hydroxypropyl cellulose | - | - | 4.00 | 79.39 | - | - |
| Soluplus | - | - | - | - | 6.99 | 69.81 |
| Miglyol 812 | - | - | - | - | 1.00 | 9.99 |
| Ethanol | 150.50 | - | 21.51 | - | 15.00 | - |
| Total | 185.65 | 100.00 | 26.54 | 100.01 | 25.02 | 100.00 |
| Area weight [g/m²] | 50.0 | | 48.6 | | 58.5 | |
| Agomelatine content µg/cm²] | 497.6 | | 482.6 | | 584.0 | |
| Diecut size [cm²] | 0.522 | | | | | |

**Table 4.2**

| **Agomelatine-containing layer** | | | | | |
|---|---|---|---|---|---|
| **Ingredient (Trade Name)** | **Examples 4d, 4e and 4f** | | | | |
| | **Amt [g]** | | **Solids [%]** | | |
| Agomelatine | 0.50 | | 9.98 | | |
| Eucalyptol | 0.02 | | 0.49 | | |
| Menthol | 0.05 | | 1.00 | | |
| Methyl salicylate | 0.03 | | 0.57 | | |
| Novamint Fresh Peppermint | 0.17 | | 3.45 | | |
| Kolliphor RH 40 (Cremophor) | 0.10 | | 2.07 | | |
| FD&C Red #40 | 0.01 | | 0.11 | | |
| Sucralose | 0.03 | | 0.51 | | |
| Polyvinyl-pyrrolidone (Povidone K90) | 3.99 | | 79.63 | | |
| Polysorbate 80 (Tween 80) | 0.11 | | 2.19 | | |
| Ethanol | 15.01 | | - | | |
| Total | 20.02 | | 100.00 | | |
| Area weight [g/m²] | 115.4 | | | | |
| Agomelatine content µg/cm²] | 1150.8 | | | | |
| **Diecut** | **Ex. 4d** | **Examples 4e and 4f** | | | |
| Diecut size [cm²] | 0.522 | 0.28 | | | |
| | | | | | |

| **Backing layer** | | | | | |
|---|---|---|---|---|---|
| **Ingredient (Trade Name)** | **Ex. 4d** | **Examples 4e and 4f** | | | |
| | | **Amt [g]** | | **Solids [%]** | |
| Kollidon VA 64 | | 10.63 | | 42.55 | |
| Eudragit L100-5 5 | | 10.60 | | 42.42 | |
| Glycerol (99.5% solid content) | | 3.76 | | 14.97 | |
| FD&C blue No. 1 | | 0.01 | | 0.06 | |
| Ethanol | | 28.11 | | - | |
| Aqua purificata | | 9.44 | | - | |
| Total | | 62.55 | | 100.00 | |
| Area weight [g/m²] | | 47.4 | | | |
| Diecut size [cm²] | | 1.1 | | | |
| | | | | | |

| **Adhesive layer** | | | | | |
|---|---|---|---|---|---|
| **Ingredient (Trade Name)** | **Ex. 4d** | **Ex. 4e** | | **Ex. 4f** | |
| | | **Amt [g]** | **Solids [%]** | **Amt [g]** | **Solids [%]** |
| Hydroxyethyl cellulose | | 3.70 | 73.86 | - | - |
| Hydroxypropyl cellulose | | - | - | 4.01 | 80.00 |
| Menthol | | 0.20 | 4.00 | 0.20 | 4.00 |
| Castor Oil | | 1.00 | 19.95 | 0.80 | 16.01 |
| Polysorbate 80 (Tween 80) | | 0.11 | 2.19 | - | - |
| Ethanol | | 21.25 | - | 20.01 | |
| Aqua purificata | | 7.14 | - | - | - |
| Total | | 33.40 | 100.00 | 25.02 | 100.01 |
| Area weight [g/m²] | | 25.2 | | 23.5 | |

### Preparation of a first coating composition (agomelatine-containing layer)

For Example 4a, a beaker was loaded with agomelatine. Ethanol, Menthol, Eucalyptol, methyl salicylate, Kolliphor RH 49, FD&C Red #40, Sucralose, and Polysorbate 80 were added and the mixture was then stirred. The polyvinylpyrrolidone and Novamint Fresh Peppermint were added under stirring to obtain a viscose mixture.

For Example 4b, a beaker was loaded with Ethanol. Eucalyptol, Menthol, methyl salicylate, Kolliphor RH 49, FD&C Red #40, Sucralose, and Polysorbate 80 were added and the mixture was then stirred. The hydroxypropyl cellulose, Novamint Fresh Peppermint, and agomelatine were added under stirring to obtain a viscose mixture.

For Example 4c, a beaker was loaded with Ethanol. Eucalyptol, Menthol, methyl salicylate, Novamint Fresh Peppermint, Kolliphor RH 49, FD&C Red #40, Sucralose, and Polysorbate 80 were added and the mixture was then stirred. The Soluplus, Miglyol 812, and agomelatine were added under stirring to obtain a mixture with a low viscosity.

For Examples 4d, 4e, and 4f, a beaker was loaded with agomelatine. Ethanol, Eucalyptol, Menthol, methyl salicylate, Novamint Fresh Peppermint, Kolliphor RH 49, FD&C Red #40, Sucralose, and Polysorbate 80 were added and the mixture was then stirred. The polyvinylpyrrolidone was added under stirring to obtain a viscose mixture.

### Preparation of a second coating composition (backing layer)

For Examples 4e and 4f, a beaker was loaded with ethanol. Aqua purificata and FD&C blue No. 1 were added and the mixture was then stirred. Kollidon, Eudragit, and Glycerol were added under stirring to obtain a mixture.

The resulting second coating composition of Examples 4e and 4f was coated on a polyester film (polyethylene terephthalate film, one side siliconized, 75 µm thickness, which may function as release liner) and dried for approx. 5 min at room temperature, 10 min at 35 °C, and 2 min at 80 °C. The coating thickness gave an area weight of 47.4 g/m².

### Preparation of the adhesive composition

For Example 4e, a beaker was loaded with ethanol. Aqua purificata and menthol were added and the mixture was then stirred. Hydroxyethyl cellulose, Castor oil, and Polysorbate 80 were added under stirring to obtain an opaque mixture.

For Example 4f, a beaker was loaded with ethanol. Menthol was added and the mixture was then stirred. Hydroxypropyl cellulose and Castor oil were added under stirring to obtain a viscose mixture.

The resulting adhesive composition of Examples 4e and 4f was coated on top of the backing layer above, and dried for approx. 5 min at room temperature, 10 min at 50 °C, and 2 min at 90 °C. The coating thickness gave an area weight of 25.2 g/m² (Ex. 4e) and 23.5 g/m² (Ex. 4f), respectively. Diecuts with a size of 1.1 cm² were punched from the adhesive backing layer.

### Coating of the first coating composition

The resulting agomelatine-containing coating composition of Examples 4a to 4f was coated on a polyester film (polyethylene terephthalate film, one side siliconized, 75 µm thickness, which may function as release liner) and dried for approx. 5 min at room temperature, 10 min at 50 °C, and 2 min at 90 °C (Ex. 4a to 4c) and approx. 5 min at room temperature, 15 min at 50 °C, and 2 min at 90 °C (Ex. 4d to 4f), respectively. The coating thickness gave an area weight of 49.5 g/m² (Ex. 4a), 48.6 g/m² (Ex. 4b), 58.5 g/m² (Ex. 4c), and 115.4 g/m² (Ex. 4d, Ex. 4e, and Ex. 4f), respectively.

For Examples 4a to 4d, the dried film is the final agomelatine-containing mucoadhesive layer structure. For Examples 4e and 4f, diecuts with a size of 0.28 cm² were punched from the dried film and laminated with the above described diecuts of the adhesive backing layer in a manner so that the backing layer extends evenly to all sides of the agomelatine-containing layer, to provide an agomelatine-containing mucoadhesive layer structure.

### Preparation of the transmucosal therapeutic system

See Example 1.

### Measurement of mucosa permeation rate

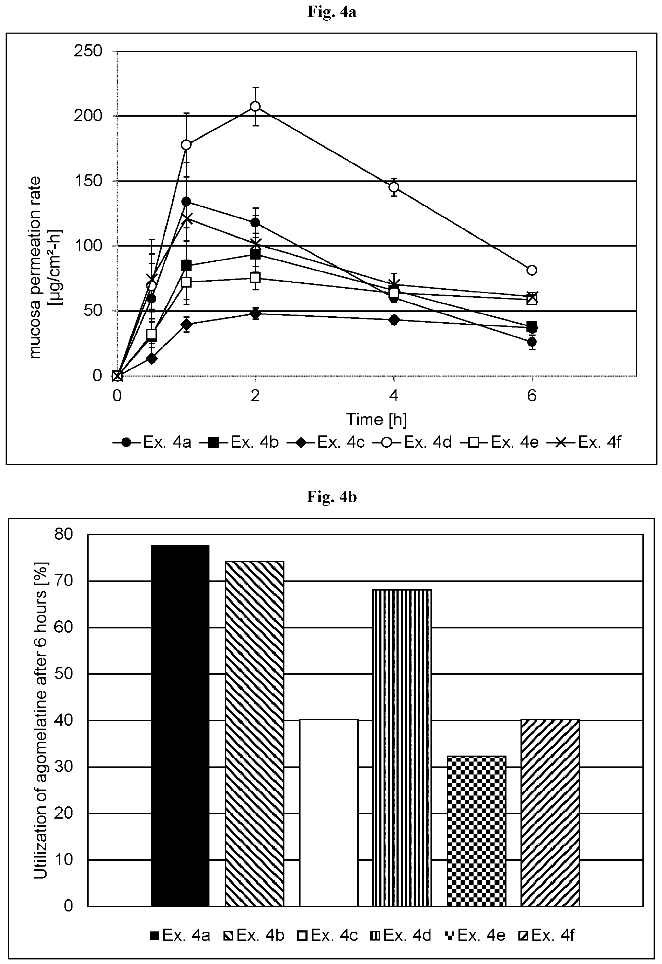

The permeated amount and the corresponding mucosa permeation rates of the transmucosal therapeutic systems prepared according to Examples 4a to 4e were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. Diecuts with an area of 0.522 cm², punched from the transmucosal therapeutic systems of Examples 4a to 4d, as well as the above-described agomelatine-containing mucoadhesive layer structures of Examples 4e and 4f were applied to the mucosa and the mucosa with the transmucosal therapeutic system was immersed on the top side in artificial saliva (the bottom side being in contact with receptor medium, and the top side being compartmentalized to a mucosa area of 1.145 cm²). The agomelatine permeated amount in the receptor medium (phosphate buffer solution pH 7.4) at a temperature of 37 ± 1°C was measured and the corresponding mucosa permeation rate calculated. The results are shown in Table 4.3 and Figure 4a.

**Table 4.3**

| **Mucosa permeation rate with SD [µg/(cm² h)]** | | | | | | | | | | | | |
|---|---|---|---|---|---|---|---|---|---|---|---|---|
| **Elapsed time [h]** | **Ex. 4a (n = 3)** | | **Ex. 4b (n = 3)** | | **Ex. 4c (n = 3)** | | **Ex. 4d (n = 3)** | | **Ex. 4e (n = 3)** | | **Ex. 4f (n = 3)** | |
| | **Rate** | **SD** | **Rate** | **SD** | **Rate** | **SD** | **Rate** | **SD** | **Rate** | **SD** | **Rate** | **SD** |
| **0.5** | 59.41 | 34.53 | 30.02 | 19.57 | 13.61 | 3.06 | 69.09 | 17.66 | 31.79 | 9.93 | 74.49 | 30.52 |
| **1** | 134.26 | 30.32 | 84.56 | 29.46 | 39.64 | 5.78 | 177.76 | 24.57 | 72.03 | 13.17 | 121.30 | 31.96 |
| **2** | 117.90 | 11.26 | 93.72 | 16.24 | 48.10 | 4.40 | 207.27 | 14.61 | 75.41 | 8.86 | 101.78 | 22.07 |
| **4** | 60.05 | 3.29 | 65.82 | 1.90 | 43.23 | 2.98 | 145.18 | 6.82 | 63.91 | 4.45 | 70.31 | 8.51 |
| **6** | 26.01 | 5.60 | 37.78 | 4.21 | 36.91 | 1.35 | 81.17 | 2.38 | 58.47 | 2.78 | 60.94 | 3.18 |

| | | | | | | | | | | | | |
|---|---|---|---|---|---|---|---|---|---|---|---|---|
| *: Standard deviation in this Example was, as in all other Examples, calculated based on the n-method. | | | | | | | | | | | | |

### Utilization of agomelatine

The utilization of agomelatine at 6 hours was calculated based on the cumulative permeated amount at 6 hours and the initial agomelatine content. The results are shown in Table 4.4 and in Figure 4b.

**Table 4.4**

| **Utilization of agomelatine after 6 hours [%]** | | | | | |
|---|---|---|---|---|---|
| **Example 4a (n = 3)** | **Example 4b (n = 3)** | **Example 4c (n = 3)** | **Example 4d (n = 3)** | **Example 4e (n = 3)** | **Example 4f (n = 3)** |
| **77.75** | **74.22** | **40.24** | **68.07** | **32.33** | **40.16** |

The *in vitro* experiments show a good mucosa permeation rate and a satisfying utilization. In particular, the mucosa permeation is surprisingly high in comparison to the same formulations equipped with a backing layer (Example 4d in comparison to Examples 4e and 4f). It also shows that hydroxypropyl cellulose has a similar performance to polyvinylpyrrolidone (see Examples 4a and 4b), which is even better than systems equipped with backing layer, which have over double the active content (Examples 4a and 4b *vs* 4e and 4f).

### EXAMPLES 5A-5D

### Coating composition

The formulations of the agomelatine-containing coating compositions of Examples 4b and 5a to 5d are summarized in Table 4.1 above and Table 5.1 below. The formulations are based on weight percent, as also indicated in Tables 4.1 and 5.1.

**Table 5.1**

| **Agomelatine-containing layer** | | | | | | | | |
|---|---|---|---|---|---|---|---|---|
| **Ingredient (Trade Name)** | **Ex. 5a** | | **Ex. 5b** | | **Ex. 5c** | | **Ex. 5d** | |
| | **Amt [g]** | **Solids [%]** | **Amt [g]** | **Solids [%]** | **Amt [g]** | **Solids [%]** | **Amt [g]** | **Solids [%]** |
| Agomelatine | 0.75 | 5.02 | 0.76 | 5.04 | 2.40 | 20.01 | 0.50 | 9.98 |
| Hydroxypropyl cellulose | 12.43 | 82.86 | 12.44 | 82.83 | - | - | - | - |
| Polyvinyl alcohol, aqueous solution 30.4 % | - | - | - | - | 31.58 | 79.99 | - | - |
| Vanilla flavor | 0.16 | 1.06 | 0.15 | 1.07 | - | - | - | - |
| Sucralose | 0.08 | 0.52 | 0.08 | 0.52 | - | - | 0.03 | 0.51 |
| Saccharin Na | 0.08 | 0.50 | 0.07 | 0.50 | - | - | - | - |
| Oleic acid | 1.49 | 9.94 | 1.49 | 9.95 | - | - | - | - |
| FD&C Yellow #5 | 0.02 | 0.10 | 0.02 | 0.10 | - | - | - | - |
| Ethanol | 48.83 | - | 48.84 | - | - | - | 15.01 | - |
| Total | 63.84 | 100.00 | 63.86 | 100.1 | 33.98 | 100.00 | 20.02 | 100.00 |
| Area weight [g/m²] | 96.5 | | 96.1 | | 82.9 | | 115.35 | |
| Agomelatine content [µg/cm²] | 484.1 | | 484.6 | | 1658.8 | | 1150.8 | |
| Diecut size [cm²] | 0.8 | | 0.8 | | 0.28 | | 0.28 | |
| | | | | | | | | |

| **Backing layer** | | | | | | | | |
|---|---|---|---|---|---|---|---|---|
| **Ingredient (Trade Name)** | **Ex. 5a** | | **Ex. 5b** | | **Ex. 5c** | | **Ex. 5d** | |
| | | | **Amt [g]** | **Solids [%]** | **Amt [g]** | **Solids [%]** | **Amt [g]** | **Solids [%]** |
| Hydroxypropyl cellulose | | | 34.96 | 99.90 | 34.95 | 99.90 | - | - |
| Kollidon VA 64 | | | - | - | - | - | 10.63 | 42.53 |
| Eudragit L100-55 | | | - | - | - | - | 10.60 | 42.41 |
| Glycerol (99.5% solid content) | | | - | - | - | - | 3.77 | 15.00 |
| FD&C blue No. 1 | | | - | - | - | - | 0.01 | 0.05 |
| FD&C Red No. 40 | | | 0.04 | 0.10 | 0.04 | 0.10 | - | - |
| Ethanol | | | - | - | 114.05 | - | 28.11 | - |
| Aqua purificata | | | 113.93 | - | - | - | 9.43 | - |
| Total | | | 148.93 | 100.00 | 149.04 | 100.00 | 62.55 | 99.99 |
| Area weight [g/m²] | | | 105.1 (target area weight) | | 99.6 | | 78.3 | |
| Diecut size [cm²] | | | 0.8 | | 0.8 | | 0.8 | |

| **Adhesive layer** | | | | | | | | |
|---|---|---|---|---|---|---|---|---|
| **Ingredient (Trade Name)** | **Ex. 5a** | **Ex. 5b** | **Ex. 5c** | **Ex. 5d** | | | | |
| | | | | **Amt [g]** | **Solids [%]** | | | |
| Hydroxyethyl cellulose | | | | 14.80 | 73.92 | | | |
| Methanol | | | | 0.80 | 4.00 | | | |
| Castor Oil | | | | 4.00 | 19.98 | | | |
| Polysorbate 80 (Tween 80) | | | | 0.42 | 2.10 | | | |
| Ethanol | | | | 85.00 | - | | | |
| Aqua purificata | | | | 28.34 | - | | | |
| Total | | | | 133.36 | 100.00 | | | |
| Area weight [g/m²] | | | | 95.6 | | | | |

### Preparation of a first coating composition (agomelatine-containing layer)

For Examples 5a and 5b, a beaker was loaded with agomelatine. The Vanilla flavor, ethanol, sucralose, saccharin Na, oleic acid, FD&C Yellow #5, and hydroxypropyl cellulose were added and the mixture was then stirred to obtain a clear mixture.

For Example 5c, a beaker was loaded with agomelatine. Polyvinyl alcohol was added and the mixture was stirred to obtain a white mixture.

### Preparation of a second coating composition (backing layer)

For Examples 5b and 5c, a beaker was loaded with aqua purificata or ethanol, respectively. FD&C red No. 40 and hydroxypropyl cellulose were added under stirring to obtain an opaque mixture.

For Example 5d, a beaker was loaded with ethanol. Aqua purificata and FD&C blue No. 1 were added and the mixture was then stirred. Kollidon, Eudragit, and Glycerol were added under stirring to obtain a mixture.

The resulting second coating composition of Examples 5b, 5c and 5d was coated on a polyester film (polyethylene terephthalate film, one side siliconized, 75 µm thickness, which may function as release liner) and dried for approx. 15 min at 40°C and 15 min at 70°C (Ex. 5b) and approx. 10 min at room temperature, 15 min at 40 °C, and 10 min at 70 °C (Ex. 5c) and approx. 5 min at room temperature, 10 min at 35 °C, and 2 min at 80 °C (Ex. 5d), respectively. The coating thickness gave an area weight of 105.1 g/m² (Ex. 5b), 99.6 g/m² (Ex. 5c) and 78.3 g/m² (Ex. 5d), respectively. For Example 5c, diecuts with a size of 0.8 cm² were punched from the dried backing layer.

### Preparation of the adhesive composition

For Example 5d, a beaker was loaded with ethanol. Aqua purificata and methanol were added and the mixture was then stirred. Hydroxyethyl cellulose, Castor oil, and Polysorbate 80 were added under stirring to obtain a homogeneous mixture.

The resulting adhesive composition of Example 5d was coated on top of the backing layer above, and dried for approx. 5 min at room temperature, 10 min at 50 °C, and 2 min at 90 °C. The coating thickness gave an area weight of 95.6 g/m² (Ex. 5d).

Diecuts with a size of 0.8 cm² were punched from the adhesive backing layer.

### Coating of the first coating composition

The resulting agomelatine-containing coating composition of Examples 5a, 5c and 5d was coated on a polyester film (polyethylene terephthalate film, one side siliconized, 75 µm thickness, which may function as release liner) and dried for approx. 10 min at room temperature, 15 min at 40 °C, and 10 min at 70 °C (Ex. 5a) and 5 min at 70 °C (Ex. 5c), and for approx. 5 min at room temperature, 15 min at 50 °C, and 2 min at 90 °C (Ex. 5d), respectively. For Example 5a, the dried film is the final agomelatine-containing mucoadhesive layer structure. For Examples 5c and 5d, diecuts with a size of 0.28 cm² were punched from the dried film and laminated with the above described diecuts of the backing layer or of the adhesive backing layer in a manner so that the backing layer extends evenly to all sides of the agomelatine-containing layer, to provide an agomelatine-containing mucoadhesive layer structure.

The resulting agomelatine-containing first coating composition of Example 5b was coated on top of the dried backing layer and dried for approx. 10 min at room temperature, 15 min at 40 °C, and 10 min at 70 °C.

The coating thickness gave an area weight of 95.7 g/m² (Ex. 5a), 95.9 g/m² (Ex. 5b), 82.9 g/m² (Ex. 5c), and 115.4 g/m² (5d), respectively.

### Preparation of the transmucosal therapeutic system

See Example 1.

### Measurement of mucosa permeation rate

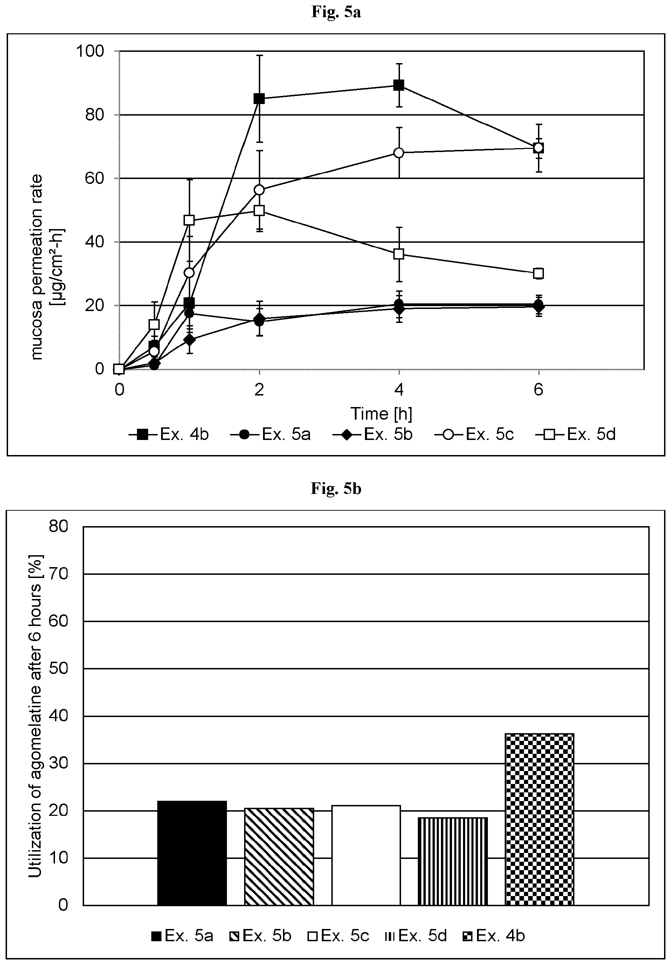

The permeated amount and the corresponding mucosa permeation rates of the transmucosal therapeutic systems prepared according to Examples 4b as well as 5a to 5d were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. Diecuts with an area of 0.28 cm² (Example 4b) and 0.8 cm² (Examples 5a and 5b), punched from the transmucosal therapeutic systems, as well as the above-described agomelatine-containing mucoadhesive layer structures of Examples 5c and 5d were applied to the mucosa and the mucosa with the transmucosal therapeutic system was immersed on the top side in artificial saliva (the bottom side being in contact with receptor medium, and the top side being compartmentalized to a mucosa area of 1.145 cm²). The agomelatine permeated amount in the receptor medium (phosphate buffer solution pH 7.4) at a temperature of 37 ± 1°C was measured and the corresponding mucosa permeation rate calculated. The results are shown in Table 5.2 and Figure 5a.

**Table 5.2**

| **Mucosa permeation rate with SD [µg/(cm² h)]** | | | | | | | | | | |
|---|---|---|---|---|---|---|---|---|---|---|
| **Elapsed time [h]** | **Ex. 5a (n = 6)** | | **Ex. 5b (n = 6)** | | **Ex. 5c (n = 6)** | | **Ex. 5d (n = 6)** | | **Ex. 4b (n = 6)** | |
| | **Rate** | **SD** | **Rate** | **SD** | **Rate** | **SD** | **Rate** | **SD** | **Rate** | **SD** |
| **0.5** | 1.24 | 0.75 | 1.99 | 1.14 | 5.57 | 3.60 | 14.07 | 7.15 | 7.16 | 3.22 |
| **1** | 17.60 | 4.93 | 9.30 | 4.34 | 30.32 | 11.46 | 46.82 | 12.84 | 20.71 | 9.10 |
| **2** | 14.90 | 4.27 | 15.97 | 5.43 | 56.44 | 12.31 | 49.92 | 6.60 | 85.08 | 13.62 |
| **4** | 20.49 | 4.18 | 19.03 | 4.24 | 68.05 | 7.98 | 36.13 | 8.51 | 89.33 | 6.76 |
| **6** | 20.45 | 2.94 | 19.67 | 2.96 | 69.60 | 7.40 | 30.10 | 1.68 | 69.51 | 3.10 |

| | | | | | | | | | | |
|---|---|---|---|---|---|---|---|---|---|---|
| *: Standard deviation in this Example was, as in all other Examples, calculated based on the n-method. | | | | | | | | | | |

### Utilization of agomelatine

The utilization of agomelatine at 6 hours was calculated based on the cumulative permeated amount at 8 hours and the initial agomelatine content. The results are shown in Table 5.3 and in Figure 5b.

**Table 5.3**

| **Utilization of agomelatine after 6 hours [%]** | | | | |
|---|---|---|---|---|
| **Example 5a (n = 6)** | **Example 5b (n = 6)** | **Example 5c (n = 6)** | **Example 5d (n = 6)** | **Example 4b (n = 6)** |
| **21.94** | **20.49** | **21.08** | **18.51** | **36.23** |

The *in vitro* experiments show a good mucosa permeation rate and a satisfying utilization. While all formulations show good permeation behavior, comparing Example 4b (area of release 0.28 cm², mucosa area 1.145 cm²) to Examples 5a and 5b (area of release 0.8 cm², mucosa area 1.145 cm²) demonstrate the important role of total mucosa area available for permeation, in open systems.

### EXAMPLES 6A-6D

### Coating composition

The formulations of the agomelatine-containing coating compositions of Examples 6a to 6d are summarized in Table 6.1 below. The formulations are based on weight percent, as also indicated in Table 6.1.

**Table 6.1**

| **Ingredient (Trade Name)** | **Ex. 6a** | | **Ex. 6b** | | **Ex. 6c** | | **Ex. 6d** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| Agomelatine | 0.30 | 5.00 | 0.30 | 5.00 | 0.30 | 5.00 | 0.75 | 5.00 |
| Hydroxypropyl cellulose | 5.58 | 92.98 | 5.28 | 87.94 | 4.98 | 83.00 | - | - |
| Povidone K90 | - | - | - | - | - | - | 12.30 | 82.02 |
| Vanilla flavor | 0.06 | 1.03 | 0.06 | 1.00 | 0.06 | 0.99 | 0.15 | 1.01 |
| Sucralose | 0.03 | 0.50 | 0.03 | 0.50 | 0.03 | 0.50 | 0.08 | 0.50 |
| Saccharin Na | 0.03 | 0.49 | 0.03 | 0.50 | 0.03 | 0.50 | 0.08 | 0.50 |
| Oleic acid | - | - | 0.30 | 5.07 | 0.60 | 10.01 | 1.49 | 9.96 |
| Titanium dioxide | - | - | - | - | - | - | 0.15 | 1.00 |
| Ethanol | 19.53 | - | 19.52 | - | 19.55 | - | 48.73 | - |
| Total | 25.53 | 100.00 | 25.52 | 100.01 | 25.55 | 100.00 | 63.73 | 99.99 |
| Area weight [g/m²] | 93.8 | | 93.6 | | 94.3 | | 98.3 | |
| Agomelatine content [µg/cm²] | 468.7 | | 467.8 | | 471.6 | | 491.9 | |

### Preparation of the coating composition

For Examples 6a to 6c, a beaker was loaded with agomelatine. Vanilla flavor, ethanol, sucralose, saccharin Na, and oleic acid (Ex. 6b and 6c) were added and the mixture was then stirred. Hydroxypropyl cellulose was added under stirring to obtain a clear solution.

For Example 6d, a beaker was loaded with agomelatine. Vanilla flavor, ethanol, sucralose, saccharin Na, and oleic acid were added and the mixture was then stirred. Titanium dioxide and hydroxypropyl cellulose were added under stirring to obtain a white mixture.

### Coating of the coating composition

The resulting agomelatine-containing coating composition of Examples 6a to 6d was coated on a polyester film (one side siliconized, 75 µm thickness, which may function as release liner) and dried for approx. 10 min at room temperature, 15 min at 40 °C, and 10 min at 70 °C. The coating thickness gave an area weight of 93.8 /m² (Ex. 6a), 93.6 g/m² (Ex. 6b), 94.3 g/m² (Ex. 6c), and 98.3 g/m² (Ex. 6d), respectively. The dried film not further laminated with an additional backing layer and is therefore the final agomelatine-containing mucoadhesive layer structure.

### Preparation of the transmucosal therapeutic system

See Example 1.

### Measurement of mucosa permeation rate

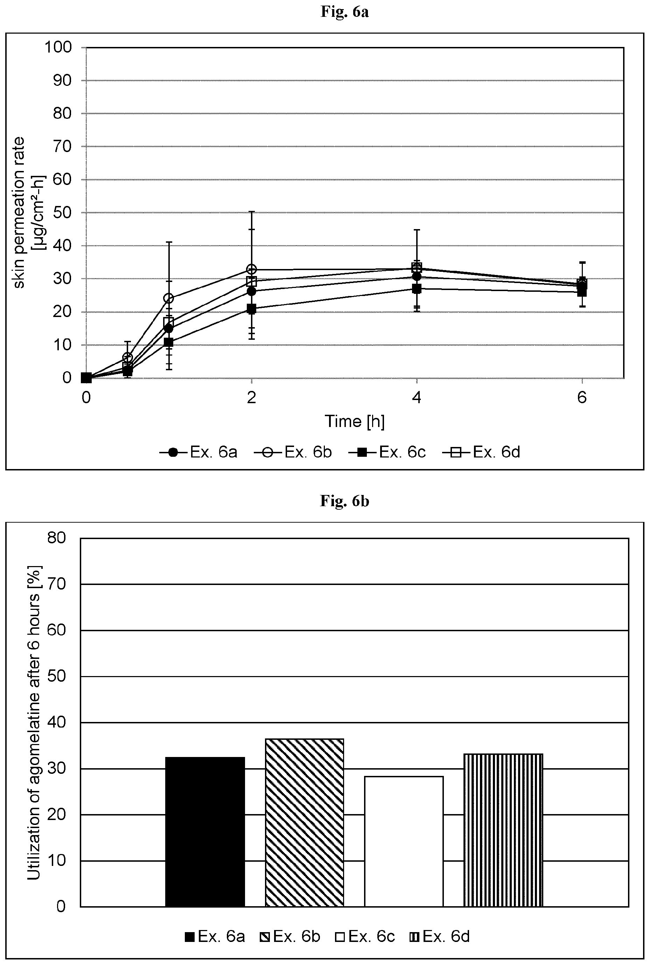

The permeated amount and the corresponding mucosa permeation rates of the transmucosal therapeutic systems prepared according to Examples 6a to 6d were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. Diecuts with an area of 0.524 cm² were punched from the transmucosal therapeutic systems, applied to the mucosa and the mucosa with the transmucosal therapeutic system was immersed on the top side in artificial saliva (the bottom side being in contact with receptor medium, and the top side being compartmentalized to a mucosa area of 1.145 cm²). The agomelatine permeated amount in the receptor medium (phosphate buffer solution pH 7.4) at a temperature of 37 ± 1°C was measured and the corresponding mucosa permeation rate calculated. The results are shown in Table 6.2 and Figure 6a.

**Table 6.2**

| **Mucosa permeation rate with SD [µg/(cm² h)]** | | | | | | | | |
|---|---|---|---|---|---|---|---|---|
| **Elapsed time [h]** | **Ex. 6a (n = 6)** | | **Ex. 6b (n = 6)** | | **Ex. 6c (n = 6)** | | **Ex. 6d (n = 6)** | |
| | **Rate** | **SD** | **Rate** | **SD** | **Rate** | **SD** | **Rate** | **SD** |
| **0.5** | 2.34 | 1.22 | 6.16 | 4.92 | 1.88 | 2.35 | 3.27 | 3.21 |
| **1** | 14.91 | 6.13 | 24.09 | 17.10 | 10.77 | 8.14 | 16.79 | 12.48 |
| **2** | 26.24 | 6.81 | 32.84 | 17.62 | 21.03 | 9.25 | 29.21 | 15.73 |
| **4** | 30.66 | 4.94 | 33.06 | 11.81 | 27.05 | 6.87 | 33.32 | 11.57 |
| **6** | 27.76 | 2.85 | 28.18 | 6.61 | 25.96 | 4.24 | 28.41 | 6.74 |

| | | | | | | | | |
|---|---|---|---|---|---|---|---|---|
| *: Standard deviation in this Example was, as in all other Examples, calculated based on the n-method. | | | | | | | | |

### Utilization of agomelatine

The utilization of agomelatine at 6 hours was calculated based on the cumulative permeated amount at 6 hours and the initial agomelatine content. The results are shown in Table 6.3 and in Figure 6b.

**Table 6.3**

| **Utilization of agomelatine after 6 hours [%]** | | | |
|---|---|---|---|
| **Example 6a (n = 6)** | **Example 6b (n = 6)** | **Example 6c (n = 6)** | **Example 6d (n = 6)** |
| **32.37** | **36.43** | **28.28** | **33.08** |

The *in vitro* experiments show a good mucosa permeation rate and a satisfying utilization. These Examples show that a satisfying permeation behavior can be obtained even at lower agomelatine concentration, and that the performance of hydroxypropyl cellulose is comparable to that of polyvinylpyrrolidone. It also shows that a certain amount of a fatty acid also seems to increase the permeation rate (see Example 6b in comparison to Examples 6a and 6c).

### EXAMPLES 7A-7G

### Coating composition

The formulations of the agomelatine-containing coating compositions of Examples 4b and 6b to 6d are summarized in Tables 4.1 and 6.1, above. The formulations of the agomelatine-containing coating compositions of Examples 7a to 7g are summarized in Tables 7.1 and 7.2 below. The formulations are based on weight percent, as also indicated in these Tables.

**Table 7.1**

| **Agomelatine-containing layer** | | | | | | | | |
|---|---|---|---|---|---|---|---|---|
| **Ingredient (Trade Name)** | **Ex. 7a** | | **Ex. 7b** | | **Ex. 7c** | | **Ex. 7d** | |
| | **Amt [g]** | **Solids [%]** | **Amt [g]** | **Solids [%]** | **Amt [g]** | **Solids [%]** | **Amt [g]** | **Solids [%]** |
| Agomelatine | 2.01 | 19.97 | 2.00 | 19.95 | 2.01 | 19.96 | 2.40 | 20.01 |
| Hydroxypropyl cellulose | - | - | 8.01 | 80.05 | - | - | - | - |
| Polyvinyl alcohol | - | - | - | - | - | - | 31.58 | 79.99 |
| Povidone K90 | 4.00 | 39.99 | - | - | 8.04 | 80.04 | - | - |
| Povidone K30 | 4.01 | 40.05 | - | - | - | - | - | - |
| Ethanol | 32.57 | - | 32.56 | - | 32.56 | - | - | - |
| Total | 42.59 | 100.01 | 42.57 | 100.00 | 42.61 | 100.0 | 33.98 | 100.00 |
| Area weight [g/m²] | 104.4 | | 103.2 | | 100.7 | | 82.9 | |
| Agomelatine content [µg/cm²] | 2084.3 | | 2058.6 | | 2009.7 | | 1658.8 | |
| Diecut size [cm²] | 0.28 | | | | | | | |
| | | | | | | | | |

| **Backing layer** | | | | | | | | |
|---|---|---|---|---|---|---|---|---|
| **Ingredient (Trade Name)** | **Ex. 7a to 7d** | | | | | | | |
| | **Amt [g]** | | | | **Solids [%]** | | | |
| Kollidon VA 64 | 11.69 | | | | 38.95 | | | |
| Eudragit L 100-5 5 | 11.69 | | | | 38.96 | | | |
| Sucralose | 0.15 | | | | 0.50 | | | |
| Saccharin Na | 0.15 | | | | 0.50 | | | |
| Oleic acid | 3.01 | | | | 10.04 | | | |
| FD&C Red No. 40 | 0.02 | | | | 0.05 | | | |
| Vanilla flavor | 0.30 | | | | 1.00 | | | |
| Polyethylene glycol 400 | 3.00 | | | | 10.01 | | | |
| Ethanol | 36.33 | | | | - | | | |
| Aqua purificata | 9.02 | | | | - | | | |
| Total | 75.36 | | | | 100.01 | | | |
| Area weight [g/m²] | 98.9 | | | | | | | |
| Diecut size [cm²] | 0.8 | | | | | | | |

**Table 7.2**

| **Agomelatine-containing layer** | | | | | | |
|---|---|---|---|---|---|---|
| **Ingredient (Trade Name)** | **Ex. 7e** | | **Ex. 7f** | | **Ex. 7g** | |
| | **Amt [g]** | **Solids [%]** | **Amt [g]** | **Solids [%]** | **Amt [g]** | **Solids [%]** |
| Agomelatine | 0.80 | 5.00 | 0.75 | 5.02 | 0.50 | 9.98 |
| Eucalyptol | - | - | - | - | 0.02 | 0.49 |
| Menthol | - | - | - | - | 0.05 | 1.00 |
| Methyl salicylate | - | - | - | - | 0.03 | 0.57 |
| Novamint Fresh Peppermint | - | - | - | - | 0.17 | 3.45 |
| Kolliphor RH 40 (Cremophor) | - | - | - | - | 0.10 | 2.07 |
| FD&C Red #40 | - | - | - | - | 0.01 | 0.11 |
| Polysorbate 80 (Tween 80) | - | - | - | - | 0.11 | 2.19 |
| Polyvinylpyrrolidone (Povidone K90) | - | - | - | - | 3.99 | 79.63 |
| Hydroxypropyl cellulose | - | - | 12.43 | 82.86 | - | - |
| Polyvinyl alcohol | 13.28 | 82.95 | - | - | - | - |
| Vanilla flavor | 0.16 | 1.00 | 0.16 | 1.06 | - | - |
| Sucralose | 0.08 | 0.50 | 0.08 | 0.52 | 0.03 | 0.51 |
| Saccharin Na | 0.08 | 0.51 | 0.08 | 0.50 | - | - |
| Oleic acid | 1.61 | 10.04 | 1.49 | 9.94 | - | - |
| FD&C Yellow No.5 | - | - | 0.02 | 0.10 | - | - |
| Ethanol | - | - | 48.83 | - | 15.01 | - |
| Aqua purificata | 38.07 | - | - | - | - | - |
| Total | 54.08 | 100.00 | 63.84 | 100.00 | 20.02 | 100.00 |
| Area weight [g/m²] | 98.4 | | 95.7 | | 115.4 | |
| Agomelatine content [µg/cm²] | 492.4 | | 480.1 | | 1150.8 | |
| Diecut size [cm²] | 0.28 | | | | | |
| | | | | | | |

| **Backing layer** | | | | | | |
|---|---|---|---|---|---|---|
| **Ingredient (Trade Name)** | **Ex. 7e and 7f** | | **Ex. 7g** | | | |
| | **Amt [g]** | **Solids [%]** | **Amt [g]** | **Solids [%]** | | |
| Kollidon VA 64 | 11.69 | 38.94 | 11.69 | 38.95 | | |
| Eudragit L100-55 | 11.69 | 38.94 | 11.69 | 38.96 | | |
| Sucralose | 0.15 | 0.50 | 0.15 | 0.50 | | |
| Saccharin Na | 0.15 | 0.50 | 0.15 | 0.50 | | |
| Oleic acid | 3.01 | 10.01 | 3.01 | 10.04 | | |
| FD&C Red No. 40 | 0.02 | 0.05 | 0.02 | 0.05 | | |
| Vanilla flavor | 0.30 | 1.00 | 0.30 | 1.00 | | |
| Polyethylene glycol 400 | - | - | 3.00 | 10.01 | | |
| Glycerol | 3.02 | 10.07 | - | - | | |
| Ethanol | 36.00 | - | 36.33 | - | | |
| Aqua purificata | 8.99 | - | 9.02 | - | | |
| Total | 75.02 | 100.01 | 75.36 | 100.01 | | |
| Area weight [g/m²] | 81.2 | | 98.9 | | | |
| Size | 0.8 | | | | | |

### Preparation of a first coating composition (agomelatine-containing layer)

The first coating composition for Example 7g was prepared in analogy to that of Example 4b.

For Examples 7a to 7c, a beaker was loaded with agomelatine. Ethanol was added and the mixture was then stirred. Povidone K90 and Povidone K30 (Ex. 7a), hydroxypropyl cellulose (Ex. 7b), and Povidone K90 (Ex. 7c), respectively, were added under stirring to obtain a viscose mixture.

For Example 7d, a beaker was loaded with agomelatine. Polyvinyl alcohol was added and the mixture was stirred to obtain a white mixture.

For Examples 7e, a beaker was loaded with polyvinyl alcohol. Aqua purificata was added and the mixture was then stirred and heated to 95 °C. Vanilla flavor, Sucralose, Saccharin Na, oleic acid, and agomelatine were added under stirring to obtain a viscose mixture.

For Examples 7f, a beaker was loaded with agomelatine. Ethanol, Vanilla flavor, Sucralose, Saccharin Na, oleic acid, and FD&C Yellow No.5 were added and the mixture was then stirred. Hydroxypropyl cellulose was added under stirring to obtain a viscose mixture.

### Coating of the first coating composition

The resulting agomelatine-containing coating composition of Examples 7a to 7f was coated on a polyester film (one side siliconized, 75 µm thickness, which may function as release liner) and dried for approx. 10 min at room temperature, 15 min at 40 °C, and 10 min at 70 °C (Ex. 7a to 7c and 7f) and 5 min at 70 °C (Ex. 7d) and 15 min at 70 °C (Ex. 7e), respectively. The coating thickness gave an area weight of 104.4 /m² (Ex. 7a), 103.2 g/m² (Ex. 7b), 100.7 g/m² (Ex. 7c), 82.9 g/m² (Ex. 7d), 98.4 g/m² (Ex. 7e), and 95.7 g/m² (Ex. 7f).

The resulting agomelatine-containing coating composition of Examples 7g was coated on a polyester film (one side siliconized, 75 µm thickness, which may function as release liner) and dried for approx. 5 min at room temperature, 15 min at 50 °C, and 2 min at 90 °C. The coating thickness gave an area weight of 115.4 g/m².

Diecuts with a size of size of 0.28 cm² were punched from the dried agomelatine-containing layers.

### Preparation of a second coating composition (backing layer)

For Examples 7a to 7d and 7g (same backing layer) as well as for Examples 7e and 7f (same backing layer), a beaker was loaded with Vanilla flavor. Sucralose, Saccharin, oleic acid, ethanol, aqua purificata, FD&C red, and polyethylene glycol (Examples 7a to 7d and 7g) and glycerol (Examples 7e and 7f), respectively, were added and the mixture was then stirred. Kollidon and Eudragit were added under stirring to obtain a clear solution.

The resulting second coating composition was coated on a polyester film (polyethylene terephthalate film, one side siliconized, 75 µm thickness, which may function as release liner) and dried for approx. 10 min at room temperature, 15 min at 40 °C, and 5 min at 70 °C. The coating thickness gave an area weight of 92.2 g/m² (7a to 7d and 7g) and 81.2 g/m² (Examples 7e and 7f), respectively.

Diecuts with a size of size of 0.8 cm² were punched from the dried backing layers.

### Preparation of the transmucosal therapeutic system

For Examples 7a to 7d and 7g, the diecuts of the agomelatine-containing layers were attached to the diecuts of the respective backing layer using small amounts of a 23.5 % ethanolic PVP90 solution in a manner so that the backing layer extends evenly to all sides of the agomelatine-containing layer and then laminated, to provide an agomelatine-containing mucoadhesive layer structure. For Examples 7e and 7f, the dried film was laminated with the respective backing layer, also so that the backing layer extends evenly to all sides of the agomelatine-containing layer, to provide an agomelatine-containing mucoadhesive layer structure.

The further steps (e.g. punching out individual devices) were conducted as in Example 1.

### Measurement of mucosa permeation rate

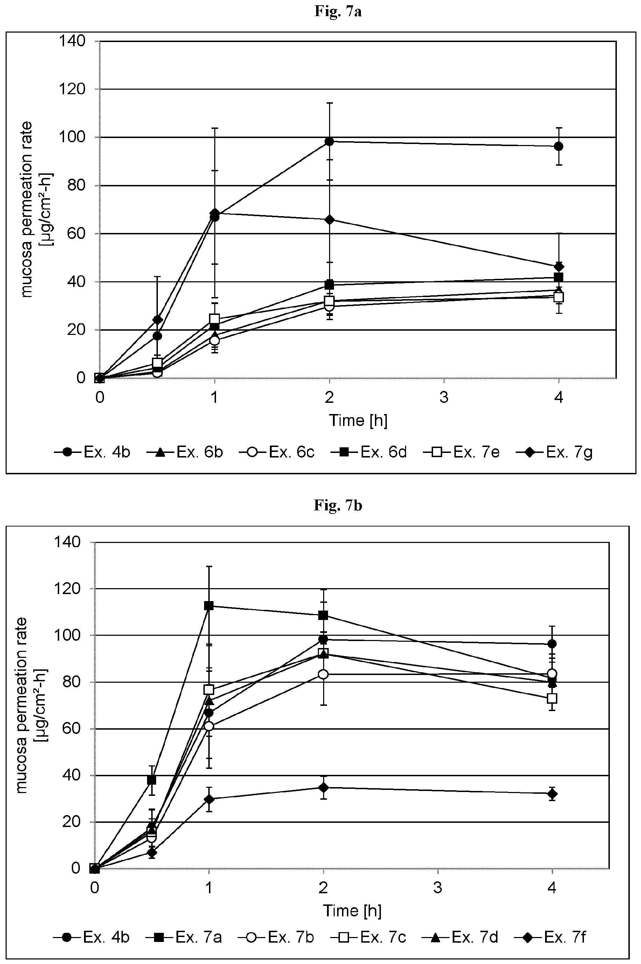

The permeated amount and the corresponding mucosa permeation rates of transmucosal therapeutic systems prepared according to Examples 4b, 6b to 6d, and 7a to 7g were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. The above-described agomelatine-containing mucoadhesive layer structures were applied to the mucosa and the mucosa with the transmucosal therapeutic system was immersed on the top side in artificial saliva (the bottom side being in contact with receptor medium, and the top side being compartmentalized to a mucosa area of 1.145 cm²). The agomelatine permeated amount in the receptor medium (phosphate buffer solution pH 7.4) at a temperature of 37 ± 1°C was measured and the corresponding mucosa permeation rate calculated. The results are shown in Tables 7.3 and 7.4 and Figures 7a and 7b.

**Table 7.3**

| **Mucosa permeation rate with SD [µg/(cm² h)]** | | | | | | | | | | | | |
|---|---|---|---|---|---|---|---|---|---|---|---|---|
| **Elapsed time [h]** | **Ex. 4b (n = 6)** | | **Ex. 6b (n = 6)** | | **Ex. 6c (n = 6)** | | **Ex. 6d (n = 6)** | | **Ex. 7a (n = 6)** | | **Ex. 7b (n = 6)** | |
| | **Rate** | **SD** | **Rate** | **SD** | **Rate** | **SD** | **Rate** | **SD** | **Rate** | **SD** | **Rate** | **SD** |
| **0.5** | 17.52 | 7.95 | 2.66 | 1.69 | 2.16 | 1.23 | 4.29 | 2.55 | 37.84 | 6.30 | 13.05 | 6.60 |
| **1** | 66.80 | 19.43 | 17.85 | 5.78 | 15.61 | 4.97 | 22.04 | 9.08 | 112.72 | 17.02 | 60.97 | 17.93 |
| **2** | 98.31 | 15.99 | 32.07 | 5.85 | 29.75 | 5.41 | 38.58 | 9.58 | 108.59 | 11.05 | 83.27 | 13.18 |
| **4** | 96.28 | 7.74 | 36.73 | 3.47 | 34.46 | 3.47 | 41.83 | 6.33 | 81.67 | 8.66 | 83.50 | 8.65 |

| | | | | | | | | | | | | |
|---|---|---|---|---|---|---|---|---|---|---|---|---|
| *: Standard deviation in this Example was, as in all other Examples, calculated based on the n-method. | | | | | | | | | | | | |

**Table 7.4**

| **Mucosa permeation rate with SD [µg/(cm² h)]** | | | | | | | | | | |
|---|---|---|---|---|---|---|---|---|---|---|
| **Elapsed time [h]** | **Ex. 7c (n = 6)** | | **Ex. 7d (n = 6)** | | **Ex. 7e (n = 6)** | | **Ex. 7f (n = 6)** | | **Ex. 7g (n = 6)** | |
| | **Rate** | **SD** | **Rate** | **SD** | **Rate** | **SD** | **Rate** | **SD** | **Rate** | **SD** |
| **0.5** | 15.58 | 9.67 | 16.63 | 4.76 | 6.24 | 3.44 | 6.94 | 2.36 | 24.26 | 17.96 |
| **1** | 76.59 | 19.77 | 72.08 | 12.71 | 24.50 | 6.81 | 29.75 | 5.17 | 68.59 | 35.26 |
| **2** | 92.23 | 9.38 | 92.10 | 9.37 | 32.00 | 5.22 | 34.75 | 4.81 | 65.89 | 24.94 |
| **4** | 72.90 | 5.05 | 79.94 | 5.17 | 33.57 | 6.55 | 32.13 | 2.76 | 46.31 | 14.00 |

| | | | | | | | | | | |
|---|---|---|---|---|---|---|---|---|---|---|
| *: Standard deviation in this Example was, as in all other Examples, calculated based on the n-method. | | | | | | | | | | |

### Utilization of agomelatine

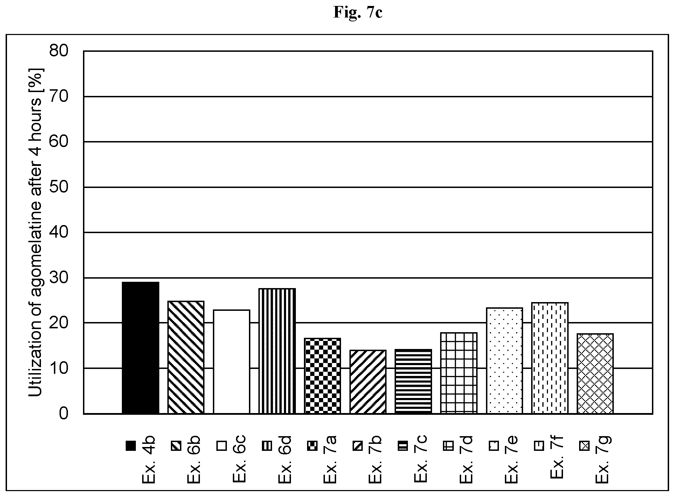

The utilization of agomelatine at 4 hours was calculated based on the cumulative permeated amount at 4 hours and the initial agomelatine content. The results are shown in Tables 7.5 and in Figure 7c.

**Table 7.5**

| **Utilization of agomelatine after 4 hours [%]** | | | | | | | | | | |
|---|---|---|---|---|---|---|---|---|---|---|
| **Ex. 4b (n = 6)** | **Ex. 6b (n = 6)** | **Ex. 6c (n = 6)** | **Ex. 6d (n = 6)** | **Ex. 7a (n = 6)** | **Ex. 7b (n = 6)** | **Ex. 7c (n = 6)** | **Ex. 7d (n = 6)** | **Ex. 7e (n = 6)** | **Ex. 7f (n = 6)** | **Ex. 7g (n = 6)** |
| **28.93** | **24.75** | **22.81** | **27.53** | **16.66** | **13.96** | **14.14** | **17.86** | **23.26** | **24.44** | **17.62** |

The *in vitro* experiments show a good mucosa permeation rate and a satisfying utilization. These Examples show that a satisfying permeation behavior can be obtained even at lower agomelatine concentration, and that the performance of hydroxypropyl cellulose and polyvinylalcohol is comparable to that of polyvinylpyrrolidone (see Examples 7b, 7c and 7d).

### EXAMPLES 8A-8C

### Coating composition

The formulations of the agomelatine-containing coating compositions of Examples 4b, 8a, and 8b are summarized in Tables 4.1 above and 8.1 below. The formulations are based on weight percent, as also indicated in Tables 4.1 and 8.1.

**Table 8.1**

| **Agomelatine-containing layer** | | | | | |
|---|---|---|---|---|---|
| **Ingredient (Trade Name)** | **Examples 8a, 8b and 8c** | | | | |
| | **Amt [g]** | **Solids [%]** | | | |
| Agomelatine | 1.50 | 10.00 | | | |
| Eucalyptol | 0.09 | 0.58 | | | |
| Menthol | 0.15 | 1.01 | | | |
| Methyl salicylate | 0.08 | 0.53 | | | |
| Novamint Fresh Peppermint | 0.52 | 3.44 | | | |
| Kolliphor RH 40 (Cremophor) | 0.29 | 1.94 | | | |
| FD&C Red #40 | 0.01 | 0.10 | | | |
| Sucralose | 0.08 | 0.50 | | | |
| Polyvinylpyrrolidone (Povidone K90) | 11.99 | 79.88 | | | |
| Polysorbate 80 (Tween 80) | 0.31 | 2.04 | | | |
| Ethanol | 45.02 | - | | | |
| Total | 60.04 | 100.02 | | | |
| Area weight [g/m²] | 109.7 | | | | |
| Agomelatine content [µg/cm²] | 1096.1 | | | | |
| Diecut size [cm²] | 1.6 | | | | |
| | | | | | |

| **Backing layer** | | | | | |
|---|---|---|---|---|---|
| **Ingredient (Trade Name)** | **Ex. 8a** | **Ex. 8b** | | **Ex. 8c** | |
| | | **Amt [g]** | **Solids [%]** | **Amt [g]** | **Solids [%]** |
| Kollidon VA 64 | | - | - | 10.63 | 42.53 |
| Eudragit L100-5 5 | | - | - | 10.60 | 42.41 |
| Ethyl cellulose N50F | | 5.55 | 55.36 | - | - |
| Glycerol | | 1.51 | 15.06 | 3.77 | 15.00 |
| FD&C blue No.1 | | - | - | 0.01 | 0.05 |
| FD&C green No.3 | | 0.005 | 0.05 | | |
| Castor oil | | 2.96 | 29.53 | - | - |
| Ethanol | | 56.72 | - | 28.11 | - |
| Aqua purificata | | - | - | 9.43 | - |
| Total | | 66.75 | 100.00 | 62.55 | 99.99 |
| Area weight [g/m²] | | 19.9 | | 78.3 | |
| Diecut size [cm²] | | 5.5 | | 5.5 | |
| | | | | | |

| **Adhesive layer** | | | | | |
|---|---|---|---|---|---|
| **Ingredient (Trade Name)** | **Ex. 8a** | **Examples 8b and 8c** | | | |
| | | **Amt [g]** | | **Solids [%]** | |
| Hydroxyethyl cellulose | | 14.80 | | 73.92 | |
| Menthol | | 0.80 | | 4.00 | |
| Castor oil | | 4.00 | | 19.98 | |
| Polysorbate 80 (Tween 80) | | 0.42 | | 2.10 | |
| Ethanol | | 85.00 | | - | |
| Aqua purificata | | 28.34 | | - | |
| Total | | 133.36 | | 100.00 | |
| | | **Ex. 8b** | | **Ex. 8c** | |
| Area weight [g/m²] | | 42.9 | | 95.7 | |

### Preparation of a first coating composition (agomelatine-containing layer)

For Examples 8a to 8c, a beaker was loaded with agomelatine. Ethanol, Eucalyptol, Menthol, methyl salicylate, Novamint Fresh Peppermint, Kolliphor RH 40, Sucralose, FD&C Red #40, and Polysorbate 80 were added and the mixture was then stirred. Povidon was added under stirring to obtain a viscose mixture.

### Coating of the first coating composition

The resulting agomelatine-containing coating composition of Examples 8a to 8c was coated on a polyester film (polyethylene terephthalate film, one side siliconized, 75 µm thickness, which may function as release liner) and dried for approx. 10 min at room temperature, 15 min at 50 °C, and 2 min at 90 °C. The coating thickness gave an area weight of 109.7 g/m².

For Example 8a, the dried film is the final agomelatine-containing mucoadhesive layer structure. For Examples 8b and 8c, diecuts with a size of size of 1.6 cm² were punched from the dried agomelatine-containing layers.

### Preparation of a second coating composition (backing layer)

For Example 8b, a beaker was loaded with Ethanol. FD&C green No.3 was added and the mixture was then stirred. Ethyl cellulose N50F, Castor oil, and Glycerol were added under stirring to obtain a mixture.

The second coating composition of Example 8c was prepared in analogy to Example 5d above.

The resulting second coating composition was coated on a polyester film (polyethylene terephthalate film, one side siliconized, 75 µm thickness, which may function as release liner) and dried for approx. 10 min at room temperature and 20 min at 70 °C (Ex. 8b) and approx. 5 min at room temperature, 10 min at 35 °C, and 2 min at 80 °C (Ex. 8c). The coating thickness gave an area weight of 19.2 g/m² (Ex. 8b) and 78.3 g/m² (Ex. 8c), respectively.

### Preparation of the adhesive composition

A beaker was loaded with Ethanol. Aqua purificata and Menthol were added and the mixture was then stirred. Hydroxyethyl cellulose, castor oil, and Polysorbate 80 were added under stirring to obtain a homogeneous mixture.

The resulting adhesive composition was coated on top of the dried backing layer and dried for approx. 5 min at room temperature, 10 min at 50 °C, and 2 min at 90 °C. The coating thickness gave an area weight of 42.9 g/m² (Ex. 8b) and 95.7 g/m² (Ex. 8c), respectively. Diecuts with a size of size of 5.5 cm² were punched from the dried adhesive backing layer.

### Preparation of the transmucosal therapeutic system

For Examples 8b and 8c, the dried film was laminated with the respective adhesive backing layer to provide an agomelatine-containing mucoadhesive layer structure.

The further steps (e.g. punching out individual devices) were conducted as in

### Example 1.

### In vivo studies using Goettingen minipigs

In order to evaluate the delivery of agomelatine, *in vivo* experiments using *Goettingen* minipigs (female, about 6 to 7 months, body weight was 13.8-14.3 kg at the start of the study) were conducted. Four minipigs were used. Transmucosal therapeutic systems of Examples 8a, 8b and 8c, prepared as described above, were administered to the buccal mucosa of one animal each for Examples 8a and 8b, and two animals for Example 8c (one system per animal). The total wear time of the transmucosal therapeutic systems was 4 hours (after 4 hours the application area was cleaned to remove potential leftovers of the transmucosal therapeutic system). No leftovers were observed for Ex. 8a and minor leftovers were observed for Ex. 8b and 8c with backings.

During the study, the minipigs were maintained under sedation.

Blood samples were collected at 8 time points following application of the transmucosal therapeutic systems. Samples were collected at hours 0 (prior to treatment), 0.5, 1, 1.5, 2, 4 (immediately prior to test item removal), 5 and 8. The analysis of plasma samples were conducted in compliance with the OECD Principles of Good Laboratory Practice (as revised in 1997). These Principles are in conformity with other international GLP regulations.

Agomelatine concentrations in minipig plasma were determined using a validated liquid-liquid extraction followed by LC-MS/MS. All samples, collected before treatment start, were measured to be below the limit of quantification (0.100 ng/mL).

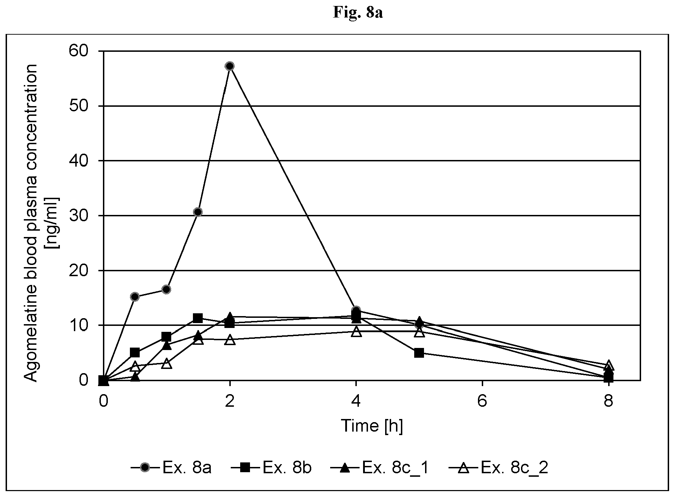

The agomelatine blood plasma concentration measured is summarized in table 8.2 and illustrated in Fig. 8a.

In addition, the mucosa condition was macroscopically determined and a Draize score obtained based on the score scheme below which is in accordance with the OECD Guideline for Testing of Chemicals No. 404, adopted 28^{th} July, 2015: "Acute Dermal Irritation/Corrosion" directly after removal of the OTF, 4 hours after application and cleaning of the OTF application side.

None of the formulations showed any irritation 4 hours after removal of the transmucosal therapeutic system (see Table 8.2).

**Table 8.2**

| **Agomelatine blood plasma concentration [ng/ml]** | | | | |
|---|---|---|---|---|
| **Elapsed time [h]** | **Ex. 8a (n = 1)** | **Ex. 8b (n = 1)** | **Ex. 8c_1 (n = 1)** | **Ex. 8c_2 (n = 1)** |
| | **Rate** | **Rate** | **Rate** | **Rate** |
| **0** | 0.00 | 0.00 | 0.00 | 0.00 |
| **0.5** | 15.20 | 5.06 | 0.71 | 2.65 |
| **1** | 16.50 | 7.93 | 6.49 | 3.15 |
| **1.5** | 30.60 | 11.30 | 8.28 | 7.55 |
| **2** | 57.20 | 10.40 | 11.60 | 7.48 |
| **4** | 12.70 | 11.80 | 11.30 | 8.99 |
| **5** | 10.10 | 5.01 | 10.80 | 8.93 |
| **8** | 0.49 | 0.48 | 2.01 | 2.82 |
| AUC₍₀₋₄₎ [/ng/ml) h] | 115.4 | 36.9 | 33.5 | 25.0 |
| Draize score* | 0 | 0 | 0 | 0 |

| | | | | |
|---|---|---|---|---|
| *: Score schemes for the evaluation of mucosa irritation potential at 4 hours according to Draize: 0 = No erythema, no edema, 1 = Very slight erythema (barely perceptible), very slight edema (barely perceptible), 2 = Well-defined erythema, Slight edema, 3 = Moderate to severe erythema, moderate edema, 4 = Severe erythema, severe edema. | | | | |

The maximum plasma concentrations were measured 2-4 hours after start of treatment and was in the range 9.0 to 11.8 ng/mL for the OTFs according to Ex. 8b and 8c with backing and 57.2 ng/mL for the OTF according to Ex. 8a without any backing. Very high PK-data for the transmucosal therapeutic system according to Ex. 8a without backing could be achieved, which can be explained by the open system, allowing the API to be delivered through the mucosa of the total oral cavity. The high delivery rate is still highly advantageous, as patch size and/or active concentration can be reduced in comparison to the systems comprising a backing layer. The transmucosal therapeutic systems with backing layer, while limiting the permeations area to 1.6 cm², show a lower, but still high permeability over 4 hours. As agomelatine was quantifiable 4 hours post removal of the transmucosal therapeutic systems with higher PK data, potential residual agomelatine could be within the mucosa.

### EXAMPLES 9A-9F

### Coating composition

The formulations of the agomelatine-containing coating compositions of Examples 4b and 9a to 9f are summarized in Tables 4.1 above and 9.1 below. The formulations are based on weight percent, as also indicated in Tables 4.1 and 9.1.

**Table 9.1**

| **Agomelatine-containing layer** | | | | | | | | |
|---|---|---|---|---|---|---|---|---|
| **Ingredient (Trade Name)** | **Ex. 9a** | | **Examples 9b, 9c and 9d** | | **Ex. 9e** | | **Ex. 9f** | |
| | **Amt [g]** | **Solids [%]** | **Amt [g]** | **Solids [%]** | **Amt [g]** | **Solids [%]** | **Amt [g]** | **Solids [%]** |
| Agomelatine | 5.01 | 5.01 | 6.51 | 5.00 | 0.75 | 4.99 | 0.75 | 4.98 |
| Hydroxypropyl cellulose GF (approx. 10% EtOH) | - | - | 210.90 | 16.21 | - | - | - | - |
| Hydroxypropyl cellulose EF (approx. 30% EtOH) | - | - | 280.90 | 64.79 | - | - | - | - |
| Hydroxypropyl cellulose EF | 82.04 | 81.99 | - | - | 7.40 | 49.11 | - | - |
| Ethyl cellulose N50NF | - | - | - | - | 4.76 | 31.57 | - | - |
| Hydroxypropyl cellulose LF | - | - | - | - | - | - | 12.15 | 80.75 |
| Vanilla flavor | 1.00 | 1.00 | 1.30 | 1.00 | 0.15 | 1.00 | 0.15 | 1.01 |
| Sucralose | 0.50 | 0.50 | 0.65 | 0.50 | 0.08 | 0.50 | 0.07 | 0.50 |
| Saccharin Na | 0.50 | 0.50 | 0.65 | 0.50 | 0.08 | 0.50 | 0.08 | 0.50 |
| Carbopol 974P solution (2% EtOH) | - | - | 64.98 | 1.00 | 7.55 | 1.00 | 7.49 | 1.00 |
| TiO₂ solution (9.09 % in oleic acid) | 11.00 | 11.00 | 14.30 | 11.00 | 1.71 | 11.34 | 1.69 | 11.26 |
| Ethanol | 325.52 | - | 122.90 | - | 41.47 | - | 41.51 | - |
| Total | 425.57 | 100.00 | 703.09 | 100.00 | 63.95 | 100.01 | 63.89 | 100.00 |
| Area weight [g/m²] | 117.0 | | 117.0 | | 123.5 | | 117.5 | |
| Agomelatine content [µg/cm²] | 586.17 | | 585.00 | | 616.3 | | 585.15 | |
| Diecut size [cm²] | 0.28 | | | | | | | |

| **Backing layer** | | | | | | | | |
|---|---|---|---|---|---|---|---|---|
| **Ingredient (Trade Name)** | **Ex. 9a** | **Ex. 9b** | **Ex. 9c** | | **Ex. 9d** | | **Examples 9e and 9f** | |
| | | | **Amt [g]** | **Solids [%]** | **Amt [g]** | **Solids [%]** | **Amt [g]** | **Solids [%]** |
| Hydroxypropyl cellulose EF | | | 64.53 | 29.33 | 23.46 | 29.31 | 26.39 | 29.32 |
| Hydroxyethyl cellulose | | | 128.96 | 58.61 | 46.91 | 58.61 | 52.77 | 58.63 |
| Vanilla flavor | | | 2.23 | 1.01 | 0.80 | 1.00 | 0.90 | 1.00 |
| Sucralose | | | 1.10 | 0.50 | 0.40 | 0.50 | 0.45 | 0.50 |
| Saccharin Na | | | 1.10 | 0.50 | 0.40 | 0.50 | 0.45 | 0.50 |
| Oleic acid | | | 21.99 | 10.00 | 8.03 | 10.03 | 9.00 | 10.00 |
| FD & C Red No. 40 | | | 0.11 | 0.05 | 0.04 | 0.05 | 0.05 | 0.05 |
| Ethanol | | | 358.06 | - | 130.22 | - | 155.10 | - |
| Aqua purificata | | | 364.54 | - | 130.30 | - | 155.22 | - |
| Total | | | 942.62 | 100.00 | 340.56 | 100.00 | 400.33 | 100.00 |
| Total area weight (agomelatine-containing layer and backing layer) [g/m²] | | | 382 | | 272.2 | | 388.1 | |
| Diecut size [cm²] | | | 0.28 | | | | | |

### Preparation of a first coating composition (agomelatine-containing layer)

For the TiO₂ solution, a beaker was loaded with 2.80 g TiO₂. Oleic acid was added and the mixture was then stirred to obtain the TiO₂ solution.

For Examples 9b, 9c and 9d, a beaker was loaded with Vanilla flavor. Sucralose, saccharin Na, Ethanol, hydroxypropyl cellulose GF, hydroxypropyl cellulose EF, Carbopol solution, and agomelatine were added and the mixture was then stirred. The TiO₂ solution was added under stirring to obtain a viscose mixture.

For Example 9a, a beaker was loaded with Vanilla flavor. Sucralose, saccharin Na, agomelatine, and Ethanol were added and the mixture was then stirred. The TiO₂ solution and hydroxypropyl cellulose were added under stirring to obtain a viscose mixture.

For Examples 9e and 9f, a beaker was loaded with Vanilla flavor. Sucralose, saccharin Na, and Ethanol, were added and the mixture was then stirred. The Carbopol solution, ethyl cellulose, hydroxypropyl cellulose, agomelatine, and the TiO₂ solution were added under stirring to obtain a viscose mixture.

### Coating of the first coating composition

The resulting agomelatine-containing coating composition was coated on a polyester film (polyethylene terephthalate film, one side siliconized, 75 µm thickness, which may function as release liner) and dried for approx. 10 min at room temperature, 15 min at 40 °C, and 10 min at 70 °C. The coating thickness gave an area weight of 117.0 g/m² (Ex. 9a to 9d), 123.5 g/m² (Ex. 9e), and 117.5 g/m² (Ex. 9f), respectively.

For Examples 9a and 9b, the dried film is the final agomelatine-containing mucoadhesive layer structure. For Example 9d, a diecut with a size of size of 0.28 cm² was punched from the dried agomelatine-containing layers.

### Preparation of a second coating composition (backing layer)

For Examples 9c to 9f, a beaker was loaded with Vanilla flavor. Sucralose, saccharin Na, oleic acid, FD & C Red No. 40, ethanol, and aqua purificata were and the mixture was then stirred. Hydroxypropyl cellulose and Hydroxyethyl cellulose were added under stirring to obtain a viscose mixture.

The resulting backing composition was coated twice on top of the dried agomelatine-containing layer (for Examples 9c, 9e and 9f) or on a polyester film (polyethylene terephthalate film, one side siliconized, 75 µm thickness, which may function as release liner) (for Example 9d) and dried for approx. 10 min at room temperature, 15 min at 50 °C, and 10 min at 80 °C. The coating thickness gave a total area weight (agomelatine-containing layer and backing layer) of 382 g/cm² (Ex. 9c), 272.2 g/m² (Ex. 9d) or 388.1 g/m² (Ex. 9e and 9f).

For Examples 9c, 9e and 9f, diecuts with a size of 0.28 cm² were punched from the dried layers to provide an agomelatine-containing mucoadhesive layer structure.

For Example 9d, a diecut with a size of 0.28 cm² was punched from the backing layer and attached to the respective agomelatine-containing layer via a little amount of the second coating compositing in a manner so that the backing layer extends evenly to all sides of the agomelatine-containing layer, to provide an agomelatine-containing mucoadhesive layer structure.

### Preparation of the transmucosal therapeutic system

See Example 1.

### Measurement of mucosa permeation rate

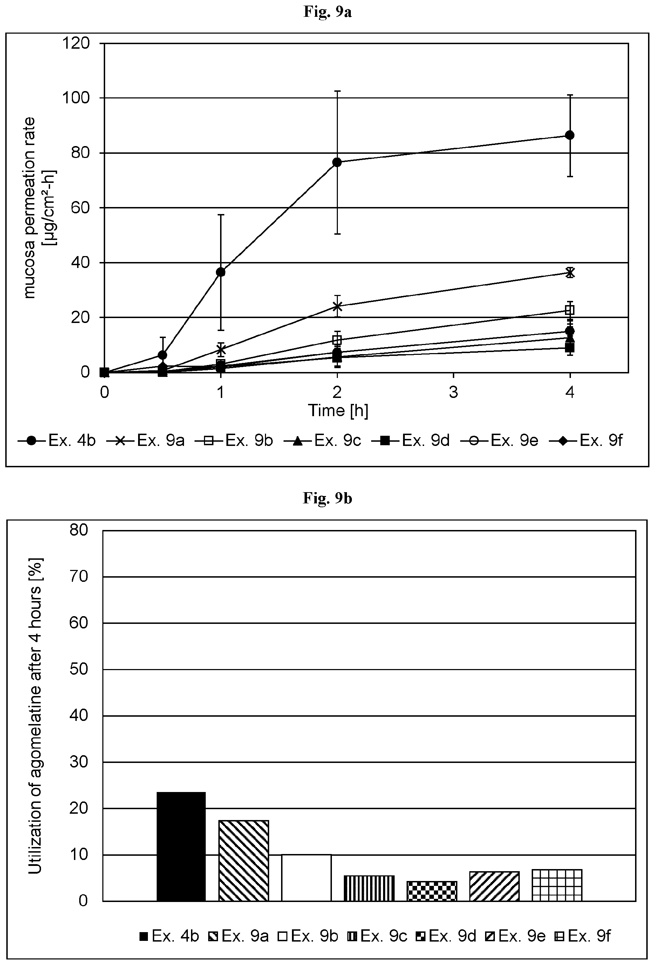

The permeated amount and the corresponding mucosa permeation rates of transmucosal therapeutic systems prepared according to Examples 4b, and 9a to 9f were determined by *in vitro* experiments in accordance with the OECD Guideline (adopted April 13, 2004) using pig mucosa (mucosa oesophagus). A dermatome was used to prepare mucosa to a thickness of 400 µm, with an intact barrier function for all transmucosal therapeutic systems. Diecuts with an area of 0.28 cm² punched from the transmucosal therapeutic systems of Examples 9a and 9b, as well as the above-described agomelatine-containing mucoadhesive layer structures were applied to the mucosa and the mucosa with the transmucosal therapeutic system was immersed on the top side in artificial saliva (the bottom side being in contact with receptor medium, and the top side being compartmentalized to a mucosa area of 1.145 cm²). The agomelatine permeated amount in the receptor medium (phosphate buffer solution pH 7.4) at a temperature of 37 ± 1°C was measured and the corresponding mucosa permeation rate calculated. The results are shown in Tables 9.2 and 9.3 and Figure 9a.

**Table 9.2**

| **Mucosa permeation rate with SD [µg/(cm² h)]** | | | | | | | | |
|---|---|---|---|---|---|---|---|---|
| **Elapsed time [h]** | **Ex. 4b (n = 6)** | | **Ex. 9a (n = 6)** | | **Ex. 9b (n = 6)** | | **Ex. 9c (n = 6)** | |
| | **Rate** | **SD** | **Rate** | **SD** | **Rate** | **SD** | **Rate** | **SD** |
| **0.5** | 6.33 | 6.55 | 0.72 | 0.32 | 0.19 | 0.16 | 0.13 | 0.16 |
| **1** | 36.51 | 21.05 | 8.35 | 2.48 | 3.07 | 1.65 | 1.46 | 1.56 |
| **2** | 76.60 | 25.99 | 24.17 | 3.95 | 11.75 | 3.32 | 5.67 | 3.79 |
| **4** | 86.39 | 14.89 | 36.48 | 1.81 | 22.65 | 3.27 | 12.72 | 5.07 |

| | | | | | | | | |
|---|---|---|---|---|---|---|---|---|
| *: Standard deviation in this Example was, as in all other Examples, calculated based on the n-method. | | | | | | | | |

**Table 9.3**

| **Mucosa permeation rate with SD [µg/(cm² h)]** | | | | | | |
|---|---|---|---|---|---|---|
| **Elapsed time [h]** | **Ex. 9d (n = 6)** | | **Ex. 9e (n = 6)** | | **Ex. 9f (n = 6)** | |
| | **Rate** | **SD** | **Rate** | **SD** | **Rate** | **SD** |
| **0.5** | 0.25 | 0.32 | 0.48 | 0.69 | 2.36 | 5.08 |
| **1** | 2.11 | 1.92 | 2.25 | 0.93 | 2.05 | 1.18 |
| **2** | 5.35 | 3.01 | 7.33 | 1.48 | 7.34 | 2.83 |
| **4** | 8.99 | 2.66 | 15.05 | 1.66 | 15.11 | 3.81 |

| | | | | | | |
|---|---|---|---|---|---|---|
| *: Standard deviation in this Example was, as in all other Examples, calculated based on the n-method. | | | | | | |

### Utilization of agomelatine

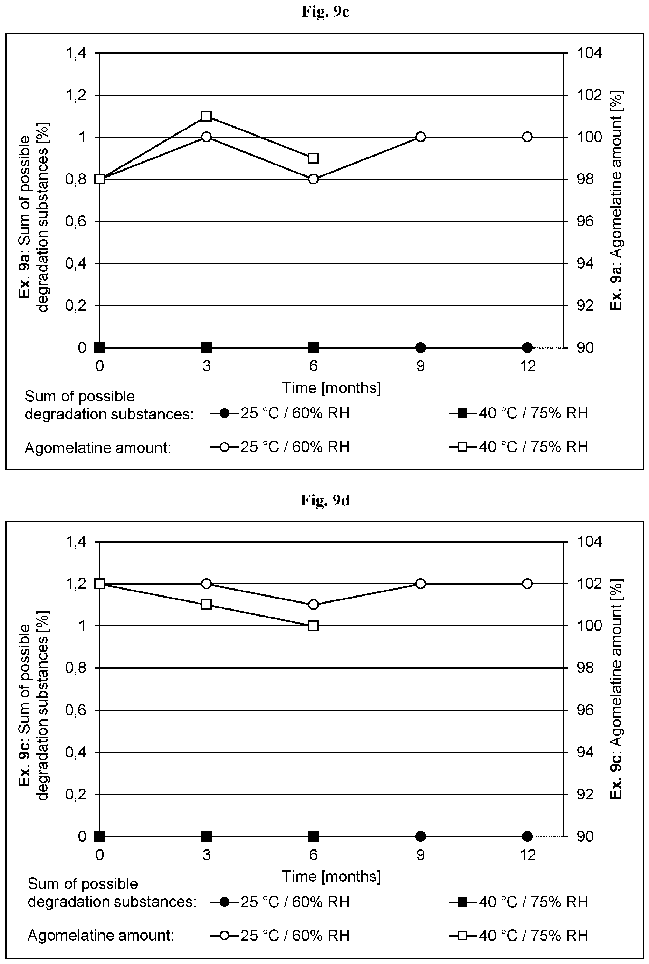

The utilization of agomelatine at 4 hours was calculated based on the cumulative permeated amount at 4 hours and the initial agomelatine content. The results are shown in Table 9.4 in Figure 9c.

**Table 9.4**

| **Utilization of agomelatine after 4 hours [%]** | | | | | | |
|---|---|---|---|---|---|---|
| **Ex. 4b (n = 6)** | **Ex. 9a (n = 6)** | **Ex. 9b (n = 6)** | **Ex. 9c (n = 6)** | **Ex. 9d (n = 6)** | **Ex. 9e (n = 6)** | **Ex. 9f (n = 6)** |
| 23.52 | 17.35 | 10.03 | 5.45 | 4.19 | 6.30 | 6.79 |

The *in vitro* experiments show a good mucosa permeation rate and a good utilization. These Examples again demonstrate that the open systems without backing layer provide increased active delivery when compared to those with backing layers.

### Storage stability measurements

A long term storage stability test was conducted for Examples 9a and 9c under different test conditions, i.e. storage at 25 °C and 60 % relative humidity (RH) and at 40 °C and 75 % RH. Samples were taken from the transmucosal therapeutic systems after 3, 6, 9 and 12 months storage at 25 °C and 60 % RH, and after 3 and 6 months storage at 40 °C and 75 % RH, the ethanol and water content was determined, and the amount of agomelatine, as well as various possible degradation substances was determined by a specific quantitative HPLC method based on the agomelatine content calculated from the (actual) area weight of the tested transmucosal therapeutic systems. The results are shown in Tables 9.5 to 9.8 as well as in Figures 9c and 9d.

**Table 9.5**

| **Ex. 9a** - **25 °C / 60 % RH** | Initial | 3 months | 6 months | 9 months | 12 months |
|---|---|---|---|---|---|
| Detected amounts: Agomelatine [%] | 98 | 100 | 98 | 100 | 100 |
| Detected amounts: Sum of possible degradation substances [%] | n.d. | n.d. | < LOQ | < LOQ | < LOQ |
| Ethanol (residual solvent) [wt-%] | 0 | 0 | 0 | 0 | 0 |
| Water [wt-%] | 3.5 | 3.2 | 3.1 | 3.0 | 3.4 |

| | | | | | |
|---|---|---|---|---|---|
| * n.d. = not detected, LOQ = Limit of Quantitation (0.05 %) | | | | | |

**Table 9.6**

| **Ex. 9c** - **25 °C / 60 % RH** | Initial | 3 months | 6 months | 9 months | 12 months |
|---|---|---|---|---|---|
| Detected amounts: Agomelatine [%] | 102 | 102 | 101 | 102 | 102 |
| Detected amounts: Sum of possible degradation substances [%] | n.d. | n.d. | < LOQ | < LOQ | < LOQ |
| Ethanol (residual solvent) [wt-%] | 5 | 3 | 3 | 3 | 3 |
| Water [wt-%] | 3.5 | 3.1 | 3.1 | 3.2 | 3.2 |

| | | | | | |
|---|---|---|---|---|---|
| * n.d. = not detected, LOQ = Limit of Quantitation (0.05 %) | | | | | |

**Table 9.7**

| **Ex. 9a - 40 °C / 75 % RH** | Initial | 3 months | 6 months | 9 months | 12 months |
|---|---|---|---|---|---|
| Detected amounts: Agomelatine [%] | 98 | 101 | 99 | N/A | N/A |
| Detected amounts: Sum of possible degradation substances [%] | n.d. | n.d. | < LOQ | N/A | N/A |
| Ethanol (residual solvent) [wt-%] | 0 | 0 | 0 | N/A | N/A |
| Water [wt-%] | 3.5 | 3.2 | 3.3 | N/A | N/A |

| | | | | | |
|---|---|---|---|---|---|
| * n.d. = not detected, LOQ = Limit of Quantitation (0.05 %), N/A = No data | | | | | |

**Table 9.8**

| **Ex. 9c - 40 °C / 75 % RH** | Initial | 3 months | 6 months | 9 months | 12 months |
|---|---|---|---|---|---|
| Detected amounts: Agomelatine [%] | 102 | 101 | 100 | N/A | N/A |
| Detected amounts: Sum of possible degradation substances [%] | n.d. | n.d. | < LOQ | N/A | N/A |
| Ethanol (residual solvent) [wt-%] | 5 | 3 | 3 | N/A | N/A |
| Water [wt-%] | 3.5 | 3.1 | 3.3 | N/A | N/A |

| | | | | | |
|---|---|---|---|---|---|
| * n.d. = not detected, LOQ = Limit of Quantitation (0.05 %), N/A = No data | | | | | |

The stability data show that the initial as well as storage stability is excellent for Examples 9a and 89c, both in terms of the amount of agomelatine (in particular with respect to the amount of agomelatine remaining after storage) as well as the sum of possible degradation substances.

### DISSOLUTION EXPERIMENT 10

As outlined above in more detail, the dissolvable film-forming agent is preferably soluble, dispersible or otherwise disintegrable in aqueous media, such as water, and, on the other hand, film-forming agents that are soluble in e.g. ethanol, are also preferred.

In order to observe the dissolving behavior of potential film-forming agents, 10 wt-% (+/- 0.1 %-point) mixtures of polymer in either water, in ethanol, or in a 1:1 water/ethanol mixture were prepared, and the dissolution behavior noted. The viscosity as well as other observations were noted. The result is summarized in table 10.1 below.

**Table 10.1**

| **Polymer** | **Solvent** | **Dissolved** (yes/no) | **viscosity** | **Other comments** |
|---|---|---|---|---|
| Croscarmellose-Na PhEur/NF | Water | No | | Polymer swollen |
| | Ethanol | No | | Turbid + precipitates |
| | Water / ethanol 1:1 | No | | Turbid + precipitates |
| Ethyl cellulose N50 NF | Water | No | | Precipitates |
| | Ethanol | Yes | Low-medium | Turbid |
| | Water / ethanol 1:1 | No | | Precipitates |
| Eudragit L100 | Water | No | | Precipitates |
| | Ethanol | Yes | Low | |
| | Water / ethanol 1:1 | Yes | Low | |
| Hydroxyethylcellulose | Water | Yes | Medium | |
| | Ethanol | No | | Precipitates |
| | Water / ethanol 1:1 | Yes | Medium | |
| Hydroxypropylcellulose EF | Water | Yes | Low | Slightly turbid |
| | Ethanol | Yes | Low | Slightly turbid |
| | Water / ethanol 1:1 | Yes | medium | |
| Hydroxypropylcellulose GF | Water | Yes | High | |
| | Ethanol | No | High | Turbid + undissolved matter |
| | Water / ethanol 1:1 | Yes | High | |
| Hydroxypropylcellulose HF | Water | No | | Gel-like, solid, turbid |
| | Ethanol | No | | Gel-like, solid, turbid |
| | Water / ethanol 1:1 | No | | Solid, rubber-like |
| Hydroxypropylcellulose LF | Water | Yes | Medium | Slightly turbid |
| | Ethanol | Yes | Low-medium | Slightly turbid |
| | Water / ethanol 1:1 | Yes | Medium | |
| Hydroxypropylmethyl-cellulose 2910/603- | Water | Yes | Low | |
| | Ethanol | No | | undissolved particles |
| | Water / ethanol 1:1 | Yes | low | |
| Hydroxypropylmethyl-cellulose 2910/60SH50 | Water | Yes | Medium | |
| | Ethanol | No | | Precipitates |
| | Water / ethanol 1:1 | Yes | High | Slightly turbid |
| Povidone K30 | Water | Yes | Low | |
| | Ethanol | Yes | Low | |
| | Water / ethanol 1:1 | Yes | Low | |
| Povidone K90 F | Water | Yes | Low-medium | |
| | Ethanol | Yes | Low-medium | |
| | Water / ethanol 1:1 | Yes | Low-medium | |
| Kollidon VA64 | Water | Yes | Low | |
| | Ethanol | Yes | Low | |
| | Water / ethanol 1:1 | Yes | Low | |
| Methyl cellulose 15 cP | Water | Yes | High | Slightly turbid |
| | Ethanol | No | | Precipitates |
| | Water / ethanol 1:1 | Yes | High | Slightly turbid |
| Methyl cellulose 400 cP | Water | Yes | | Solid, turbid matter |
| | Ethanol | No | | Precipitates |
| | Water / ethanol 1:1 | No | | Solid gel-like particles |
| Methyl cellulose 4000 cP | Water | No | | Solid rubber-like particles |
| | Ethanol | No | | Precipitates |
| | Water / ethanol 1:1 | No | | Solid gel-like particles |
| Sodium-CMC | Water | Yes | High | |
| | Ethanol | No | | Precipitates |
| | Water / ethanol 1:1 | No | | Precipitates |
| Soluplus | Water | Yes | Low | Opaque |
| | Ethanol | Yes | Low | Turbid |
| | Water / ethanol 1:1 | Yes | Low | |

### WATER ABSORPTION EXPERIMENT 11

Certain of the dissolvable film-forming agents exhibit hygroscopic behavior. In order to assess the water absorption over time, different coating compositions were prepared and a film was obtained by coating the coating compositions and drying the coated layers. The films were stored for 7 days at room temperature, either openly or packaged in a seam-sealed pouch. The detailed composition, drying conditions for the coated films and results obtained are given in Table 11.1 below.

**Table 11.1**

| **Coating composition** | | | | | | |
|---|---|---|---|---|---|---|
| | **Ex. 11a** | **Ex. 11b** | **Ex. 11c** | **Ex. 11d** | **Ex. 11e** | **Ex. 11f** |
| **Ingredient** | Amount [wt-%] | | | | | |
| Agomelatine | 10.0 | 10.0 | 10.0 | 10.0 | 10.0 | 10.0 |
| Povidone K90 | 90.0 | 85.1 | 80.0 | 85.0 | 79.9 | - |
| Hydroxypropyl cellulose | - | - | - | - | - | 90.0 |
| Glycerol | - | 4.9 | 10.0 | - | - | - |
| Aqua purificata | - | - | - | 5.0 | 10.1 | - |

| **Solvent** | Ethanol, 75 - 77 wt-% of coating composition | | | | | |
|---|---|---|---|---|---|---|
| **Coating and film properties** | | | | | | |
| **Drying conditions** | A | A | B | C | C | C |
| **Area weight [g/m²]** | 114.1 | 113.2 | 115.5 | 100.7 | 94.7 | 96.3 |
| **Film property, 7d RT** | Rigid, pliable, curved | pliable, curved | Elastic, curved | Pliable, risk of breakage | pliable | elastic |
| **Film property, 7d pouch** | Rigid, pliable | pliable | elastic | pliable | elastic | elastic |

| **Water content** | | | | | | |
|---|---|---|---|---|---|---|
| **7d RT [wt-%]** | 14.2 | 12.3 | 11.1 | 14.1 | 13.9 | 3.4 |
| **7d pouch [wt-%]** | 7.3 | 8.3 | 8.0 | 7.3 | 7.7 | 3.0 |

| | | | | | | |
|---|---|---|---|---|---|---|
| Drying condition A: 10 minutes at 50 °C and 15 minutes at 70 °C Drying condition B: 10 minutes at 40 °C and 15 minutes at 60 °C Drying condition B: 15 minutes at 40 °C and 10 minutes at 70 °C | | | | | | |

**The invention relates in particular to the following further items:**
1. Transmucosal therapeutic system for the transmucosal administration of agomelatine comprising a mucoadhesive layer structure, said mucoadhesive layer structure comprising
   A) an agomelatine-containing layer comprising
      i) agomelatine; and
      ii) a dissolvable film-forming agent.
2. Transmucosal therapeutic system according to item 1,
   wherein
   the dissolvable film-forming agent, if casted into a film having an area weight of from 30 to 100 g/m², or of 50 g/m², dissolves in water, in artificial or natural saliva, or in any other aqueous medium, at 37 °C and 150 rpm, in less than 5 hours, less than 3 hours, less than 2 hours, or less than 1 hour, or in more than 5 seconds, more than 30 seconds, more than 1 minute, or more than 2 minutes, or more than 5 seconds and less than 5 hours, in more than 30 seconds and less than 3 hours, more than 1 minute and less than 2 hours, or in more than 2 minutes and less than 1 hour.
3. Transmucosal therapeutic system according to item 1 or 2,
   wherein
   the dissolvable film-forming agent is selected from the group consisting of polymers such as polyvinylpyrrolidone, methyl cellulose, ethyl cellulose, hydroxyethyl cellulose, hydroxypropyl cellulose, hydroxypropylmethyl cellulose, carboxymethyl cellulose sodium, polyethylene glycol-polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, methacrylic acid - methyl methacrylate copolymers, and methacrylic acid - ethyl methacrylate copolymers, and natural film-forming agents such as shellac, pectin, gelatine, alginate, pullulan and starch derivatives, and any mixtures thereof.
4. Transmucosal therapeutic system according to item 3,
   wherein
   the dissolvable film-forming agent is selected from the group consisting of polymers such as polyvinylpyrrolidone, methyl cellulose, hydroxyethyl cellulose, hydroxypropyl cellulose, hydroxypropylmethyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof.
5. Transmucosal therapeutic system according to item 4,
   wherein
   the dissolvable film-forming agent is selected from the group consisting of polymers such as polyvinylpyrrolidone, hydroxypropyl cellulose, polyethylene glycol- polyvinyl acetate- and polyvinylcaprolactame-based graft copolymers, polyvinylpyrrolidone-polyvinylacetate copolymers, and any mixtures thereof.
6. Transmucosal therapeutic system according to item 5,
   wherein the dissolvable film-forming agent is a hydroxypropyl cellulose.
7. Transmucosal therapeutic system according to item 6,
   wherein the dissolvable film-forming agent is a hydroxypropyl cellulose having a molecular weight of from 50,000 to 1,500,000.
8. Transmucosal therapeutic system according to item 7,
   wherein the dissolvable film-forming agent is a hydroxypropyl cellulose having a molecular weight of 80,000, 95,000, 370,000 or 1,150,000.
9. Transmucosal therapeutic system according to item 5,
   wherein the dissolvable film-forming agent is a polyvinylpyrrolidone.
10. Transmucosal therapeutic system according to item 9,
   wherein the polyvinylpyrrolidone is selected from soluble polyvinylpyrrolidones.
11. Transmucosal therapeutic system according to item 9,
   wherein the polyvinylpyrrolidone is selected from polyvinylpyrrolidones having a K-Value within a range selected from the group of ranges consisting of
   9 to 15, and preferably 10.2 to 13.8,
   15 to 20, and preferably 15.3 to 18.4,
   20 to 27, and preferably 22.5 to 27.0,
   27 to 35, and preferably 27.0 to 32.4, and
   75 to 110, and preferably 81.0 to 97.2.
12. Transmucosal therapeutic system according to any one of items 1 to 11,
   wherein the amount of the dissolvable film-forming agent is at least 65 wt-%, at least 75 wt-% or at least 85 wt-%, or the amount of the dissolvable film-forming agent is less than or equal to 98 wt-%, less than or equal to 94 wt-% or less than or equal to 90 wt-%, or the amount of the dissolvable film-forming agent ranges from 65 to 98 wt-%, from 75 to 94 wt-%, or from 80 to 90 wt-% of the agomelatine-containing layer.
13. Transmucosal therapeutic system according to any one of items 1 to 12,
   wherein the agomelatine-containing layer comprises at least 1 wt-% agomelatine, at least 2 wt-% agomelatine, or at least 3 wt-% agomelatine.
14. Transmucosal therapeutic system according to any one of items 1 to 13,
   wherein the agomelatine-containing layer comprises less than or equal to 25 wt-% agomelatine, less than or equal to 20 wt-% agomelatine, or less than or equal to 10 wt-% agomelatine.
15. Transmucosal therapeutic system according to any one of items 1 to 14,
   wherein the agomelatine-containing layer comprises from 1 to less than or equal to 25 wt-% agomelatine, from 2 to less than or equal to 20 wt-% agomelatine, or from 3 to less than or equal to 10 wt-% agomelatine.
16. Transmucosal therapeutic system according to any one of items 1 to 15,
   wherein the agomelatine-containing layer further comprises one or more excipients selected from the group consisting of fatty acids, sweeteners, flavoring agents, colorants, permeation enhancers, solubilizers, plasticizers, humectants, disintegrants, emulsifiers, antioxidants, stabilizers, buffer reagents and further film-forming agents.
17. Transmucosal therapeutic system according to item 16,
   wherein the agomelatine-containing layer further comprises one or more excipients selected from the group consisting of fatty acids, sweeteners, and flavoring agents.
18. Transmucosal therapeutic system according to item 17,
   wherein the fatty acid is a saturated or unsaturated, linear or branched carboxylic acid comprising 4 to 24 carbon atoms, and in particular is selected from the group consisting of caprylic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid.
19. Transmucosal therapeutic system according to item 18,
   wherein the fatty acid is oleic acid or linoleic acid.
20. Transmucosal therapeutic system according to any one of items 16 to 19,
   wherein the agomelatine-containing layer comprises one or more fatty acids in an amount of at least 1 wt-%, at least 3 wt-% or at least 4 wt-%, or in an amount of less than or equal to 15 wt-%, less than or equal to 12 wt-%, or less than or equal to 10 wt-%, or in an amount of from 1 to 15 wt-%, from 3 to 12 wt-%, or from 4 to 10 wt-%.
21. Transmucosal therapeutic system according to item 16,
   wherein the agomelatine-containing layer comprises one or more natural or artificial sweeteners selected from the group consisting of saccharose, glucose, fructose, sorbitol, mannitol, isomalt maltitol lactitol, xylitol, erythritol, sucralose, acesulfame potassium, aspartame, cyclamate, neohesperidine, neotame, steviol glycosides, thaumatin and saccharin sodium.
22. Transmucosal therapeutic system according to item 21,
   wherein the agomelatine-containing layer comprises one or more natural or artificial sweeteners selected from the group consisting of saccharose, sucralose and saccharin sodium.
23. Transmucosal therapeutic system according to item 22,
   wherein the agomelatine-containing layer comprises one or more natural or artificial sweeteners selected from the group consisting of sucralose, saccharose and saccharin sodium in an amount of at least 0.05 wt-%, at least 0.1 wt-% or at least 0.3 wt-%, or in an amount of less than or equal to 2.0 wt-%, less than or equal to 1.5 wt-% or less than or equal to 1.0 wt-%, or in an amount of from 0.05 to 2.0 wt-%, from 0.1 to 1.5 wt-%, or from 0.3 to 1.0 wt-% each.
24. Transmucosal therapeutic system according to item 16,
   wherein the agomelatine-containing layer comprises one or more natural or artificial flavoring agents selected from the group consisting of vanillin, methyl salicylate, menthol, manzanate, diacetyl, acetylpropionyl, acetoin, isoamyl acetate, benzaldehyde, cinnamaldehyde, ethyl propionate, methyl anthranilate, limonene, ethyl decadienoate, allyl hexanoate, ethyl maltol, 2,4-dithiapentane, ethylvanillin and eucalyptol as well as flavoring compositions such as peppermint flavor.
25. Transmucosal therapeutic system according to item 24,
   wherein the agomelatine-containing layer comprises one or more flavoring agents selected from the group consisting of vanillin, methyl salicylate, menthol, peppermint flavor and eucalyptol in an amount of at least 0.1 wt-%, at least 0.3 wt-% or at least 0.4 wt-%, or in an amount of less than or equal to 10 wt-%, less than or equal to 6 wt-%, or less than or equal to 4 wt-%, or in an amount of from 0.1 to 10 wt-%, from 0.3 to 6 wt-%, or from 0.4 to 4 wt-% each, or
   in an amount of at least 0.1 wt-%, at least 0.5 wt-% or at least 0.7 wt-%, or in an amount of less than or equal to 15 wt-%, less than or equal to 10 wt-%, or less than or equal to 8 wt-%, or in an amount of from 0.1 to 15 wt-%, from 0.5 to 10 wt-%, or from 0.7 to 8 wt-% in total.
26. Transmucosal therapeutic system according to item 16,
   wherein the agomelatine-containing layer comprises one or more colorants selected from the group consisting of titanium dioxide, brilliant blue FCF, indigo carmine, fast green FCF, erythrosine, allura red AC, tartrazine and sunset yellow FCF, curcumin, riboflavin, rivoflavin-5'-phosphate, quinoline yellow, orange yellow S, cochineal, carminic acid, azorubine, carmoisine, amaranth, ponceau 4R, cochineal red A, patent blue V, indigotine, chlorophylls, chlorophyllins, copper complexes of chlorophyll and chlorophyllins, green S, plain caramel, caustic sulphite caramel, ammonia caramel, sulphite ammonia caramel, brilliant black BN, black PN, vegetable carbon, brown HT, carotenes, annatto, bixin, norbixin, paprika extract, capsanthian, capsorubin, lycopene, beta-apo-8'-carotenal, lutein, canthaxanthin, beetroot red, betanin, anthocyanins, calcium carbonate, iron oxides and hydroxides, aluminium, silver, gold and litholrubine BK.
27. Transmucosal therapeutic system according to item 16,
   wherein the agomelatine-containing layer comprises one or more further film-forming agents, wherein the further film-forming agents are different from the dissolvable film-forming agent.
28. Transmucosal therapeutic system according to item 27,
   wherein the agomelatine-containing layer comprises one or more further film-forming agents in an amount of at least 2 wt-%, at least 5 wt-% or at least 10 wt-%, or in an amount of less than or equal to 40 wt-%, less than or equal to 30 wt-%, or less than or equal to 25 wt-%, or in an amount of from 2 to 40 wt-%, from 5 to 30 wt-%, or from 10 to 25 wt-% each, or in an amount of at least 5 wt-%, at least 15 wt-% or at least 20 wt-%, or in an amount of less than or equal to 40 wt-%, less than or equal to 30 wt-%, or less than or equal to 25 wt-%, or in an amount of from 5 to 40 wt-%, from 15 to 30 wt-%, or from 20 to 25 wt-% in total.
29. Transmucosal therapeutic system according to item 16,
   wherein the agomelatine-containing layer comprises one or more solubilizers selected from the group consisting of ethoxylated sorbitan esterified with fatty acids such as polyoxyethylene sorbitan monolaurate, polyoxyethylene sorbitan monopalmitate, polyoxyethylene sorbitan monostearate and polyoxyethylene sorbitan monooleate, safflower oleosomes, propanediol and polyethoxylated castor oil.
30. Transmucosal therapeutic system according to item 29,
   wherein the agomelatine-containing layer comprises one or more solubilizers in an amount of at least 0.3 wt-%, at least 0.5 wt-% or at least 1.0 wt-%, or in an amount of less than or equal to 5 wt-%, less than or equal to 4 wt-%, or less than or equal to 3 wt-%, or in an amount of from 0.3 to 5 wt-%, from 0.5 to 4 wt-%, or from 1.0 to 3 wt-% each.
31. Transmucosal therapeutic system according to item 16,
   wherein the agomelatine-containing layer comprises one or more emulsifiers selected from the group consisting of soy lecithin, sodium phosphates, mono- and diglycerides of fatty acids, sodium stearoyl lactylate, diacetyl tartaric acid esters of mono- and diglycerides, and polyethoxylated hydrogenated castor oil .
32. Transmucosal therapeutic system according to item 31,
   wherein the agomelatine-containing layer comprises one or more emulsifiers in an amount of at least 1 wt-%, at least 3 wt-% or at least 5 wt-%, or in an amount of less than or equal to 25 wt-%, less than or equal to 20 wt-%, or less than or equal to 15 wt-%, or in an amount of from 1 to 25 wt-%, from 3 to 20 wt-%, or from 5 to 15 wt-% each.
33. Transmucosal therapeutic system according to item 16,
   wherein the agomelatine-containing layer comprises one or more plasticizers selected from the group consisting of mono-, di-, oligo- and polysaccharides and derivatives such as sorbitol, polyethylene glycol, triacetin, triethyl citrate, propylene glycol, glycerol and medium chain triglycerides.
34. Transmucosal therapeutic system according to item 33,
   wherein the agomelatine-containing layer comprises one or more plasticizers in an amount of at least 0.5 wt-%, at least 1 wt-% or at least 5 wt-%, or in an amount of less than or equal to 25 wt%, less than or equal to 20 wt-%, or less than or equal to 15 wt-%, or in an amount of from 0.5 to 25 wt-%, from 1 to 20 wt-%, or from 5 to 15 wt-% each.
35. Transmucosal therapeutic system according to any one of items 1 to 34,
   wherein the agomelatine-containing layer does not comprise a permeation enhancer selected from the group consisting of bile acid, bile acid salts, bile acid derivatives, acyl carnitines, sodium dodecylsulfate, dimethylsulfoxide, sodium laurylsulfate, terpenes, cyclodextrins, cyclodextrin derivatives, saponins, saponin derivatives, chitosan, EDTA, citric acid, and salicylates in an amount of more than 0.1 wt-%, more than 0.2 wt-%, more than 1 wt-% or more than 5 wt-%.
36. Transmucosal therapeutic system according to any one of items 1 to 35,
   wherein the agomelatine-containing layer comprises a permeation enhancer selected from the group consisting of diethylene glycol monoethyl ether, dipropylene glycol, levulinic acid, 2,5-dimethyl isosorbide, lauryl lactate, dimethylethylene urea, N,N-diethyl-meta-toluamide, propylene glycol monocaprylate, 2-methoxy-4-(prop-2-en-1-yl)phenol, lactic acid and laurocapram.
37. Transmucosal therapeutic system according to any one of items 16 to 36,
   wherein the agomelatine-containing layer comprises a permeation enhancer in an amount of at least 1 wt-%, at least 2 wt-% or at least 5 wt-%, or in an amount of less than or equal to 20 wt-%, less than or equal to 15 wt-%, or less than or equal to 10 wt-%, or in an amount of from 1 to 20 wt-%, from 2 to 15 wt-%, or from 5 to 10 wt-% each.
38. Transmucosal therapeutic system according to any one of items 1 to 37,
   wherein the agomelatine-containing layer comprises substantially no water.
39. Transmucosal therapeutic system according to item 38,
   wherein the agomelatine-containing layer comprises less than or equal to 12 wt-%, less than or equal to 8 wt-%, less than or equal to 5 wt-%, or less than or equal to 4 wt-% water.
40. Transmucosal therapeutic system according to any one of items 1 to 39,
   wherein the agomelatine-containing layer comprises substantially no volatile solvent,
   wherein the volatile solvent is selected from the group consisting of C1 to C3 linear and branched alcohols, ethyl acetate, hexane, n-heptane, and any mixtures thereof.
41. Transmucosal therapeutic system according to item 40,
   wherein the agomelatine-containing layer comprises less than or equal to 5 wt-%, less than or equal to 3 wt-%, or less than or equal to 1 wt-% volatile solvent.
42. Transmucosal therapeutic system according to any one of items 1 to 41,
   wherein the agomelatine-containing layer is obtainable by drying a coated coating composition comprising the agomelatine, the dissolvable film-forming agent, and ethanol.
43. Transmucosal therapeutic system according to any one of items 1 to 42,
   wherein the agomelatine-containing layer is obtainable by drying a coated coating composition comprising the agomelatine, the dissolvable film-forming agent, and water.
44. Transmucosal therapeutic system according to item 42 or 43,
   wherein the agomelatine-containing layer is obtainable by drying a coated coating composition comprising the agomelatine, the dissolvable film-forming agent, ethanol and water.
45. Transmucosal therapeutic system according to any one of items 42 to 44,
   wherein the agomelatine-containing layer is obtainable by drying a coated coating composition comprising less than 50 wt-%, or less than 20 wt-%, or less than 10 wt-%, or less than 5 wt-% water.
46. Transmucosal therapeutic system according to any one of items 1 to 45,
   wherein the agomelatine-containing layer has an area weight of at least 25 g/m², at least 35 g/m², or at least 40 g/m², or has an area weight of less than or equal to 300 g/m², less than or equal to 250 g/m², or less than or equal to 200 g/m², or has an area weight of from 25 to 300 g/m², from 35 to 250 g/m², or from 40 to 200 g/m².
47. Transmucosal therapeutic system according to any one of items 1 to 46,
   wherein the agomelatine-containing layer comprises at least 0.1 mg/cm², at least 0.2 mg/cm², or at least 0.4 mg/cm² agomelatine, or wherein the agomelatine-containing layer comprises less than or equal to 2.0 mg/cm², less than or equal to 1.5 mg/cm², or less than or equal to 1.2 mg/cm² agomelatine, or wherein the agomelatine-containing layer comprises from 0.1 to 2.0 mg/cm², from 0.2 to 1.5 mg/cm², or from 0.4 to 1.2 mg/cm² agomelatine.
48. Transmucosal therapeutic system according to any one of items 1 to 47,
   wherein the agomelatine is included in the agomelatine-containing layer in dissolved form, in dispersed form, in crystalline form, in particular in one of its polymorph forms, in an amorphous form, as a hydrate, a solvate, a hybrid type form of any of the foregoing forms or a mixture thereof.
49. Transmucosal therapeutic system according to any one of items 1 to 48,
   wherein the agomelatine-containing layer is obtainable by incorporating the agomelatine in dissolved form, in dispersed form, in crystalline form, in particular in one of its polymorph forms, in an amorphous form, as a hydrate, a solvate, a hybrid type form of any of the foregoing forms or a mixture thereof.
50. Transmucosal therapeutic system according to any one of items 1 to 49,
   wherein the agomelatine in the agomelatine-containing layer is dissolved or is present in dispersed form.
51. Transmucosal therapeutic system according to item 50,
   wherein the agomelatine in the agomelatine-containing layer is present in dispersed form.
52. Transmucosal therapeutic system according to item 50,
   wherein the agomelatine in the agomelatine-containing layer is dissolved.
53. Transmucosal therapeutic system according to any one of items 1 to 52,
   wherein at least 90 mol%, preferably at least 95 mol%, more preferably at least 98 mol% and most preferably at least 99 mol% of the agomelatine in the agomelatine-containing layer is present in dissolved form.
54. Transmucosal therapeutic system according to any one of items 1 to 53,
   wherein the agomelatine-containing layer is free of agomelatine crystals.
55. Transmucosal therapeutic system according to any one of items 1 to 54,
   wherein the agomelatine has a purity of at least 95%, preferably of at least 98% and more preferably of at least 99% as determined by quantitative HPLC.
56. Transmucosal therapeutic system according to any one of items 1 to 55,
   wherein the mucoadhesive layer structure further comprises one or more further layers selected from
      B) a mucosa-contacting layer, and
      C) a cosmetic layer,
   wherein
   the further layers adjoin the agomelatine-containing layer, and
   the mucosa-contacting layer and the cosmetic layer, if both are present, adjoin the agomelatine-containing layer on opposite sides.
57. Transmucosal therapeutic system according to item 56,
   wherein the mucoadhesive layer structure does not comprise a mucosa-contacting layer.
58. Transmucosal therapeutic system according to item 56,
   wherein the mucoadhesive layer structure further comprises a mucosa-contacting layer and the mucosa-contacting layer is mucoadhesive.
59. Transmucosal therapeutic system according to item 56 or 58,
   wherein the mucoadhesive layer structure further comprises a mucosa-contacting layer, wherein the size of the agomelatine-containing layer and the size of the mucosa-contacting layer are coextensive.
60. Transmucosal therapeutic system according to any one of items 56 to 59,
   wherein the mucoadhesive layer structure does not comprise a cosmetic layer.
61. Transmucosal therapeutic system according to any one of items 56 to 59,
   wherein the mucoadhesive layer structure further comprises a cosmetic layer,
   wherein the cosmetic layer prevents the patient from touching the agomelatine-containing layer during administration of the transmucosal therapeutic system.
62. Transmucosal therapeutic system according to any one of items 56 to 59 and 61,
   wherein the mucoadhesive layer structure further comprises a cosmetic layer,
   wherein the size of the agomelatine-containing layer and the size of the cosmetic layer are coextensive, or the cosmetic layer is larger in size than and extends the surface area of the agomelatine-containing layer.
63. Transmucosal therapeutic system according to any one of items 56 to 59, 61 and 62,
   wherein the cosmetic layer dissolves in water, in artificial or natural saliva, or in any other aqueous medium at 37 °C and 150 rpm, in less than 3 minutes, less than 1 minute, or in less than 30 seconds.
64. Transmucosal therapeutic system according to any one of items 1 to 55,
   wherein the mucoadhesive layer structure consists of the agomelatine-containing layer.
65. Transmucosal therapeutic system according to any one of items 1 to 64,
   wherein the agomelatine-containing layer is mucoadhesive.
66. Transmucosal therapeutic system according to any one of items 1 to 65,
   wherein the mucoadhesive layer structure contains agomelatine in a therapeutically effective amount.
67. Transmucosal therapeutic system according to any one of items 1 to 66,
   wherein the mucoadhesive layer structure comprises at least 0.1 mg, at least 0.2 mg, or at least 0.4 mg agomelatine, or wherein the agomelatine-containing layer comprises less than or equal to 20 mg, less than or equal to 15 mg, or less than or equal to 10 mg agomelatine, or wherein the agomelatine-containing layer comprises from 0.1 mg to 20 mg, from 0.2 mg to 15 mg, or from 0.4 mg to 10 mg agomelatine.
68. Transmucosal therapeutic system according to any one of items 1 to 67,
   wherein
   the mucoadhesive layer structure dissolves in water, in artificial or natural saliva, or in any other aqueous medium at 37 °C and 150 rpm, in more than 30 seconds and less than 5 hours, or in more than 1 minute and less than 4 hours, or more than 2 minutes and less than 3 hours, or more than 4 minutes and less than 2 hours.
69. Transmucosal therapeutic system according to any one of items 1 to 68,
   wherein the transmucosal therapeutic system has an area of release of at least 0.1 cm², at least 0.2 cm², or at least 0.5 cm², or has an area of release of less than or equal to 10 cm², less than or equal to 7 cm², or less than or equal to 5 cm², or has an area of release of from 0.1 to 10 cm², from 0.2 to 7 cm², or from 0.5 to 5 cm².
70. Transmucosal therapeutic system according to any one of items 1 to 69,
   wherein the transmucosal therapeutic system does not comprise a backing layer,
   wherein the backing layer does not dissolve in less than 15 minutes, in less than 10 minutes, or in less than 5 minutes upon administration of the transmucosal therapeutic system to a human patient.
71. Transmucosal therapeutic system according to any one of items 1 to 70,
   wherein the transmucosal therapeutic system does not comprise a backing layer,
   wherein the backing layer does not completely dissolve in water, in artificial or natural saliva, or in any other aqueous medium, at 37 °C and 150 rpm, in less than 30 minutes, in less than 15 minutes, or in less than 10 minutes.
72. Transmucosal therapeutic system according to any one of items 1 to 71,
   wherein the transmucosal therapeutic system does not comprise a backing layer.
73. Transmucosal therapeutic system according to any one of items 1 to 72,
   further comprising a release liner.
74. Transmucosal therapeutic system according to any one of items 1 to 65,
   wherein the transmucosal therapeutic system does not comprise a release liner.
75. Transmucosal therapeutic system according to any one of items 1 to 74,
   wherein the transmucosal therapeutic system is in the form of a film.
76. Transmucosal therapeutic system according to item 75,
   wherein the transmucosal therapeutic system is in the form of a thin film having an area weight of at least 25 g/m², at least 35 g/m², or at least 40 g/m², or an area weight of less than or equal to 300 g/m², less than or equal to 250 g/m², or less than or equal to 200 g/m², or an area weight of from 25 to 300 g/m², from 35 to 250 g/m², or from 40 to 200 g/m².
77. Transmucosal therapeutic system according to any one of items 1 to 76,
   wherein the transmucosal therapeutic system is in the form of a film having a circular, rectangular or square shape.
78. Transmucosal therapeutic system according to any one of items 1 to 77,
   wherein the administration of the transmucosal therapeutic system consists of applying the mucoadhesive layer structure to the mucosa of the oral cavity of a human patient and maintaining the same on the mucosa until dissolved.
79. Transmucosal therapeutic system according to item 78,
   wherein the administration of the transmucosal therapeutic system consists of applying the mucoadhesive layer structure to the buccal, sublingual, gingival or palatal mucosa of the oral cavity of a human patient and maintaining the same on the mucosa until dissolved.
80. Transmucosal therapeutic system according to any one of items 1 to 79,
   wherein the transmucosal therapeutic system provides a mucosal permeation rate of agomelatine as measured with pig esophagus mucosa of from 10 µg/cm²-hr to 150 µg/cm²-hr after 1 hour.
81. Transmucosal therapeutic system according to any one of items 1 to 80,
   wherein the transmucosal therapeutic system provides a cumulative release of agomelatine as measured with pig esophagus mucosa of at least 0.02 mg/cm², at least 0.05 mg/cm² or at least 0.1 mg/cm², or less than or equal to 0.5 mg/cm², less than or equal to 0.4 mg/cm², or less than or equal to 0.3 mg/cm², or of from 0.02 mg/cm² to 0.5 mg/cm², from 0.05 mg/cm² to 0.4 mg/cm², or from 0.1 mg/cm² to 0.3 mg/cm² over a time period of 8 hours.
82. Transmucosal therapeutic system according to any one of items 1 to 81
   for use in a method of treating a human patient.
83. Transmucosal therapeutic system according to item 82
   for use in a method of treating major depression.
84. Transmucosal therapeutic system according to item 82 or 83
   for use in a method of treatment,
   wherein the transmucosal therapeutic system is administered by applying the mucoadhesive layer structure to the mucosa of the oral cavity of a human patient and maintained on the mucosa until dissolved.
85. Transmucosal therapeutic system according to item 84
   for use in a method of treatment,
   wherein the transmucosal therapeutic system is administered by applying the mucoadhesive layer structure to the buccal, sublingual, gingival or palatal mucosa of the oral cavity of a human patient and maintained on the mucosa until dissolved.
86. Transmucosal therapeutic system according to any one of items 82 to 85
   for use in a method of treatment,
   wherein the transmucosal therapeutic system is administered in the evening or at night time before going to bed.
87. A method of treatment,
   wherein the transmucosal therapeutic system according to any one of items 1 to 81
   is administered to a human patient.
88. A method of treating major depression according to item 88,
   wherein the transmucosal therapeutic system according to any one of items 1 to 81
   is administered to a human patient.
89. A method of treatment according to item 88 or 89,
   wherein the transmucosal therapeutic system according to any one of items 1 to 81
   is administered by applying the mucoadhesive layer structure to the mucosa of the oral cavity of a human patient and maintained on the mucosa until dissolved.
90. A method of treatment according to according to item 89,
   wherein the transmucosal therapeutic system according to any one of items 1 to 82
   is administered by applying the mucoadhesive layer structure to the mucosa, in particular to the buccal, sublingual, gingival or palatal mucosa, of the oral cavity of a human patient and maintained on the mucosa until dissolved.
91. A method of treatment according to any one of items 87 to 90
   wherein the transmucosal therapeutic system is administered in the evening or at night time before going to bed.
92. Process of manufacture of an agomelatine-containing layer comprising the steps of:
   i) combining at least agomelatine and a dissolvable film-forming agent in a solvent to obtain a coating composition;
   ii) coating the coating composition onto a release liner; and
   iii) drying the coated coating composition to form the agomelatine-containing layer.
93. The process according to item 92,
   wherein in step i), the agomelatine is dissolved.
94. The process according to item 92,
   wherein in step i), the agomelatine is dispersed.
95. The process according to any one of items 92 to 94,
   wherein the solvent comprises an alcoholic solvent selected from methanol, ethanol, isopropanol and mixtures thereof.
96. The process according to item 95,
   wherein the solvent comprises ethanol, or consists of ethanol.
97. The process according to any one of items 92 to 95,
   wherein the solvent comprises water.
98. The process according to any one of items 92 to 96,
   wherein the solvent does not comprise water in an amount of more than 5 wt-%, more than 2 wt%, more than 1 wt-% or more than 0.5 wt-%.
99. The process according to any one of items 92 to 98,
   wherein drying is performed at a temperature of from 40 to 90 °C, or from 60 to 80 °C.
100. The process according to any one of items 92 to 99,
   wherein step i) consists of combining at least agomelatine, a dissolvable film-forming agent, and one or more excipients selected from the group consisting of fatty acids, sweeteners, and flavoring agents, in a solvent to obtain a coating composition.
101. The process according to any one of items 92 to 100,
   wherein the dissolvable film-forming agent, if casted into a film having an area weight of 50 g/m², dissolves in water, in artificial or natural saliva, or in any other aqueous medium, at 37 °C and 150 rpm, in less than 5 hours, less than 3 hours, less than 2 hours, or less than 1 hour, or in more than 5 seconds, more than 30 seconds, more than 1 minute, or more than 2 minutes, or more than 5 seconds and less than 5 hours, in more than 30 seconds and less than 3 hours, more than 1minute and less than 2 hours, or in more than 2 minutes and less than 1 hour.
102. The process according to any one of items 92 to 101,
   wherein the dissolvable film-forming agent is selected from the group consisting of polymers such as polyvinylpyrrolidone, methyl cellulose, ethyl cellulose, hydroxyethyl cellulose, hydroxypropyl cellulose, hydroxypropyl methyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, methacrylic acid - methyl methacrylate copolymers, and methacrylic acid - ethyl methacrylate copolymers, natural film-forming agents such as shellac, pectin, gelatine, alginate, pullulan and starch derivatives, and any mixtures thereof.
103. The process according to item 102,
   wherein
   the dissolvable film-forming agent is selected from the group consisting of polymers such as polyvinylpyrrolidone, methyl cellulose, hydroxyethyl cellulose, hydroxypropyl cellulose, hydroxypropylmethyl cellulose, carboxymethyl cellulose sodium, polyethylene glycol- polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, and any mixtures thereof.
104. The process according to item 103,
   wherein
   the dissolvable film-forming agent is selected from the group consisting of polymers such as polyvinylpyrrolidone, hydroxypropyl cellulose, polyethylene glycol- polyvinyl acetate- and polyvinylcaprolactame-based graft copolymers, polyvinylpyrrolidone-polyvinylacetate copolymers, and any mixtures thereof.
105. The process according to item 104,
   wherein the dissolvable film-forming agent is a hydroxypropyl cellulose.
106. The process according to item 105,
   wherein the dissolvable film-forming agent is a hydroxypropyl cellulose having a molecular weight of from 50,000 to 1,500,000.
107. The process according to item 106,
   wherein the dissolvable film-forming agent is a hydroxypropyl cellulose having a molecular weight of 80,000, 95,000, 370,000 or 1,150,000.
108. The process according to item 104,
   wherein the dissolvable film-forming agent is a polyvinylpyrrolidone.
109. The process according to item 108,
   wherein the polyvinylpyrrolidone is selected from soluble polyvinylpyrrolidones.
110. The process according to item 108 or 109,
   wherein the polyvinylpyrrolidone is selected from polyvinylpyrrolidones having a K-Value within a range selected from the group of ranges consisting of
   9 to 15, and preferably 10.2 to 13.8,
   15 to 20, and preferably 15.3 to 18.4,
   20 to 27, and preferably 22.5 to 27.0,
   27 to 35, and preferably 27.0 to 32.4, and
   75 to 110, and preferably 81.0 to 97.2.
111. The process according to any one of items 92 to 110,
   wherein in step 1), the agomelatine is combined in dissolved form, in dispersed form, in crystalline form, in particular in one of its polymorph forms, in an amorphous form, as a cocrystal, as a hydrate, a solvate, a hybrid type form of any of the foregoing forms or a mixture thereof.
112. A transmucosal therapeutic system for the transmucosal administration of agomelatine, obtainable by the process of manufacture according to any one of items 92 to 111.
113. Transmucosal therapeutic system for the transmucosal administration of agomelatine comprising a mucoadhesive layer structure, said mucoadhesive layer structure comprising at least
   A) an agomelatine-containing layer comprising
      i) 3 to 10 wt-% agomelatine ;
      ii) a dissolvable film-forming agent, and
      iii) from 5 to 15 wt-% of a fatty acid,
      iv) from 0.1 to 2 wt-% of one or more sweeteners, and
      v) from 0.2 to 2.0 wt-% of a flavoring agent
   wherein
   the dissolvable film-forming agent is selected from the group consisting of polyvinylpyrrolidone and hydroxypropyl cellulose, and
   the area weight of the agomelatine-containing layer ranges from 100 to 150 g/m².

## Claims

1. Transmucosal therapeutic system for the transmucosal administration of agomelatine comprising a mucoadhesive layer structure, said mucoadhesive layer structure comprising
A) an agomelatine-containing layer comprising
i) agomelatine; and
ii) a dissolvable film-forming agent.

2. Transmucosal therapeutic system according to claim 1,
wherein
the dissolvable film-forming agent, if casted into a film having an area weight of from 30 to 100 g/m², or of 50 g/m², dissolves in water, in artificial or natural saliva, or in any other aqueous medium, at 37 °C and 150 rpm, in less than 5 hours, less than 3 hours, less than 2 hours, or less than 1 hour, or in more than 5 seconds, more than 30 seconds, more than 1 minute, or more than 2 minutes, or more than 5 seconds and less than 5 hours, in more than 30 seconds and less than 3 hours, more than 1 minute and less than 2 hours, or in more than 2 minutes and less than 1 hour.

3. Transmucosal therapeutic system according to claim 1 or 2,
wherein
the dissolvable film-forming agent is selected from the group consisting of polymers such as polyvinylpyrrolidone, methyl cellulose, ethyl cellulose, hydroxyethyl cellulose, hydroxypropyl cellulose, hydroxypropylmethyl cellulose, carboxymethyl cellulose sodium, polyethylene glycol-polyvinyl acetate- and polyvinylcaprolactame-based graft copolymers, polyvinyl alcohol, polyvinyl alcohol-polyethylene glycol copolymers, polyvinylpyrrolidone-polyvinylacetate copolymers, polyethylene oxides, polyethylene glycols, methacrylic acid - methyl methacrylate copolymers, and methacrylic acid - ethyl methacrylate copolymers, and natural film-forming agents such as shellac, pectin, gelatine, alginate, pullulan and starch derivatives, and any mixtures thereof, and/or
wherein the amount of the dissolvable film-forming agent is at least 65 wt-%, at least 75 wt-% or at least 85 wt-%, or the amount of the dissolvable film-forming agent is less than or equal to 98 wt-%, less than or equal to 94 wt-% or less than or equal to 90 wt-%, or the amount of the dissolvable film-forming agent ranges from 65 to 98 wt-%, from 75 to 94 wt-%, or from 80 to 90 wt-% of the agomelatine-containing layer.

4. Transmucosal therapeutic system according to any one of claims 1 to 3,
wherein the agomelatine-containing layer comprises at least 1 wt-% agomelatine, at least 2 wt-% agomelatine, or at least 3 wt-% agomelatine, and/or
wherein the agomelatine-containing layer comprises less than or equal to 25 wt-% agomelatine, less than or equal to 20 wt-% agomelatine, or less than or equal to 10 wt-% agomelatine, and/or
wherein the agomelatine-containing layer comprises from 1 to less than or equal to 25 wt-% agomelatine, from 2 to less than or equal to 20 wt-% agomelatine, or from 3 to less than or equal to 10 wt-% agomelatine, and/or
wherein the agomelatine in the agomelatine-containing layer is dissolved or is present in dispersed form, and/or
wherein the agomelatine-containing layer is free of agomelatine crystals.

5. Transmucosal therapeutic system according to any one of claims 1 to 4,
wherein the agomelatine-containing layer further comprises one or more excipients selected from the group consisting of fatty acids, sweeteners, flavoring agents, colorants, permeation enhancers, solubilizers, plasticizers, humectants, disintegrants, emulsifiers, antioxidants, stabilizers, buffer reagents and further film-forming agents,
wherein, in particular, the fatty acid is a saturated or unsaturated, linear or branched carboxylic acid comprising 4 to 24 carbon atoms, and in particular is selected from the group consisting of caprylic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid, and/or
wherein, in particular, the agomelatine-containing layer comprises one or more natural or artificial sweeteners selected from the group consisting of saccharose, glucose, fructose, sorbitol, mannitol, isomalt maltitol lactitol, xylitol, erythritol, sucralose, acesulfame potassium, aspartame, cyclamate, neohesperidine, neotame, steviol glycosides, thaumatin and saccharin sodium, and/or
wherein, in particular, the agomelatine-containing layer comprises one or more natural or artificial flavoring agents selected from the group consisting of vanillin, methyl salicylate, menthol, manzanate, diacetyl, acetylpropionyl, acetoin, isoamyl acetate, benzaldehyde, cinnamaldehyde, ethyl propionate, methyl anthranilate, limonene, ethyl decadienoate, allyl hexanoate, ethyl maltol, 2,4-dithiapentane, ethylvanillin and eucalyptol as well as flavoring compositions such as peppermint flavor.

6. Transmucosal therapeutic system according to any one of claims 1 to 5,
wherein the agomelatine-containing layer comprises substantially no water, and/or
wherein the agomelatine-containing layer comprises less than or equal to 12 wt-%, less than or equal to 8 wt-%, less than or equal to 5 wt-%, or less than or equal to 4 wt-% water.

7. Transmucosal therapeutic system according to any one of claims 1 to 6,
wherein the agomelatine-containing layer is obtainable by drying a coated coating composition comprising
the agomelatine, the dissolvable film-forming agent, and ethanol, or
the agomelatine, the dissolvable film-forming agent, and water, and/or
the agomelatine, the dissolvable film-forming agent, ethanol and water, and/or
wherein the agomelatine-containing layer is obtainable by drying a coated coating composition comprising less than 50 wt-%, or less than 20 wt-%, or less than 10 wt-%, or less than 5 wt-% water.

8. Transmucosal therapeutic system according to any one of claims 1 to 7,
wherein the agomelatine-containing layer has an area weight of at least 25 g/m², at least 35 g/m², or at least 40 g/m², or has an area weight of less than or equal to 300 g/m², less than or equal to 250 g/m², or less than or equal to 200 g/m², or has an area weight of from 25 to 300 g/m², from 35 to 250 g/m², or from 40 to 200 g/m².

9. Transmucosal therapeutic system according to claim 8,
wherein the mucoadhesive layer structure comprises or does not comprise a mucosa-contacting layer, and/or
wherein the mucoadhesive layer structure comprises or does not comprise a cosmetic layer, and/or
wherein the transmucosal therapeutic system does not comprise a backing layer, and the backing layer in particular does not dissolve in less than 15 minutes, in less than 10 minutes, or in less than 5 minutes upon administration of the transmucosal therapeutic system to a human patient.

10. Transmucosal therapeutic system according to any one of claims 1 to 9,
wherein
the mucoadhesive layer structure dissolves in water, in artificial or natural saliva, or in any other aqueous medium at 37 °C and 150 rpm, in more than 30 seconds and less than 5 hours, or in more than 1 minute and less than 4 hours, or more than 2 minutes and less than 3 hours, or more than 4 minutes and less than 2 hours.

11. Transmucosal therapeutic system according to any one of claims 1 to 10,
wherein the transmucosal therapeutic system provides a mucosal permeation rate of agomelatine as measured with pig esophagus mucosa of from 10 µg/cm²-hr to 150 µg/cm²-hr after 1 hour, and/or
wherein the transmucosal therapeutic system provides a cumulative release of agomelatine as measured with pig esophagus mucosa of at least 0.02 mg/cm², at least 0.05 mg/cm² or at least 0.1 mg/cm², or less than or equal to 0.5 mg/cm², less than or equal to 0.4 mg/cm², or less than or equal to 0.3 mg/cm², or of from 0.02 mg/cm² to 0.5 mg/cm², from 0.05 mg/cm² to 0.4 mg/cm², or from 0.1 mg/cm² to 0.3 mg/cm² over a time period of 8 hours.

12. Transmucosal therapeutic system according to any one of claims 1 to 11 for use in a method of treating a human patient.

13. Transmucosal therapeutic system according to claim 12,
wherein the method of treatment is a method of treating major depression, and/or
wherein the transmucosal therapeutic system is administered by applying the mucoadhesive layer structure to the mucosa, and in particular to the buccal, sublingual, gingival or palatal mucosa, of the oral cavity of a human patient and maintained on the mucosa until dissolved, and/or
wherein the transmucosal therapeutic system is administered in the evening or at night time before going to bed.

14. Process of manufacture of an agomelatine-containing layer comprising the steps of:
i) combining at least agomelatine and a dissolvable film-forming agent in a solvent to obtain a coating composition;
ii) coating the coating composition onto a release liner; and
iii) drying the coated coating composition to form the agomelatine-containing layer.

15. Transmucosal therapeutic system for the transmucosal administration of agomelatine comprising a mucoadhesive layer structure, said mucoadhesive layer structure comprising at least
A) an agomelatine-containing layer comprising
i) 3 to 10 wt-% agomelatine ;
ii) a dissolvable film-forming agent, and
iii) from 5 to 15 wt-% of a fatty acid,
iv) from 0.1 to 2 wt-% of one or more sweeteners, and
v) from 0.2 to 2.0 wt-% of a flavoring agent
wherein
the dissolvable film-forming agent is selected from the group consisting of polyvinylpyrrolidone and hydroxypropyl cellulose, and
the area weight of the agomelatine-containing layer ranges from 100 to 150 g/m².
